# EUROPEAN PATENT APPLICATION

(11) **EP 3 862 019 A1**
(43) Date of publication of application: **11.08.2021**
(21) Application number: 19869054.7
(22) Date of filing: 04.10.2019
(51) Int. Cl.: A61K 38/26, A61K 47/56, A61K 47/68, A61P 3/00, A61P 3/04, C07K 14/605, A61K 31/4985, A61K 45/06

(54) **THERAPEUTIC USES OF GLUCAGON AND COMBINED PRODUCT COMPRISING SAME**

(30) Priority: 04.10.2018 KR 20180118462; 04.10.2018 KR 20180118463
(71) Applicant: Hanmi Pharm. Co., Ltd., Hwaseong-si, Gyeonggi-do 18536 (KR)
(72) Inventor: KIM, Jung Kuk, Hwaseong-si, Gyeonggi-do 18469 (KR); PARK, Young Jin, Hwaseong-si, Gyeonggi-do 18469 (KR); CHOI, In Young, Hwaseong-si, Gyeonggi-do 18469 (KR); LEE, Sang Don, Hwaseong-si, Gyeonggi-do 18469 (KR); LEE, Jong Suk, Hwaseong-si, Gyeonggi-do 18469 (KR)
(74) Representative: Mewburn Ellis LLP
(86) International application number: PCT/KR2019/013057
(87) International publication number: WO 2020/071865

(57) **Abstract**

Provided are a combination including glucagon and a compound or substance with therapeutic activity against a metabolic syndrome and a use thereof.

<110>    HANMI PHARM. CO., LTD.

<120>    Therapeutic use of Glucagon or a combination comprising the same

<130>    OPA19215-PCT

<150>    KR 10-2018-0118462
<151>    2018-10-04

<150>    KR 10-2018-0118463
<151>    2018-10-04

<160>    50

<170>    KoPatentIn 3.0

<210>    1
<211>    29
<212>    PRT
<213>    homo sapiens

<400>    1
His Ser Gln Gly Thr Phe Thr Ser Asp Tyr Ser Lys Tyr Leu Asp Ser
  1               5                  10                  15

Arg Arg Ala Gln Asp Phe Val Gln Trp Leu Met Asn Thr
             20                  25


<210>    2
<211>    29
<212>    PRT
<213>    Artificial Sequence

<220>
<223>    glucagon derivative


<400>    2
His Ser Gln Gly Thr Phe Thr Ser Asp Tyr Ser Lys Tyr Leu Asp Cys
  1               5                  10                  15

Asp Arg Ala Gln Asp Phe Val Gln Trp Leu Met Asn Thr
             20                  25


<210>    3
<211>    29
<212>    PRT
<213>    Artificial Sequence

<220>
<223>    glucagon derivative


<400>    3
His Ser Gln Gly Thr Phe Thr Ser Asp Tyr Ser Lys Tyr Leu Asp Cys
  1               5                  10                  15

Glu Arg Ala Gln Asp Phe Val Gln Trp Leu Met Asn Thr
             20                  25

<210>    4
<211>    29
<212>    PRT
<213>    Artificial Sequence

<220>
<223>    glucagon derivative


<400>    4
His Ser Gln Gly Thr Phe Thr Ser Asp Tyr Ser Lys Tyr Leu Asp Ser
1               5                   10                  15

Cys Asp Ala Gln Asp Phe Val Gln Trp Leu Met Asn Thr
            20                  25


<210>    5
<211>    29
<212>    PRT
<213>    Artificial Sequence

<220>
<223>    glucagon derivative


<400>    5
His Ser Gln Gly Thr Phe Thr Ser Asp Tyr Ser Lys Tyr Leu Asp Ser
1               5                   10                  15

Cys Glu Ala Gln Asp Phe Val Gln Trp Leu Met Asn Thr
            20                  25


<210>    6
<211>    29
<212>    PRT
<213>    Artificial Sequence

<220>
<223>    glucagon derivative


<400>    6
His Ser Gln Gly Thr Phe Thr Ser Asp Tyr Ser Lys Tyr Leu Asp Ser
1               5                   10                  15

Cys Glu Ala Asp Asp Phe Val Gln Trp Leu Met Asn Thr
            20                  25


<210>    7
<211>    29
<212>    PRT
<213>    Artificial Sequence

<220>
<223>    glucagon derivative


<400>    7
Tyr Ser Gln Gly Thr Phe Thr Ser Asp Tyr Ser Lys Tyr Leu Asp Ser
1               5                   10                  15

Cys Glu Ala Asp Asp Phe Val Gln Trp Leu Met Asn Thr
            20                  25

<210> 8
<211> 29
<212> PRT
<213> Artificial Sequence

<220>
<223> glucagon derivative

<220>
<221> MISC_FEATURE
<222> (2)
<223> Xaa is aminoisobutyric acid (Aib)

<400> 8
Tyr Xaa Gln Gly Thr Phe Thr Ser Asp Tyr Ser Lys Tyr Leu Asp Ser
1               5                   10                  15

Cys Asp Ala Gln Asp Phe Val Gln Trp Leu Ile Asn Thr
            20                  25

<210> 9
<211> 29
<212> PRT
<213> Artificial Sequence

<220>
<223> glucagon derivative

<220>
<221> MISC_FEATURE
<222> (2)
<223> Xaa is aminoisobutyric acid (Aib)

<400> 9
Tyr Xaa Gln Gly Thr Phe Thr Ser Asp Tyr Ser Lys Tyr Leu Asp Ser
1               5                   10                  15

Cys Asp Ala Gln Asp Phe Val Val Trp Leu Ile Asn Thr
            20                  25

<210> 10
<211> 29
<212> PRT
<213> Artificial Sequence

<220>
<223> glucagon derivative

<220>
<221> MISC_FEATURE
<222> (2)

<223>    Xaa is aminoisobutyric acid (Aib)

<400>    10
Tyr Xaa Gln Gly Thr Phe Thr Ser Asp Tyr Ser Lys Tyr Leu Asp Ser
1               5                   10                  15

Cys Asp Ala Asp Asp Phe Val Val Trp Leu Ile Asn Thr
            20                  25

<210>    11
<211>    29
<212>    PRT
<213>    Artificial Sequence

<220>
<223>    glucagon derivative

<220>
<221>    MISC_FEATURE
<222>    (2)
<223>    Xaa is aminoisobutyric acid (Aib)

<400>    11
Tyr Xaa Gln Gly Thr Phe Thr Ser Asp Tyr Ser Lys Tyr Leu Asp Glu
1               5                   10                  15

Lys Cys Ala Lys Glu Phe Val Gln Trp Leu Met Asn Thr
            20                  25

<210>    12
<211>    30
<212>    PRT
<213>    Artificial Sequence

<220>
<223>    glucagon derivative

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    12
Tyr Xaa Gln Gly Thr Phe Thr Ser Asp Tyr Ser Lys Tyr Leu Asp Glu
1               5                   10                  15

Lys Arg Ala Lys Glu Phe Val Gln Trp Leu Met Asn Thr Cys
            20                  25                  30

<210>    13
<211>    29
<212>    PRT
<213>    Artificial Sequence

<220>
<223>    glucagon derivative


<220>
<221>    MISC_FEATURE
<222>    (2)
<223>    Xaa is aminoisobutyric acid (Aib)


<400>    13
Tyr Xaa Gln Gly Thr Phe Thr Ser Asp Tyr Ser Cys Tyr Leu Asp Ser
1               5                   10                  15

Arg Arg Ala Gln Asp Phe Val Gln Trp Leu Met Asn Thr
            20                  25


<210>    14
<211>    29
<212>    PRT
<213>    Artificial Sequence

<220>
<223>    glucagon derivative


<220>
<221>    MISC_FEATURE
<222>    (2)
<223>    Xaa is aminoisobutyric acid (Aib)


<400>    14
Tyr Xaa Gln Gly Thr Phe Thr Ser Asp Tyr Ser Lys Tyr Leu Asp Cys
1               5                   10                  15

Lys Arg Ala Lys Glu Phe Val Gln Trp Leu Met Asn Thr
            20                  25


<210>    15
<211>    29
<212>    PRT
<213>    Artificial Sequence

<220>
<223>    glucagon derivative


<220>
<221>    MISC_FEATURE
<222>    (2)
<223>    Xaa is aminoisobutyric acid (Aib)


<400>    15
Tyr Xaa Gln Gly Thr Phe Thr Ser Asp Tyr Ser Lys Tyr Leu Cys Glu

 1               5                   10                  15

Lys Arg Ala Gln Asp Phe Val Val Trp Leu Met Asn Thr
            20                  25

<210>    16
<211>    29
<212>    PRT
<213>    Artificial Sequence

<220>
<223>    glucagon derivative

<220>
<221>    MISC_FEATURE
<222>    (2)
<223>    Xaa is aminoisobutyric acid (Aib)

<400>    16
Tyr Xaa Gln Gly Thr Phe Thr Ser Asp Tyr Ser Lys Tyr Leu Asp Cys
 1               5                   10                  15

Arg Arg Ala Gln Val Phe Val Gln Trp Leu Met Arg Thr
            20                  25

<210>    17
<211>    29
<212>    PRT
<213>    Artificial Sequence

<220>
<223>    glucagon derivative

<220>
<221>    MISC_FEATURE
<222>    (2)
<223>    Xaa is aminoisobutyric acid (Aib)

<400>    17
Tyr Xaa Gln Gly Thr Phe Thr Ser Asp Tyr Ser Lys Tyr Leu Asp Cys
 1               5                   10                  15

Val Arg Ala Gln Asp Phe Val Gln Trp Leu Met Arg Thr
            20                  25

<210>    18
<211>    29
<212>    PRT
<213>    Artificial Sequence

<220>
<223>    glucagon derivative

<220>
<221>    MISC_FEATURE

<222>    (2)
<223>    Xaa is aminoisobutyric acid (Aib)

<400>    18
Tyr Xaa Gln Gly Thr Phe Thr Ser Asp Tyr Ser Lys Tyr Leu Asp Ser
1               5                   10                  15

Arg Arg Ala Cys Asp Phe Arg Leu Trp Leu Met Asn Thr
            20                  25

<210>    19
<211>    29
<212>    PRT
<213>    Artificial Sequence

<220>
<223>    glucagon derivative

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    19
Tyr Xaa Gln Gly Thr Phe Thr Ser Asp Tyr Ser Lys Tyr Leu Cys Glu
1               5                   10                  15

Lys Arg Ala Lys Glu Phe Val Gln Trp Leu Met Asn Thr
            20                  25

<210>    20
<211>    29
<212>    PRT
<213>    Artificial Sequence

<220>
<223>    glucagon derivative

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    20

Tyr Xaa Gln Gly Thr Phe Thr Ser Asp Tyr Ser Lys Tyr Leu Asp Glu
1               5                   10                  15

Cys Arg Ala Lys Glu Phe Val Gln Trp Leu Met Asn Thr
            20                  25

<210>   21
<211>   29
<212>   PRT
<213>   Artificial Sequence

<220>
<223>   glucagon derivative

<220>
<221>   MISC_FEATURE
<222>   (2)
<223>   Xaa is aminoisobutyric acid (Aib)

<220>
<221>   MISC_FEATURE
<222>   (16)..(20)
<223>   amino acids at positions 16 and 20 form a ring

<400>   21
Tyr Xaa Gln Gly Thr Phe Thr Ser Asp Tyr Ser Lys Tyr Leu Asp Glu
1               5                   10                  15

Lys Cys Ala Lys Glu Phe Val Gln Trp Leu Met Asn Thr
            20                  25

<210>   22
<211>   29
<212>   PRT
<213>   Artificial Sequence

<220>
<223>   glucagon derivative

<220>
<221>   MISC_FEATURE
<222>   (2)
<223>   Xaa is aminoisobutyric acid (Aib)

<220>
<221>   MISC_FEATURE
<222>   (16)..(20)
<223>   amino acids at positions 16 and 20 form a ring

<400>   22
Tyr Xaa Gln Gly Thr Phe Thr Ser Asp Tyr Ser Lys Tyr Leu Asp Glu
1               5                   10                  15

Lys Arg Cys Lys Glu Phe Val Gln Trp Leu Met Asn Thr
            20                  25

<210>    23
<211>    29
<212>    PRT
<213>    Artificial Sequence

<220>
<223>    glucagon derivative


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    23
Tyr Xaa Gln Gly Thr Phe Thr Ser Asp Tyr Ser Lys Tyr Cys Asp Glu
1               5                   10                  15

Lys Arg Ala Lys Glu Phe Val Gln Trp Leu Met Asn Thr
            20                  25


<210>    24
<211>    29
<212>    PRT
<213>    Artificial Sequence

<220>
<223>    glucagon derivative


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    24
Tyr Xaa Gln Gly Thr Phe Thr Ser Asp Tyr Ser Lys Cys Leu Asp Glu
1               5                   10                  15

Lys Arg Ala Lys Glu Phe Val Gln Trp Leu Met Asn Thr
            20                  25


<210>    25
<211>    29
<212>    PRT

<213>    Artificial Sequence

<220>
<223>    glucagon derivative


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    25
Tyr Xaa Gln Gly Thr Phe Thr Ser Asp Tyr Ser Lys Tyr Leu Asp Glu
1               5                   10                  15

Lys Arg Ala Lys Cys Phe Val Gln Trp Leu Met Asn Thr
            20                  25


<210>    26
<211>    29
<212>    PRT
<213>    Artificial Sequence

<220>
<223>    glucagon derivative


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    26
Trp Xaa Gln Gly Thr Phe Thr Ser Asp Tyr Ser Lys Tyr Leu Asp Glu
1               5                   10                  15

Cys Arg Ala Lys Asp Phe Val Gln Trp Leu Met Asn Thr
            20                  25


<210>    27
<211>    29
<212>    PRT
<213>    Artificial Sequence

<220>
<223>    glucagon derivative

<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    27
Tyr Xaa Gln Gly Thr Phe Val Ser Asp Tyr Ser Lys Tyr Leu Asp Glu
1               5                   10                  15

Cys Arg Ala Lys Asp Phe Val Gln Trp Leu Met Asn Thr
            20                  25


<210>    28
<211>    29
<212>    PRT
<213>    Artificial Sequence

<220>
<223>    glucagon derivative


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    28
Trp Xaa Gln Gly Thr Phe Val Ser Asp Tyr Ser Lys Tyr Leu Asp Glu
1               5                   10                  15

Cys Arg Ala Lys Asp Phe Val Gln Trp Leu Met Asn Thr
            20                  25


<210>    29
<211>    29
<212>    PRT
<213>    Artificial Sequence

<220>
<223>    glucagon derivative


<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring


<400>    29
Tyr Xaa Gln Gly Thr Phe Thr Ser Asp Tyr Ser Lys Cys Leu Asp Glu
1               5                   10                  15

Arg Arg Ala Lys Asp Phe Val Gln Trp Leu Met Asn Thr
            20                  25


<210>    30
<211>    29
<212>    PRT
<213>    Artificial Sequence

<220>
<223>    glucagon derivative


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    30
Trp Xaa Gln Gly Thr Phe Thr Ser Asp Tyr Ser Lys Cys Leu Asp Glu
1               5                   10                  15

Arg Arg Ala Lys Asp Phe Val Gln Trp Leu Met Asn Thr
            20                  25


<210>    31
<211>    29
<212>    PRT
<213>    Artificial Sequence

<220>
<223>    glucagon derivative


<220>
<221>    MISC_FEATURE
<222>    (2)
<223>    Xaa is aminoisobutyric acid (Aib)


<220>
<221>    MISC_FEATURE
<222>    (17)..(21)

<223>    amino acids at positions 17 and 21 form a ring

<400>    31
Tyr Xaa Gln Gly Thr Phe Thr Ser Asp Tyr Ser Lys Tyr Leu Asp Cys
1               5                   10                  15

Lys Arg Ala Lys Glu Phe Val Gln Trp Leu Met Asn Thr
            20                  25

<210>    32
<211>    28
<212>    PRT
<213>    Artificial Sequence

<220>
<223>    glucagon derivative

<220>
<221>    MISC_FEATURE
<222>    (15)..(19)
<223>    amino acids at positions 15 and 19 form a ring

<400>    32
Ser Gln Gly Thr Phe Thr Ser Asp Tyr Ser Lys Tyr Leu Asp Glu Cys
1               5                   10                  15

Arg Ala Lys Glu Phe Val Gln Trp Leu Met Asn Thr
            20                  25

<210>    33
<211>    29
<212>    PRT
<213>    Artificial Sequence

<220>
<223>    glucagon derivative

<220>
<221>    MISC_FEATURE
<222>    (2)
<223>    Xaa is aminoisobutyric acid (Aib)

<400>    33
Tyr Xaa Gln Gly Thr Phe Thr Ser Asp Tyr Ser Lys Tyr Leu Asp Ser
1               5                   10                  15

Arg Arg Ala Gln Asp Phe Val Gln Trp Leu Met Asn Thr
            20                  25

<210>    34
<211>    29
<212>    PRT
<213>    Artificial Sequence

<220>

<223>    glucagon derivative

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    34
Trp Xaa Gln Gly Thr Phe Thr Ser Asp Tyr Ser Lys Tyr Cys Asp Glu
1               5                   10                  15

Arg Arg Ala Lys Glu Phe Val Gln Trp Leu Met Asn Thr
            20                  25

<210>    35
<211>    29
<212>    PRT
<213>    Artificial Sequence

<220>
<223>    glucagon derivative

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    35
Tyr Xaa Gln Gly Thr Phe Thr Ser Asp Tyr Ser Lys Tyr Cys Asp Glu
1               5                   10                  15

Arg Arg Ala Lys Glu Phe Val Gln Trp Leu Met Asn Thr
            20                  25

<210>    36
<211>    29
<212>    PRT
<213>    Artificial Sequence

<220>
<223>    glucagon derivative

<220>
<221>    MISC_FEATURE

<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    36
Tyr Xaa Gln Gly Thr Phe Thr Ser Asp Cys Ser Lys Tyr Leu Asp Glu
1               5                   10                  15

Arg Arg Ala Lys Glu Phe Val Gln Trp Leu Met Asn Thr
            20                  25

<210>    37
<211>    30
<212>    PRT
<213>    Artificial Sequence

<220>
<223>    glucagon derivative

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    37
Tyr Xaa Gln Gly Thr Phe Thr Ser Asp Tyr Ser Lys Tyr Leu Asp Glu
1               5                   10                  15

Arg Arg Ala Lys Glu Phe Val Gln Trp Leu Met Asn Thr Cys
            20                  25                  30

<210>    38
<211>    29
<212>    PRT
<213>    Artificial Sequence

<220>
<223>    glucagon derivative

<220>
<221>    MISC_FEATURE
<222>    (2)
<223>    Xaa is aminoisobutyric acid (Aib)

<220>

<221>    MISC_FEATURE
<222>    (16)..(20)
<223>    amino acids at positions 16 and 20 form a ring


<400>    38
Tyr Xaa Gln Gly Thr Phe Cys Ser Asp Tyr Ser Lys Tyr Leu Asp Glu
1               5                   10                  15

Arg Arg Ala Lys Glu Phe Val Gln Trp Leu Met Asn Thr
            20                  25


<210>    39
<211>    29
<212>    PRT
<213>    Artificial Sequence

<220>
<223>    glucagon derivative


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    39
Tyr Xaa Gln Gly Thr Phe Val Ser Asp Cys Ser Lys Tyr Leu Asp Glu
1               5                   10                  15

Arg Arg Ala Lys Asp Phe Val Gln Trp Leu Met Asn Thr
            20                  25


<210>    40
<211>    30
<212>    PRT
<213>    Artificial Sequence

<220>
<223>    glucagon derivative


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring

<400>    40
Tyr Xaa Gln Gly Thr Phe Val Ser Asp Tyr Ser Lys Tyr Leu Asp Glu
1               5                   10                  15

Arg Arg Ala Lys Asp Phe Val Gln Trp Leu Met Asn Thr Cys
            20                  25                  30


<210>    41
<211>    29
<212>    PRT
<213>    Artificial Sequence

<220>
<223>    glucagon derivative


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    41
Tyr Xaa Gln Gly Thr Phe Cys Ser Asp Tyr Ser Lys Tyr Leu Asp Glu
1               5                   10                  15

Arg Arg Ala Lys Asp Phe Val Gln Trp Leu Met Asn Thr
            20                  25


<210>    42
<211>    29
<212>    PRT
<213>    Artificial Sequence

<220>
<223>    glucagon derivative


<220>
<221>    MISC_FEATURE
<222>    (2)
<223>    Xaa is aminoisobutyric acid (Aib)


<400>    42
Tyr Xaa Gln Gly Thr Phe Cys Ser Asp Tyr Ser Lys Tyr Leu Asp Ser
1               5                   10                  15

Arg Arg Ala Gln Asp Phe Val Gln Trp Leu Met Asn Thr
            20                  25


<210>    43
<211>    29
<212>    PRT

<213>    Artificial Sequence

<220>
<223>    glucagon derivative


<220>
<221>    MISC_FEATURE
<222>    (2)
<223>    Xaa is aminoisobutyric acid (Aib)


<400>    43
Tyr Xaa Gln Gly Thr Phe Thr Ser Asp Cys Ser Lys Tyr Leu Asp Ser
1               5                   10                  15

Arg Arg Ala Gln Asp Phe Val Gln Trp Leu Met Asn Thr
            20                  25


<210>    44
<211>    30
<212>    PRT
<213>    Artificial Sequence

<220>
<223>    glucagon derivative


<220>
<221>    MISC_FEATURE
<222>    (2)
<223>    Xaa is aminoisobutyric acid (Aib)


<400>    44
Tyr Xaa Gln Gly Thr Phe Thr Ser Asp Tyr Ser Lys Tyr Leu Asp Ser
1               5                   10                  15

Arg Arg Ala Gln Asp Phe Val Gln Trp Leu Met Asn Thr Cys
            20                  25                  30


<210>    45
<211>    30
<212>    PRT
<213>    Artificial Sequence

<220>
<223>    glucagon derivative


<220>
<221>    MISC_FEATURE
<222>    (1)
<223>    Xaa is histidine, desamino-histidyl, N-dimethyl-histidyl,
         beta-hydroxy imidazopropionyl, 4-imidazoacetyl, beta-carboxy
         imidazopropionyl, tryptophan, or tyrosine, or is absent


<220>
<221>    MISC_FEATURE
<222>    (2)

<223>    Xaa is alpha-methyl-glutamic acid, aminoisobutyric acid (Aib),
         D-alanine, glycine, Sar (N-methylglycine), serine, or D-serine

<220>
<221>    MISC_FEATURE
<222>    (7)
<223>    Xaa is threonine, valine, or cysteine

<220>
<221>    MISC_FEATURE
<222>    (10)
<223>    Xaa is tyrosine or cysteine

<220>
<221>    MISC_FEATURE
<222>    (12)
<223>    Xaa is lysine or cysteine

<220>
<221>    MISC_FEATURE
<222>    (13)
<223>    Xaa is tyrosine or cysteine

<220>
<221>    MISC_FEATURE
<222>    (14)
<223>    Xaa is leucine or cysteine

<220>
<221>    MISC_FEATURE
<222>    (15)
<223>    Xaa is aspartic acid, glutamic acid, or cysteine

<220>
<221>    MISC_FEATURE
<222>    (16)
<223>    Xaa is glutamic acid, aspartic acid, serine,
         alpha-methyl-glutamic acid, or cysteine, or is absent;

<220>
<221>    MISC_FEATURE
<222>    (17)
<223>    Xaa is aspartic acid, glutamine, glutamic acid, lysine, arginine,
         serine, cysteine, or valine, or is absent

<220>
<221>    MISC_FEATURE
<222>    (18)
<223>    Xaa is alanine, aspartic acid, glutamic acid, arginine, valine,
         or cysteine, or is absent

<220>
<221>    MISC_FEATURE

<222>    (19)
<223>    Xaa is alanine, arginine, serine, valine, or cysteine, or is
         absent

<220>
<221>    MISC_FEATURE
<222>    (20)
<223>    Xaa is lysine, histidine, glutamine, aspartic acid, lysine,
         arginine, alpha-methyl-glutamic acid, or cysteine, or is absent

<220>
<221>    MISC_FEATURE
<222>    (21)
<223>    Xaa is aspartic acid, glutamic acid, leucine, valine, or
         cysteine, or is absent

<220>
<221>    MISC_FEATURE
<222>    (23)
<223>    Xaa is isoleucine, valine, or arginine, or is absent

<220>
<221>    MISC_FEATURE
<222>    (24)
<223>    Xaa is valine, arginine, alanine, cysteine, glutamic acid,
         lysine, glutamine, alpha-methyl-glutamic acid, or leucine, or is
         absent;

<220>
<221>    MISC_FEATURE
<222>    (27)
<223>    Xaa is isoleucine, valine, alanine, lysine, methionine,
         glutamine, or arginine, or is absent

<220>
<221>    MISC_FEATURE
<222>    (28)
<223>    Xaa is glutamine, lysine, asparagine, or arginine, or is absent

<220>
<221>    MISC_FEATURE
<222>    (29)
<223>    Xaa is lysine, alanine, glycine, or threonine, or is absent

<220>
<221>    MISC_FEATURE
<222>    (30)
<223>    Xaa is cysteine, or is absent

<400>    45
Xaa Xaa Gln Gly Thr Phe Xaa Ser Asp Xaa Ser Xaa Xaa Xaa Xaa Xaa
1               5                   10                  15

Xaa Xaa Xaa Xaa Xaa Phe Xaa Xaa Trp Leu Xaa Xaa Xaa Xaa

20                  25                  30

<210>   46
<211>   30
<212>   PRT
<213>   Artificial Sequence

<220>
<223>   glucagon derivative

<220>
<221>   MISC_FEATURE
<222>   (2)
<223>   Xaa is aminoisobutyric acid (Aib)

<220>
<221>   MISC_FEATURE
<222>   (7)
<223>   Xaa is threonine, valine, or cysteine

<220>
<221>   MISC_FEATURE
<222>   (10)
<223>   Xaa is tyrosine or cysteine

<220>
<221>   MISC_FEATURE
<222>   (12)
<223>   Xaa is lysine or cysteine

<220>
<221>   MISC_FEATURE
<222>   (15)
<223>   Xaa is aspartic acid or cysteine

<220>
<221>   MISC_FEATURE
<222>   (16)
<223>   Xaa is glutamic acid or serine

<220>
<221>   MISC_FEATURE
<222>   (17)
<223>   Xaa is lysine or arginine

<220>
<221>   MISC_FEATURE
<222>   (20)
<223>   Xaa is glutamine or lysine

<220>
<221>   MISC_FEATURE
<222>   (21)

<223>    Xaa is aspartic acid or glutamic acid

<220>
<221>    MISC_FEATURE
<222>    (24)
<223>    Xaa is valine or glutamine

<220>
<221>    MISC_FEATURE
<222>    (30)
<223>    Xaa is cysteine or is absent

<400>    46
Tyr Xaa Gln Gly Thr Phe Xaa Ser Asp Xaa Ser Xaa Tyr Leu Xaa Xaa
1               5                   10                  15

Xaa Arg Ala Xaa Xaa Phe Val Xaa Trp Leu Met Asn Thr Xaa
            20                  25                  30

<210>    47
<211>    34
<212>    DNA
<213>    Artificial Sequence

<220>
<223>    forward primer

<400>    47
cagcgacacc gaccgtcccc ccgtacttaa ggcc                                34

<210>    48
<211>    32
<212>    DNA
<213>    Artificial Sequence

<220>
<223>    reverse primer

<400>    48
ctaaccgact ctcggggaag actgagctcg cc                                  32

<210>    49
<211>    29
<212>    PRT
<213>    Artificial Sequence

<220>
<223>    glucagon derivative

<220>
<221>    MISC_FEATURE
<222>    (2)
<223>    Xaa is aminoisobutyric acid

<400>    49
Tyr Xaa Gln Gly Thr Phe Thr Ser Asp Tyr Ser Cys Tyr Leu Asp Glu
1               5                   10                  15

Lys Arg Ala Lys Glu Phe Val Gln Trp Leu Met Asn Thr
            20                  25

<210>    50
<211>    29
<212>    PRT
<213>    Artificial Sequence

<220>
<223>    glucagon derivative

<220>
<221>    MISC_FEATURE
<222>    (2)
<223>    Xaa is aminoisobutyric acid

<400>    50
Tyr Xaa Gln Gly Thr Phe Thr Ser Asp Tyr Ser Lys Tyr Leu Cys Glu
1               5                   10                  15

Lys Arg Ala Gln Asp Phe Val Gln Trp Leu Met Asn Thr
            20                  25

## Description

### [Technical Field]

The present invention relates to a compound or substance having glucagon activity; or a combination including the compound or substance having glucagon activity and a compound or substance with therapeutic activity against a metabolic syndrome, and a therapeutic use thereof.

### [Background Art]

With economic development and changes in eating habits in recent years, the incidence of metabolic syndrome-related diseases including various disease such as obesity, hyperlipidemia, hypertension, arteriosclerosis, hyperinsulinemia, diabetes, or liver diseases is rapidly increasing. Although such diseases may occur independently, they generally occur in close relationship with one another and are accompanied by various symptoms in most cases.

Overweight and obesity are responsible for increasing blood pressure and cholesterol levels, thereby causing or worsening various diseases, such as cardiac diseases, diabetes, and arthritis. In addition, overweight and obesity are also becoming a major cause in the increased incidence of arteriosclerosis, hypertension, hyperlipidemia, or cardiac diseases in children or teenagers as well as in adults.

Obesity is a disease which is hard to treat because it is a complex disease associated with the mechanisms of appetite control and energy metabolism. Thus, for the treatment of obesity, both efforts of obese patients and a method capable of treating abnormal mechanisms associated with appetite control and energy metabolism are simultaneously required. Thus, efforts have been made to develop drugs for treating such abnormal mechanisms.

As a result of these efforts, drugs such as Rimonabant (Sanofi-Aventis), Sibutramin (Abbott), Contrave (Takeda), and Orlistat (Roche) have been developed to treat obesity, but these are disadvantageous in that serious adverse effects may occur or therapeutic effects on obesity may be negligible. For example, it has been reported that Rimonabant causes a side-effect of central nervous system disorder, Sibutramine and Contrave cause cardiovascular side-effects, and Orlistat shows only about 4 kg of weight loss when taken for one year.

Meanwhile, glucagon is produced by the pancreas when blood glucose levels drop as a result of other medications or diseases or hormone or enzyme deficiencies. Glucagon sends a signal for glycogen breakdown in the liver and a subsequent glucose release to have a role in increasing blood glucose levels to a normal range. In addition to the effect of increasing the blood glucose levels, glucagon suppresses appetite and activates hormone-sensitive lipase of adipocytes to facilitate lipolysis, thereby showing an anti-obesity effect. However, the use of glucagon as a therapeutic agent has been limited due to low solubility and precipitation at neutral pH.

One of the glucagon derivatives, glucagon-like peptide-1 (GLP-1), is under development as a therapeutic agent for treating hyperglycemia in patients with diabetes. GLP-1 has the functions of promoting insulin synthesis and secretion, inhibiting glucagon secretion, slowing gastric emptying, increasing glucose utilization, and inhibiting food intake.

Exendin-4, prepared from lizard venom and having an amino acid homology of about 50% with GLP-1, is also known to activate a GLP-1 receptor, thereby alleviating hyperglycemia in patients with diabetes (J Biol Chem. 1992 Apr 15; 267(11):7402-5.). However, anti-obesity drugs containing GLP-1 or exendin-4 are reported to cause side-effects such as vomiting and nausea.

In general, glucagon and a compound or substance with therapeutic activity against a metabolic syndrome are known to have opposite actions, and thus they have been used as therapeutic agents for different symptoms. Particularly, Korean Patent Laid-open Publication No. 10-2017-0023066 discloses a method of treating diabetes by supplementing insulin while antagonizing glucagon action by blocking a glucagon receptor with an antagonistic antigen protein.

That is, since insulin and glucagon serve as antagonists to each other in a living body, no pharmacotherapy of simultaneously administering them together has been reported to date. Meanwhile, when various therapeutic agents related to a metabolic syndrome are administered to a patient, there may be a risk of side-effects such as weight gain, overdose, and hypoglycemia.

### [Disclosure]

### [Technical Problem]

An object of the present invention is to provide a combination including a substance with activity to a glucagon receptor or a conjugate thereof and a compound or substance with therapeutic activity against a metabolic syndrome.

Another object of the present invention is to provide a combination, pharmaceutical composition, or kit including a substance with activity to a glucagon receptor or a conjugate thereof and a compound or substance with therapeutic activity against a metabolic syndrome, specifically a combination, pharmaceutical composition, or kit for preventing or treating a metabolic syndrome.

Another object of the present invention is to provide a method of preventing or treating a metabolic syndrome, the method including administering a substance with activity to a glucagon receptor or a conjugate thereof and a compound or substance with therapeutic activity against a metabolic syndrome to an individual in need thereof.

Another object of the present invention is to provide a composition including a peptide with activity to a glucagon receptor or a conjugate including the same, specifically a pharmaceutical composition for preventing or treating a metabolic syndrome, hypoglycemia, or congenital hyperinsulinism.

Another object of the present invention is to provide a kit including a peptide with activity to a glucagon receptor or a conjugate including the same, specifically a kit for preventing or treating a metabolic syndrome, hypoglycemia, or congenital hyperinsulinism.

Another object of the present invention is to provide a method of preventing or treating congenital hyperinsulinism, the method including administering a peptide with activity to the glucagon receptor, or a conjugate including the same, or a composition including the same to an individual in need thereof.

Another object of the present invention is to provide a use of a peptide with activity to the glucagon receptor, or a conjugate including the same, or a composition including the same for preparation of a medicament for preventing or treating congenital hyperinsulinism.

Another object of the present invention is to provide a method of preventing or treating hypoglycemia, the method including administering the peptide with activity to a glucagon receptor, or a conjugate including the same, or a composition including the same to an individual in need thereof.

Another object of the present invention is to provide a use of the peptide with activity to the glucagon receptor, or a conjugate including the same, or a composition including the same for preparation of a medicament for preventing or treating hypoglycemia.

Another object of the present invention is to provide a method of preventing or treating obesity, the method including administering a peptide or an isolated conjugate with activity to a glucagon receptor, or a composition including the same to an individual in need thereof.

Another object of the present invention is to provide a use of a peptide or an isolated conjugate with activity to a glucagon receptor, or the composition for preparation of a medicament (or pharmaceutical composition) for preventing or treating obesity.

Another object of the present invention is to provide a method of preventing or treating a metabolic syndrome, the method including administering a peptide or an isolated conjugate with activity to a glucagon receptor, or a composition including the same to an individual in need thereof.

Another object of the present invention is to provide a use of a peptide or an isolated conjugate with activity to a glucagon receptor, or the composition for preparation of a medicament (or pharmaceutical composition) for preventing or treating a metabolic syndrome.

### [Technical Solution]

An aspect of the present invention provides a combination including a substance with activity to a glucagon receptor or a conjugate thereof and a compound or substance with therapeutic activity against a metabolic syndrome.

In a specific embodiment, the present invention relates to a therapeutic use of co-administration of a substance with activity to a glucagon receptor or a conjugate thereof and a compound or substance with therapeutic activity against a metabolic syndrome.

In the specific embodiment, the present invention relates to a combination, pharmaceutical composition, or kit including a substance with activity to a glucagon receptor or a conjugate thereof and a compound or substance with therapeutic activity against a metabolic syndrome, specifically a combination, pharmaceutical composition, or kit for preventing or treating a metabolic syndrome.

In the specific embodiment(s), it is characterized in that the substance with activity to a glucagon receptor is native glucagon or an agonist or derivative thereof.

In the specific embodiment(s), the derivative of native glucagon is characterized in that one or more amino acids of the native glucagon are varied, and the variation is selected from the group consisting of substitution, addition, deletion, modification, and any combination thereof.

In the specific embodiment(s), it is characterized in that the substance with activity to a glucagon receptor is a peptide including an amino acid sequence of General Formula 1 below:

X1-X2-QGTF-X7-SD-X10-S-X12-X13-X14-X15-X16-X17-X18-X19-X20-X21-F-
X23-X24-W-L-X27-X28-X29-X30 (General Formula 1, SEQ ID NO: 45)

In General Formula 1 above,
X1 is histidine (H), desamino-histidyl, N-dimethyl-histidyl, β-hydroxy imidazopropionyl, 4-imidazoacetyl, β-carboxy imidazopropionyl, tryptophan (W), or tyrosine (Y), or is absent;
X2 is α-methyl-glutamic acid, aminoisobutyric acid (Aib), D-alanine, glycine (G), *N*-methylglycine (Sar), serine (S), or D-serine;
X7 is threonine (T), valine (V), or cysteine (C);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D), glutamic acid (E), or cysteine (C);
X16 is glutamic acid (E), aspartic acid (D), serine (S), α-methyl-glutamic acid, or cysteine (C), or is absent;
X17 is aspartic acid (D), glutamine (Q), glutamic acid (E), lysine (K), arginine (R), serine (S), cysteine (C), or valine (V), or is absent;
X18 is alanine (A), aspartic acid (D), glutamic acid (E), arginine (R), valine (V), or cysteine (C), or is absent;
X19 is alanine (A), arginine (R), serine (S), valine (V), or cysteine (C), or is absent;
X20 is lysine (K), histidine (H), glutamine (Q), aspartic acid (D), arginine (R), α-methyl-glutamic acid, or cysteine (C), or is absent;
X21 is aspartic acid (D), glutamic acid (E), leucine (L), valine (V), or cysteine (C), or is absent;
X23 is isoleucine (I), valine (V), or arginine (R), or is absent;
X24 is valine (V), arginine (R), alanine (A), cysteine (C), glutamic acid (E), lysine (K), glutamine (Q), α-methyl-glutamic acid, or leucine (L), or is absent;
X27 is isoleucine (I), valine (V), alanine (A), lysine (K), methionine (M), glutamine (Q), or arginine (R), or is absent;
X28 is glutamine (Q), lysine (K), asparagine (N), or arginine (R), or is absent;
X29 is lysine (K), alanine (A), glycine (G), or threonine (T), or is absent; and
X30 is cysteine (C) or is absent
(excluding a case where the amino acid sequence of General Formula 1 is identical to SEQ ID NO: 1).

In the specific embodiment(s), in General Formula 1 above, the peptide is characterized in that
X1 is histidine (H), tryptophan (W), or tyrosine (Y), or is absent;
X2 is serine (S) or aminoisobutyric acid (Aib);
X7 is threonine (T), valine (V), or cysteine (C);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D) or cysteine (C);
X16 is glutamic acid (E), serine (S), or cysteine (C);
X17 is aspartic acid (D), glutamic acid (E), lysine (K), arginine (R), serine (S), cysteine (C), or valine (V);
X18 is aspartic acid (D), glutamic acid (E), arginine (R), or cysteine (C);
X19 is alanine (A) or cysteine (C);
X20 is glutamine (Q), aspartic acid (D), lysine (K), or cysteine (C);
X21 is aspartic acid (D), glutamic acid (E), leucine (L), valine (V), or cysteine (C);
X23 is isoleucine (I), valine (V), or arginine (R);
X24 is valine (V), arginine (R), alanine (A), glutamic acid (E), lysine (K), glutamine (Q), or leucine (L);
X27 is isoleucine (I), valine (V), alanine (A), methionine (M), glutamine (Q), or arginine (R);
X28 is glutamine (Q), lysine (K), asparagine (N), or arginine (R);
X29 is threonine (T); and
X30 is cysteine (C) or is absent.

In the specific embodiment(s), in General Formula 1 above, the peptide is characterized in that
X1 is histidine (H), tryptophan (W), or tyrosine (Y);
X2 is serine (S) or aminoisobutyric acid (Aib);
X7 is cysteine (C), threonine (T), or valine (V);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D) or cysteine (C);
X16 is glutamic acid (E), serine (S), or cysteine (C);
X17 is glutamic acid (E), lysine (K), arginine (R), cysteine (C), or valine (V);
X18 is arginine (R) or cysteine (C);
X19 is alanine (A) or cysteine (C);
X20 is glutamine (Q) or lysine (K);
X21 is aspartic acid (D), glutamic acid (E), valine (V), or cysteine (C);
X23 is valine (V);
X24 is valine (V) or glutamine (Q);
X27 is methionine (M);
X28 is asparagine (N) or arginine (R);
X29 is threonine (T); and
X30 is cysteine (C) or is absent.

In the specific embodiment(s), in General Formula 1 above, the peptide is characterized in that
X1 is tyrosine (Y);
X2 is aminoisobutyric acid (Aib);
X7 is cysteine (C), threonine (T), or valine (V);
X10 is tyrosine (Y) or cysteine (C);
X12 is lysine (K);
X13 is tyrosine (Y) or cysteine (C);
X14 is leucine (L) or cysteine (C);
X15 is aspartic acid (D) or cysteine (C);
X16 is glutamic acid (E), serine (S), or cysteine (C);
X17 is lysine (K), arginine (R), cysteine (C), or valine (V);
X18 is arginine (R) or cysteine (C);
X19 is alanine (A) or cysteine (C);
X20 is glutamine (Q) or lysine (K);
X21 is aspartic acid (D), glutamic acid (E), or cysteine (C);
X23 is valine (V);
X24 is glutamine (Q);
X27 is methionine (M);
X28 is asparagine (N) or arginine (R);
X29 is threonine (T); and
X30 is cysteine (C) or is absent.

In the specific embodiment(s), in General Formula 1 above, the peptide is characterized in that
X1 is histidine (H), tryptophan (W), or tyrosine (Y), or is absent;
X2 is serine (S) or aminoisobutyric acid (Aib);
X7 is threonine (T), valine (V), or cysteine (C);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D) or cysteine (C);
X16 is glutamic acid (E), serine (S), or cysteine (C);
X17 is aspartic acid (D), glutamic acid (E), lysine (K), arginine (R), serine (S), cysteine (C), or valine (V);
X18 is aspartic acid (D), glutamic acid (E), arginine (R), or cysteine (C);
X19 is alanine (A) or cysteine (C);
X20 is glutamine (Q), aspartic acid (D), or lysine (K);
X21 is aspartic acid (D) or glutamic acid (E);
X23 is valine (V);
X24 is valine (V) or glutamine (Q);
X27 is isoleucine (I) or methionine (M);
X28 is asparagine (N) or arginine (R);
X29 is threonine (T); and
X30 is cysteine (C) or is absent.

In the specific embodiment(s), in General Formula 1 above, the peptide is characterized in that
X1 is tyrosine (Y);
X2 is aminoisobutyric acid (Aib);
X7 is threonine (T);
X10 is tyrosine (Y);
X12 is lysine (K);
X13 is tyrosine (Y);
X14 is leucine (L);
X15 is aspartic acid (D) or cysteine (C);
X16 is glutamic acid (E), serine (S), or cysteine (C);
X17 is lysine (K) or arginine (R);
X18 is arginine (R);
X19 is alanine (A);
X20 is glutamine (Q), cysteine (C), or lysine (K);
X21 is aspartic acid (D), cysteine (C), valine (V) or glutamic acid (E);
X23 is valine (V) or arginine (R);
X24 is glutamine (Q) or leucine (L);
X27 is methionine (M);
X28 is asparagine (N) or arginine (R);
X29 is threonine (T); and
X30 is absent.

In the specific embodiment(s), it is characterized in that the peptide includes an amino acid sequence of General Formula 2 below.

Y-Aib-QGTF-X7-SD-X10-S-X12-Y-L-X15-X16-X17-R-A-X20-X21-F-V-X24-W
-L-M-N-T-X30 (General Formula 2, SEQ ID NO: 46)

In General Formula 2 above,
X7 is threonine (T), valine (V), or cysteine (C);
X10 is tyrosine (Y) or cysteine (C);
X12 is lysine (K) or cysteine (C);
X15 is aspartic acid (D) or cysteine (C);
X16 is glutamic acid (E) or serine (S);
X17 is lysine (K) or arginine (R);
X20 is glutamine (Q) or lysine (K);
X21 is aspartic acid (D) or glutamic acid (E);
X24 is valine (V) or glutamine (Q); and
X30 is cysteine (C) or is absent.

In the specific embodiment(s), it is characterized in that the peptide has a pi value different from a pi value (6.8) of the native glucagon.

In the specific embodiment(s), the peptide is characterized in that each amino acid of at least one amino acid pair among amino acid pairs of X10 and X14, X12 and X16, X16 and X20, X17 and X21, X20 and X24, and X24 and X28 of General Formula 1 or 2 is substituted with glutamic acid or lysine capable of forming a ring.

In the specific embodiment(s), the peptide is characterized in that each amino acid of the amino acid pair of X12 and X16, the amino acid pair of X16 and X20, or the amino acid pair of X17 and X21 of General Formula 1 or 2 is substituted with glutamic acid or lysine capable of forming a ring.

In the specific embodiment(s), the peptide is characterized in that a ring is formed between amino acids of at least one amino acid pair, among the amino acid pairs of X10 and X14, X12 and X16, X16 and X20, X17 and X21, X20 and X24, and X24 and X28 in General Formula 1 above.

In the specific embodiment(s), the peptide is characterized in that the C-terminus of the peptide is amidated.

In the specific embodiment(s), the peptide is characterized in that the C-terminus of the peptide is not modified.

In the specific embodiment(s), it is characterized in that the peptide is a derivative of native glucagon capable of activating a glucagon receptor.

In the specific embodiment(s), it is characterized in that the peptide includes an amino acid sequence selected from the group consisting of SEQ ID NOS: 2 to 44.

In the specific embodiment(s), it is characterized in that the peptide includes an amino acid sequence selected from the group consisting of SEQ ID NOS: 12, 13, 15, and 36 to 44.

In the specific embodiment(s), it is characterized in that the peptide includes an amino acid sequence of SEQ ID NO: 37.

In the specific embodiment(s), the conjugate is characterized in that a biocompatible material is linked to the substance with activity to a glucagon receptor.

In the specific embodiment(s), it is characterized in that the substance with activity to a glucagon receptor is in the form of a peptide in which a biocompatible material is linked to a peptide site with activity to a glucagon receptor.

In the specific embodiment(s), it is characterized in that the biocompatible material is selected from the group consisting of a polymer, a fatty acid, cholesterol, albumin and fragments thereof, an albumin binding material, a polymer of a repeating unit with a particular amino acid sequence, an antibody, an antibody fragment, an FcRn binding material, *in vivo* connective tissue or a derivative thereof, a nucleotide, fibronectin, transferrin, a saccharide, heparin, and elastin.

In the specific embodiment(s), it is characterized in that the polymer is selected from the group consisting of polyethylene glycol, polypropylene glycol, an ethylene glycol-propylene glycol copolymer, polyoxyethylated polyol, polyvinylalcohol, a polysaccharide, dextran, polyvinylethylether, a biodegradable polymer, a lipid polymer, chitin, hyaluronic acid, oligonucleotide, and any combination thereof.

In the specific embodiment(s), it is characterized in that the FcRn binding material is a polypeptide including an immunoglobulin Fc region.

In the specific embodiment(s), it is characterized in that the substance with activity to a glucagon receptor is linked to the biocompatible material via a linker.

In the specific embodiment(s), it is characterized in that the linker is selected from the group consisting of a peptide, a fatty acid, a saccharide, a polymer, a low-molecular-weight compound, a nucleotide, and any combination thereof.

In the specific embodiment(s), it is characterized in that the polymer is selected from the group consisting of polyethylene glycol, polypropylene glycol, an ethylene glycol-propylene glycol copolymer, polyoxyethylated polyol, polyvinylalcohol, a polysaccharide, dextran, polyvinylethylether, a biodegradable polymer, a lipid polymer, chitin, hyaluronic acid, oligonucleotide, and any combination thereof.

In the specific embodiment(s), it is characterized in that the linker is polyethylene glycol.

In the specific embodiment(s), it is characterized in that the immunoglobulin Fc region is aglycosylated.

In the specific embodiment(s), it is characterized in that the immunoglobulin Fc region includes one selected from the group consisting of: (a) CH1 domain, CH2 domain, CH3 domain, and CH4 domain; (b) CH1 domain and CH2 domain; (c) CH1 domain and CH3 domain; (d) CH2 domain and CH3 domain; (e) a combination of one or more domains selected from the CH1 domain, CH2 domain, CH3 domain, and CH4 domain and an immunoglobulin hinge region or a part of the hinge region; and (f) a dimer of each domain of the heavy chain constant region and the light chain constant region.

In the specific embodiment(s), it is characterized in that the polypeptide including the immunoglobulin Fc region is in a dimeric form.

In the specific embodiment(s), it is characterized in that the immunoglobulin Fc region is a derivative of native Fc from which a region capable of forming a disulfide bond is deleted, from which some amino acids of the N-terminus are removed, to which a methionine residue of the N-terminus of native Fc is added, from which a complement-binding site is removed, or from which an antibody-dependent cell-mediated cytotoxicity (ADCC) region is removed.

In the specific embodiment(s), it is characterized in that the immunoglobulin Fc region is an Fc region derived from immunoglobulin selected from IgG, IgA, IgD, IgE, and IgM.

In the specific embodiment(s), it is characterized in that the immunoglobulin Fc region is an IgG4 Fc region.

In the specific embodiment(s), it is characterized in that the immunoglobulin Fc region is an aglycosylated Fc region derived from human IgG4.

In the specific embodiment(s), it is characterized in that the linker is linked to a cysteine residue of a peptide with activity to the glucagon receptor.

In the specific embodiment(s), it is characterized in that the linker of the conjugate is linked to both the peptide site with activity to a glucagon receptor and the biocompatible material via covalent bonds respectively formed by reactions between one end of the linker and an amine group or a thiol group of the biocompatible material and between the other end of the linker and an amine group or a thiol group of the peptide site.

In the specific embodiment(s), it is characterized in that the compound or substance with therapeutic activity against a metabolic syndrome is selected from the group consisting of an insulinotropic peptide, a glucagon-like peptide-1 (GLP-1) receptor agonist, a Leptin receptor agonist, a dipeptidyl peptidase-IV (DPP-IV) inhibitor, a Y5 receptor antagonist, a melanin-concentrating hormone (MCH) receptor antagonist, a Y2/4 receptor agonist, a melanocortin 3/4 (MC3/4) receptor agonist, a gastric/pancreatic lipase inhibitor, a 5-hydroxytryptamine receptor 2C (5HT2c), a G protein-coupled) agonist, a β3A receptor agonist, an amylin receptor agonist, a ghrelin antagonist, a ghrelin receptor antagonist, a peroxisome proliferator-activated receptor alpha (PPARα) agonist, a peroxisome proliferator-activated receptor delta (PPARδ) agonist, a farnesoid X receptor (FXR) agonist, an acetyl-CoA carboxylase inhibitor, peptide YY, cholecystokinin (CCK), Xenin, glicentin, obestatin, secretin, nesfatin, insulin, and a glucose-dependent insulinotropic peptide (GIP), biguanides, sulfonylureas, meglitinide, thiazolidinedione (TZD), a sodium-glucose cotransporter (SGLT2) inhibitor, and an α-glucosidase inhibitor.

In the specific embodiment(s), it is characterized in that the compound or substance with therapeutic activity against a metabolic syndrome is a glucagon-like peptide-1 (GLP-1) receptor agonist.

In the specific embodiment(s), it is characterized in that the glucagon-like peptide-1 (GLP-1) receptor agonist is selected from the group consisting of exenatide, lixisenatide, dulaglutide, liraglutide, semaglutide, albiglutide, and taspoglutide.

In the specific embodiment(s), it is characterized in that the DPP-4 inhibitor is selected from the group consisting of Sitagliptin, Vildagliiptin, Saxagliptin, Alogliptin, Linagliptin, Trayenta®, Anagliptin, Teneligliptin, Trelagliptin, migliptin, Omarigliptin, Evogliptin, and Dutogliptin; and the sodium-glucose cotransporter (SGLT2) inhibitor is characterized in that it is selected from the group consisting of Empagliflozin, Dapagliflozin, Canagliflozin, Remogliflozin, Remogliflozin Etabonate, Sergliflozin, Ipragliflozin, Tofogliflozin, Luseogliflozin, and Ertugliflozin.

In the specific embodiment(s), it is characterized in that the metabolic syndrome is selected from the group consisting of impaired glucose tolerance, hypercholesterolemia, dyslipidemia, obesity, diabetes, hypertension, non-alcoholic steatohepatitis (NASH), arteriosclerosis caused by dyslipidemia, atherosclerosis, arteriosclerosis, coronary heart disease, and stroke.

Another aspect of the present invention provides a pharmaceutical composition for preventing or treating a metabolic syndrome including a substance with activity to a glucagon receptor or a conjugate thereof and a compound or substance with therapeutic activity against a metabolic syndrome, or a combination including the same.

Another aspect of the present invention provides a pharmaceutical composition for preventing or treating a metabolic syndrome including the substance with activity to a glucagon receptor or a conjugate thereof, which can be co-administered with a compound or substance with therapeutic activity against a metabolic syndrome.

Another aspect of the present invention provides a kit for preventing or treating a metabolic syndrome including a substance with activity to a glucagon receptor or a conjugate thereof and a compound or substance with therapeutic activity against a metabolic syndrome; or a combination or composition including the same.

Another aspect of the present invention provides a method of preventing or treating a metabolic syndrome, the method including administering a substance with activity to a glucagon receptor or a conjugate thereof and a compound or substance with therapeutic activity against a metabolic syndrome to an individual in need thereof.

Another aspect of the present invention provides a composition including a peptide with activity to a glucagon receptor or a conjugate including the same, specifically a pharmaceutical composition for preventing or treating a metabolic syndrome, hypoglycemia, or congenital hyperinsulinism.

In a specific embodiment, it is characterized in that the metabolic syndrome includes at least one disease selected from the group consisting of impaired glucose tolerance, hypercholesterolemia, dyslipidemia, obesity, diabetes, hypertension, non-alcoholic steatohepatitis (NASH), arteriosclerosis caused by dyslipidemia, atherosclerosis, arteriosclerosis, coronary heart disease, and stroke.

In the specific embodiment, it is characterized in that the peptide with activity to a glucagon receptor is native glucagon or an agonist or derivative thereof.

It is characterized in that the derivative of native glucagon according to one of the specific embodiments is that where one or more amino acids of the native glucagon are varied, and the variation is selected from the group consisting of substitution, addition, deletion, modification, and any combination thereof.

In the pharmaceutical composition according to any one of the specific embodiments, it is characterized in that the peptide with activity to a glucagon receptor is a glucagon derivative peptide including an amino acid sequence of General Formula 1 below:

X1-X2-QGTF-X7-SD-X10-S-X12-X13-X14-X15-X16-X17-X18-X19-X20-X21-
F-X23-X24-W-L-X27-X28-X29-X30 (General Formula 1, SEQ ID NO: 45)

In General Formula 1 above,
X1 is histidine (H), desamino-histidyl, N-dimethyl-histidyl, β-hydroxy imidazopropionyl, 4-imidazoacetyl, β-carboxy imidazopropionyl, tryptophan (W), or tyrosine (Y), or is absent;
X2 is α-methyl-glutamic acid, aminoisobutyric acid (Aib), D-alanine, glycine (G), N-methylglycine (Sar), serine (S), or D-serine;
X7 is threonine (T), valine (V), or cysteine (C);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D), glutamic acid (E), or cysteine (C);
X16 is glutamic acid (E), aspartic acid (D), serine (S), α-methyl-glutamic acid, or cysteine (C), or is absent;
X17 is aspartic acid (D), glutamine (Q), glutamic acid (E), lysine (K), arginine (R), serine (S), cysteine (C), or valine (V), or is absent;
X18 is alanine (A), aspartic acid (D), glutamic acid (E), arginine (R), valine (V), or cysteine (C), or is absent;
X19 is alanine (A), arginine (R), serine (S), valine (V), or cysteine (C), or is absent;
X20 is lysine (K), histidine (H), glutamine (Q), aspartic acid (D), arginine (R), α-methyl-glutamic acid, or cysteine (C), or is absent;
X21 is aspartic acid (D), glutamic acid (E), leucine (L), valine (V), or cysteine (C), or is absent;
X23 is isoleucine (I), valine (V), or arginine (R), or is absent;
X24 is valine (V), arginine (R), alanine (A), cysteine (C), glutamic acid (E), lysine (K), glutamine (Q), α-methyl-glutamic acid, or leucine (L), or is absent;
X27 is isoleucine (I), valine (V), alanine (A), lysine (K), methionine (M), glutamine (Q), or arginine (R), or is absent;
X28 is glutamine (Q), lysine (K), asparagine (N), or arginine (R), or is absent;
X29 is lysine (K), alanine (A), glycine (G), or threonine (T), or is absent; and
X30 is cysteine (C) or is absent
(excluding a case where the amino acid sequence of General Formula 1 is identical to SEQ ID NO: 1).

In the specific embodiment(s), in General Formula 1 above, the peptide is characterized in that
X1 is histidine (H), tryptophan (W), or tyrosine (Y), or is absent;
X2 is serine (S) or aminoisobutyric acid (Aib);
X7 is threonine (T), valine (V), or cysteine (C);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D) or cysteine (C);
X16 is glutamic acid (E), serine (S), or cysteine (C);
X17 is aspartic acid (D), glutamic acid (E), lysine (K), arginine (R), serine (S), cysteine (C), or valine (V);
X18 is aspartic acid (D), glutamic acid (E), arginine (R), or cysteine (C);
X19 is alanine (A) or cysteine (C);
X20 is glutamine (Q), aspartic acid (D), lysine (K), or cysteine (C);
X21 is aspartic acid (D), glutamic acid (E), leucine (L), valine (V), or cysteine (C);
X23 is isoleucine (I), valine (V), or arginine (R);
X24 is valine (V), arginine (R), alanine (A), glutamic acid (E), lysine (K), glutamine (Q), or leucine (L);
X27 is isoleucine (I), valine (V), alanine (A), methionine (M), glutamine (Q), or arginine (R);
X28 is glutamine (Q), lysine (K), asparagine (N), or arginine (R);
X29 is threonine (T); and
X30 is cysteine (C) or is absent.

In the pharmaceutical composition according to any one of the specific embodiments, in General Formula 1 above, the peptide is characterized in that
X1 is histidine (H), tryptophan (W), or tyrosine (Y);
X2 is serine (S) or aminoisobutyric acid (Aib);
X7 is cysteine (C), threonine (T), or valine (V);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D) or cysteine (C);
X16 is glutamic acid (E), serine (S), or cysteine (C);
X17 is glutamic acid (E), lysine (K), arginine (R), cysteine (C), or valine (V);
X18 is arginine (R) or cysteine (C);
X19 is alanine (A) or cysteine (C);
X20 is glutamine (Q) or lysine (K);
X21 is aspartic acid (D), glutamic acid (E), valine (V), or cysteine (C);
X23 is valine (V);
X24 is valine (V) or glutamine (Q);
X27 is methionine (M);
X28 is asparagine (N) or arginine (R);
X29 is threonine (T); and
X30 is cysteine (C) or is absent.

In the pharmaceutical composition according to any one of the specific embodiments, in General Formula 1 above, the peptide is characterized in that
X1 is tyrosine (Y);
X2 is aminoisobutyric acid (Aib);
X7 is cysteine (C), threonine (T), or valine (V);
X10 is tyrosine (Y) or cysteine (C);
X12 is lysine (K);
X13 is tyrosine (Y) or cysteine (C);
X14 is leucine (L) or cysteine (C);
X15 is aspartic acid (D) or cysteine (C);
X16 is glutamic acid (E), serine (S), or cysteine (C);
X17 is lysine (K), arginine (R), cysteine (C), or valine (V);
X18 is arginine (R) or cysteine (C);
X19 is alanine (A) or cysteine (C);
X20 is glutamine (Q) or lysine (K);
X21 is aspartic acid (D), glutamic acid (E), or cysteine (C);
X23 is valine (V);
X24 is glutamine (Q);
X27 is methionine (M);
X28 is asparagine (N) or arginine (R);
X29 is threonine (T); and
X30 is cysteine (C) or is absent.

In the pharmaceutical composition according to any one of the specific embodiments, in General Formula 1 above, the peptide is characterized in that
X1 is histidine (H), tryptophan (W), or tyrosine (Y), or is absent;
X2 is serine (S) or aminoisobutyric acid (Aib);
X7 is threonine (T), valine (V), or cysteine (C);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D) or cysteine (C);
X16 is glutamic acid (E), serine (S), or cysteine (C);
X17 is aspartic acid (D), glutamic acid (E), lysine (K), arginine (R), serine (S), cysteine (C), or valine (V);
X18 is aspartic acid (D), glutamic acid (E), arginine (R), or cysteine (C);
X19 is alanine (A) or cysteine (C);
X20 is glutamine (Q), aspartic acid (D), or lysine (K);
X21 is aspartic acid (D) or glutamic acid (E);
X23 is valine (V);
X24 is valine (V) or glutamine (Q);
X27 is isoleucine (I) or methionine (M);
X28 is asparagine (N) or arginine (R);
X29 is threonine (T); and
X30 is cysteine (C) or is absent.

In the pharmaceutical composition according to any one of the specific embodiments, in General Formula 1 above, the peptide is characterized in that
X1 is tyrosine (Y);
X2 is aminoisobutyric acid (Aib);
X7 is threonine (T);
X10 is tyrosine (Y);
X12 is lysine (K);
X13 is tyrosine (Y);
X14 is leucine (L);
X15 is aspartic acid (D) or cysteine (C);
X16 is glutamic acid (E), serine (S), or cysteine (C);
X17 is lysine (K) or arginine (R);
X18 is arginine (R);
X19 is alanine (A);
X20 is glutamine (Q), cysteine (C), or lysine (K);
X21 is aspartic acid (D), cysteine (C), valine (V) or glutamic acid (E);
X23 is valine (V) or arginine (R);
X24 is glutamine (Q) or leucine (L);
X27 is methionine (M);
X28 is asparagine (N) or arginine (R);
X29 is threonine (T); and
X30 is absent.

In the pharmaceutical composition according to any one of the specific embodiments, it is characterized in that the peptide includes the amino acid sequence of General Formula 2 below:

Y-Aib-QGTF-X7-SD-X10-S-X12-Y-L-X15-X16-X17-R-A-X20-X21-F-V-X24-W
-L-M-N-T-X30 (General Formula 2, SEQ ID NO: 46)

In General Formula 2 above,
X7 is threonine (T), valine (V), or cysteine (C);
X10 is tyrosine (Y) or cysteine (C);
X12 is lysine (K) or cysteine (C);
X15 is aspartic acid (D) or cysteine (C);
X16 is glutamic acid (E) or serine (S);
X17 is lysine (K) or arginine (R);
X20 is glutamine (Q) or lysine (K);
X21 is aspartic acid (D) or glutamic acid (E);
X24 is valine (V) or glutamine (Q); and
X30 is cysteine (C) or is absent.

In the pharmaceutical composition according to any one of the specific embodiments, it is characterized in that the peptide has a pi value different from a pi value (6.8) of the native glucagon, for example, a pi value equal to or less than 6.5 and equal to or greater than 7.0.

In the pharmaceutical composition according to any one of the specific embodiments, the specific embodiment of the present invention is characterized in that each amino acid of at least one amino acid pair among amino acid pairs of X10 and X14, X12 and X16, X16 and X20, X17 and X21, X20 and X24, and X24 and X28 of General Formula 1 or 2 is substituted with glutamic acid or lysine capable of forming a ring.

In the pharmaceutical composition according to any one of the specific embodiments, the specific embodiment of the present invention is characterized in that each amino acid of the amino acid pair of X12 and X16, the amino acid pair of X16 and X20, or the amino acid pair of X17 and X21 of General Formula 1 or 2 is substituted with glutamic acid or lysine capable of forming a ring.

In the pharmaceutical composition according to any one of the specific embodiments, the specific embodiment of the present invention is characterized in that a ring (lactam ring) is formed between amino acids of at least one amino acid pair, among the amino acid pairs of X10 and X14, X12 and X16, X16 and X20, X17 and X21, X20 and X24, and X24 and X28 in General Formula 1 or 2 above.

In the pharmaceutical composition according to any one of the specific embodiments, the specific embodiment of the present invention is characterized in that X16 is glutamic acid and X20 is lysine in General Formula 1 or 2, and a lactam ring is formed between a side chain of X16 and a side chain of X20.

In the pharmaceutical composition according to any one of the specific embodiments, the peptide according to an embodiment is characterized in that the C-terminus of the peptide is amidated or not modified.

In the pharmaceutical composition according to any one of the specific embodiments, it is characterized in that the peptide is a derivative of native glucagon capable of activating a glucagon receptor.

In the pharmaceutical composition according to any one of the specific embodiments, it is characterized in that the peptide includes an amino acid sequence selected from the group consisting of SEQ ID NOS: 2 to 44.

In the pharmaceutical composition according to any one of the specific embodiments, it is characterized in that the peptide includes an amino acid sequence selected from the group consisting of SEQ ID NOS: 12, 13, 15, and 36 to 44.

In the pharmaceutical composition according to any one of the specific embodiments, it is characterized in that the peptide includes an amino acid sequence of SEQ ID NO: 37.

In the pharmaceutical composition according to any one of the specific embodiments, it is characterized in that the peptide is in the form of a long-acting conjugate in which a biocompatible material is linked to the peptide site, specifically the peptide site including the amino acid sequence of General Formula 1 or 2.

In the pharmaceutical composition according to any one of the specific embodiments, it is characterized in that the biocompatible material is selected from the group consisting of a polymer, a fatty acid, cholesterol, albumin and fragments thereof, an albumin binding material, a polymer of a repeating unit with a particular amino acid sequence, an antibody, an antibody fragment, an FcRn binding material, *in vivo* connective tissue or a derivative thereof, a nucleotide, fibronectin, transferrin, a saccharide, heparin, and elastin.

In the pharmaceutical composition according to any one of the specific embodiments, it is characterized in that the polymer is selected from the group consisting of polyethylene glycol, polypropylene glycol, an ethylene glycol-propylene glycol copolymer, polyoxyethylated polyol, polyvinylalcohol, a polysaccharide, dextran, polyvinylethylether, a biodegradable polymer, a lipid polymer, chitin, hyaluronic acid, oligonucleotide, and any combination thereof.

In the pharmaceutical composition according to any one of the specific embodiments, it is characterized in that the FcRn binding material is a polypeptide including an immunoglobulin Fc region.

In the pharmaceutical composition according to any one of the specific embodiments, it is characterized in that the peptide site is linked to the biocompatible material via the linker.

In the pharmaceutical composition according to any one of the specific embodiments, it is characterized in that the linker is selected from the group consisting of a peptide, a fatty acid, a saccharide, a polymer, a low-molecular-weight compound, a nucleotide, and any combination thereof.

In the pharmaceutical composition according to any one of the specific embodiments, it is characterized in that the polymer is selected from the group consisting of polyethylene glycol, polypropylene glycol, an ethylene glycol-propylene glycol copolymer, polyoxyethylated polyol, polyvinylalcohol, a polysaccharide, dextran, polyvinylethylether, a biodegradable polymer, a lipid polymer, chitin, hyaluronic acid, an oligonucleotide, and any combination thereof.

In the pharmaceutical composition according to any one of the specific embodiments, it is characterized in that the linker is polyethylene glycol.

In the pharmaceutical composition according to any one of the specific embodiments, it is characterized in that the immunoglobulin Fc region is aglycosylated.

In the pharmaceutical composition according to any one of the specific embodiments, it is characterized in that the immunoglobulin Fc region includes one selected from the group consisting of:
(a) CH1 domain, CH2 domain, CH3 domain, and CH4 domain;
(b) CH1 domain and CH2 domain;
(c) CH1 domain and CH3 domain;
(d) CH2 domain and CH3 domain;
(e) a combination of one or more domains selected from the CH1 domain, CH2 domain, CH3 domain, and CH4 domain and an immunoglobulin hinge region or a part of the hinge region; and
(f) a dimer of each domain of the heavy chain constant region and the light chain constant region.

In the pharmaceutical composition according to any one of the specific embodiments, it is characterized in that the polypeptide including the immunoglobulin Fc region is in a dimeric form.

In the pharmaceutical composition according to any one of the specific embodiments, it is characterized in that the immunoglobulin Fc region is a derivative of native Fc from which a region capable of forming a disulfide bond is deleted, from which some amino acids of the N-terminus are removed, to which a methionine residue of the N-terminus is added, from which a complement-binding site is removed, or from which an antibody-dependent cell-mediated cytotoxicity (ADCC) region is removed.

In the pharmaceutical composition according to any one of the specific embodiments, it is characterized in that the immunoglobulin Fc region is an FC region derived from immunoglobulin selected from the group consisting of IgG, IgA, IgD, IgE, and IgM.

In the pharmaceutical composition according to any one of the specific embodiments, it is characterized in that the immunoglobulin Fc region is an IgG4 Fc region.

In the pharmaceutical composition according to any one of the specific embodiments, it is characterized in that the immunoglobulin Fc region is an aglycosylated Fc region derived from human IgG4.

In the pharmaceutical composition according to any one of the specific embodiments, it is characterized in that the linker is linked to a cysteine residue of a peptide with activity to the glucagon receptor having an amino acid sequence of General Formula 1 or 2 above.

In the pharmaceutical composition according to any one of the specific embodiments, it is characterized in that the linker of the conjugate is linked to both the peptide site and the biocompatible material via covalent bonds respectively formed by reactions between one end of the linker and an amine group or a thiol group of the biocompatible material and between the other end of the linker and an amine group or a thiol group of the peptide site with activity to a glucagon receptor, *i.e*., the peptide site including the amino acid sequence of General Formula 1 or 2.

Another aspect of the present invention provides a pharmaceutical composition for preventing or treating hypoglycemia including a peptide with activity to a glucagon receptor or a conjugate including the same; and a pharmaceutically acceptable excipient.

In a specific embodiment, the present invention relates to a pharmaceutical composition for preventing or treating hypoglycemia including a peptide with activity to the glucagon receptor; or an isolated conjugate including a peptide site including a sequence identical to or including that of the peptide with activity to a glucagon receptor and a biocompatible material covalently linked to the peptide site.

Another aspect of the present invention provides a pharmaceutical composition for preventing or treating congenital hyperinsulinism including a peptide with activity to a glucagon receptor or a conjugate including the same; and a pharmaceutically acceptable excipient.

In a specific embodiment, the present invention relates to a pharmaceutical composition for preventing or treating congenital hyperinsulinism including a peptide with activity to the glucagon receptor; or an isolated conjugate including a peptide site including a sequence identical to or including that of the peptide with activity to a glucagon receptor and a biocompatible material covalently linked to the peptide site.

Another aspect of the present invention provides a pharmaceutical composition for preventing or treating obesity including a peptide with activity to a glucagon receptor or a conjugate including the same; and a pharmaceutically acceptable excipient.

In a specific embodiment, the present invention relates to a pharmaceutical composition for preventing or treating obesity including a peptide with activity to the glucagon receptor; or an isolated conjugate including a peptide site including a sequence identical to or including that of the peptide with activity to a glucagon receptor and a biocompatible material covalently linked to the peptide site.

Another aspect of the present invention provides a pharmaceutical composition for preventing or treating a metabolic syndrome including a peptide with activity to a glucagon receptor or a conjugate including the same; and a pharmaceutically acceptable excipient.

In a specific embodiment, the present invention relates to a pharmaceutical composition for preventing or treating a metabolic syndrome including a peptide with activity to the glucagon receptor; or an isolated conjugate including a peptide site including a sequence identical to or including that of the peptide with activity to a glucagon receptor and a biocompatible material covalently linked to the peptide site.

### [Advantageous Effects]

Administration of the peptide with activity to a glucagon receptor; or co-administration of a substance with activity to a glucagon receptor and a compound or substance with therapeutic activity against a metabolic syndrome according to the present invention may be effectively used in prevention or treatment of a metabolic syndrome, such as obesity, diabetes, and non-alcoholic steatohepatitis (NASH), congenital hyperinsulinism, and/or hypoglycemia.

### [Brief Description of Drawings]

FIG. 1 shows changes in body weight measured during 4 weeks of drug administration compared to body weight before administration.
FIG. 2(A) is a graph illustrating mesenteric fat mass after 4-week drug administration.
FIG. 2(B) is a graph illustrating changes in HOMA-IR, which is an index of insulin sensitivity, after 4-week drug administration.
FIG. 3 shows changes in blood glucose level during 4 weeks of drug administration.
FIG. 4 shows changes in body weight measured during 4 weeks of drug administration compared to body weight before administration.
FIG. 5(A) is a graph illustrating mesenteric fat mass after 4-week drug administration.
FIG. 5(B) is a graph illustrating changes in HOMA-IR, which is an index of insulin sensitivity, after 4-week drug administration.
FIG. 6 shows changes in blood glucose level during 4 weeks of drug administration.
FIG. 7 shows effects of drug administration on weight loss.
FIGS. 8(A) and 8(B) show decreases in fat and blood lipid level.
FIG. 9 shows changes in blood glucose level.

### [Best Mode]

Hereinafter, the present invention will be described in detail. Meanwhile, each of the descriptions and embodiments disclosed herein may be applied herein to describe different descriptions and embodiments. That is, all of the combinations of various factors disclosed herein belong to the scope of the present invention. Furthermore, the scope of the present invention should not be limited by the detailed descriptions provided hereinbelow.

Also, those skilled in the art will recognize or be able to ascertain, using no more than routine experimentation, many equivalents to specific embodiments of the present invention. Such equivalents are intended to be encompassed in the scope of the following claims.

Throughout the specification, not only the conventional one-letter and three-letter codes for naturally occurring amino acids, but also those three-letter codes generally allowed for other amino acids, such as α-aminoisobutyric acid (Aib) and N-methylglycine (Sar), are used. In addition, the amino acids mentioned herein are abbreviated according to the nomenclature rules of the IUPAC-IUB as follows.

| | |
|---|---|
| alanine A | arginine R |
| asparagine N | aspartic acid D |
| cysteine C | glutamic acid E |
| glutamine Q | glycine G |
| histidine H | isoleucine I |
| leucine L | lysine K |
| methionine M | phenylalanine F |
| proline P | serine S |
| threonine T | tryptophan W |
| tyrosine Y | valine V |

An aspect of the present invention provides a therapeutic use of co-administration of a substance with activity to a glucagon receptor or a conjugate thereof and a compound or substance with therapeutic activity against a metabolic syndrome.

Specifically, an aspect of the present invention provides a combination, pharmaceutical composition, or kit including a substance with activity to a glucagon receptor or a conjugate thereof and a compound or substance with therapeutic activity against a metabolic syndrome. In an embodiment, a combination, pharmaceutical composition, or kit for preventing or treating a metabolic syndrome is provided.

As used herein, the term "combination" is intended to be used in co-administration of the substance with activity to a glucagon receptor or a conjugate thereof and the compound or substance with therapeutic activity against a metabolic syndrome, and may be understood as having the same meaning as "combined use". The combination may be administered
a) as a mixture of (i) the substance with activity to a glucagon receptor or a conjugate thereof and (ii) the compound or substance with therapeutic activity against a metabolic syndrome; or
b) in separate forms of (i) the substance with activity to a glucagon receptor or a conjugate thereof and (ii) the compound or substance with therapeutic activity against a metabolic syndrome, without being limited thereto.

When the substance with activity to a glucagon receptor or a conjugate thereof and the compound or substance with therapeutic activity against a metabolic syndrome are administered in separate forms, the substance with activity to a glucagon receptor or a conjugate thereof and the compound or substance with therapeutic activity against a metabolic syndrome may be individually formulated and administered simultaneously, individually, sequentially, or in reverse order.

In the present invention, co-administration does not simply refer to concurrent administration of both the substance with activity to a glucagon receptor or a conjugate thereof and the compound or substance with therapeutic activity against a metabolic syndrome, but may be understood as administration in a dosage form wherein the substances are capable of acting together on an individual such that both components may perform functions thereof at a level identical to or higher than natural functions thereof. Thus, when the term "co-administration" is used, it should be understood that the components are administered simultaneously, individually, sequentially, or in reverse order. When administration is performed sequentially, in reverse order, or individually, the order of administration is not particularly limited, but an interval between two components should be set not to lose beneficial effects of co-administration.

As used herein, the term "composition including the combination" refers to the combination including the substance with activity to a glucagon receptor or a conjugate thereof and the compound or substance with therapeutic activity against a metabolic syndrome or a composition including the combination and having a therapeutic use thereof, without being limited thereto. For example, the composition may have a use for preventing or treating a metabolic syndrome, without being limited thereto. As used herein, the term "composition including the combination" may be used interchangeably with the "composition".

The composition including the combination according to the present invention is prepared for co-administration of the substance with activity to a glucagon receptor or a conjugate thereof and the compound or substance with therapeutic activity against a metabolic syndrome, and the substance with activity to a glucagon receptor or a conjugate thereof and the compound or substance with therapeutic activity against a metabolic syndrome may be formulated into one formulation or individually formulated. Specifically, the substance with activity to a glucagon receptor or a conjugate thereof and the compound or substance with therapeutic activity against a metabolic syndrome may be administered simultaneously, individually, sequentially, or in reverse order, without being limited thereto.

As used herein, the term "kit" may include the combination or composition according to the present invention for co-administration of the substance with activity to a glucagon receptor or a conjugate thereof and the compound or substance with therapeutic activity against a metabolic syndrome. Specifically, the kit according to the present invention may include a single formulation of the substance with activity to a glucagon receptor or a conjugate thereof and the compound or substance with therapeutic activity against a metabolic syndrome, or individual formulations of the substance with activity to a glucagon receptor or a conjugate thereof and the compound or substance with therapeutic activity against a metabolic syndrome, and may further include a substance required for co-administration of the two components, without being limited thereto.

The substance with activity to a glucagon receptor includes various substances with a significant level of activity to a glucagon receptor, e.g., a substance in the form of a compound or peptide.

Although not particularly limited thereto, the substance with a significant level of activity to a glucagon receptor may be not only native glucagon but also a substance exhibiting *in vitro* activity of 0.1% or more, 1% or more, 2% or more, 3% or more, 4% or more, 5% or more, 6% or more, 7% or more, 8% or more, 9% or more, 10% or more, 20% or more, 30% or more, 40% or more, 50% or more, 60% or more, 70% or more, 80% or more, 90% or more, or 100% or more to the glucagon receptor, compared to the native ligand (native glucagon) of the corresponding receptor.

For example, the substance with activity to a glucagon receptor may be native glucagon or an agonist or derivative thereof, without being limited thereto.

The glucagon derivative according to the present invention includes a peptide having at least one different amino acid in the amino acid sequence of the native glucagon, a peptide altered via modification in the native glucagon sequence, and mimics of the native glucagon capable of activating the glucagon receptor like the native glucagon. For example, the derivative of native glucagon is that where one or more amino acids of the native glucagon are varied, and the variation is selected from the group consisting of substitution, addition, deletion, modification, and any combination thereof, without being limited thereto.

The glucagon derivative may have an altered pi value different from that of native glucagon, thereby having improved physical properties. Also, the glucagon derivative may have improved solubility while maintaining the activity activating the glucagon receptor, without being limited thereto.

Also, the glucagon derivative may be glucagon which is not naturally occurring.

Meanwhile, the native glucagon may include the following amino acid sequence:

His-Ser-Gln-Gly-Thr-Phe-Thr-Ser-Asp-Tyr-Ser-Lys-Tyr-Leu-Asp-Ser-Arg-Arg-
Ala-Gln-Asp-Phe-Val-Gln-Trp-Leu-Met-Asn-Thr (SEQ ID NO: 1)

As used herein, the term "isoelectric point" or "pl" refers to the pH value at which a molecule such as a polypeptide or peptide has no net charge (0). In the case of a polypeptide with various charged functional groups, a sum of charges is 0 at the pl value. The total net charge of the polypeptide will be negative at higher pH than the pi, and the total net charge of the polypeptide will be positive at lower pH than the pl.

The pi values may be determined on an immobilized pH gradient gel consisting of polyacrylamide, starch, or agarose by isoelectric electrophoresis or may be estimated, for example, from an amino acid sequence using a p1/MW tool (http://expasy.org/tools/pi_tool.html; Gasteiger *et al*., 2003) in an ExPASy server.

As used herein, the term "altered pi" refers to a pi which is different from that of native glucagon due to the substitution of a part of the amino acid sequence of native glucagon with an amino acid residue having a negative charge or a positive charge, *i.e*., a reduced or increased pi value. The peptide with such an altered pl is a glucagon derivative and may exhibit improved solubility and/or high stability at neutral pH. However, the present invention is not limited thereto.

More specifically, the glucagon derivative may have an altered pi value, different from the pi value (6.8) of native glucagon, even more specifically a pi value of less than 6.8, specifically 6.7 or less, more specifically 6.5 or less, and exceeding 6.8, specifically 7 or more, more specifically 7.5 or more, but is not limited thereto, and any pl value different from that of native glucagon is within the scope of the present invention. In particular, any glucagon derivatives are within the scope of the present invention, as long as the glucagon derivatives aggregate at a lower level than native glucagon caused by improved solubility at neutral pH due to a pi value different from that of native glucagon.

More specifically, the glucagon derivative may have a pi value of 4 to 6.5 and/or 7 to 9.5, more specifically 7.5 to 9.5, even more specifically 8.0 to 9.3, without being limited thereto. In this case, since the glucagon derivative has a higher or lower pi value than native glucagon, improved solubility and higher stability may be obtained compared to native glucagon. However, the present invention is not limited thereto.

Specifically, a derivative of native glucagon may be modified by way of any one method of substitution, addition, deletion, and modification of some amino acids of the native glucagon or any combination thereof.

Examples of the glucagon derivative prepared by a combination of the above-described methods include a peptide including at least one different amino acid residue in the amino acid sequence compared to native glucagon, a delaminated amino acid residue at the N-terminus, and having the function of activating a glucagon receptor, without being limited thereto. The derivatives of native glucagon according to the present invention may be prepared by a combination of various methods for preparing the derivatives.

In addition, such variation for the preparation of the derivatives of native glucagon include all of the variations using L-type or D-type amino acids, and/or a non-native amino acid; and/or variation of a native sequence, for example, variation of a side chain functional group, intramolecular covalent bonding, such as ring formation between side chains, methylation, acylation, ubiquitination, phosphorylation, aminohexanation, biotinylation, and the like. Also, the variation includes substitution with a non-native compound.

In addition, the variation includes all of those where one or more amino acids are added to the amino and/or carboxy terminal of native glucagon.

The amino acids substituted or added may be not only 20 amino acids commonly found in human proteins but also atypical amino acids or those which do not occur naturally. Commercial sources of atypical amino acids may include Sigma-Aldrich, ChemPep Inc., and Genzyme Pharmaceuticals. The peptides including theses amino acids and typical peptide sequences may be synthesized and purchased from commercial suppliers, e.g., American Peptide Company, Bachem, or Anygen (Korea).

Amino acid derivatives may also be obtained in the same manner, for example, 4-imidazoacetic acid may be used.

Since glucagon has a pi value of above 7, it is insoluble in a solution having a physiological pH (pH 4 to pH 8) and tends to precipitate at neutral pH. In an aqueous solution with a pH of 3 or below, glucagon is dissolved at the initial stage but precipitates within one hour by forming a gel. Since the gelated glucagon mainly consists of β-sheet fibrils, administration of the precipitated glucagon via injection needle or intravenous infusion may block blood vessels, and thus glucagon is not suitable for use as an injection agent. In order to delay the precipitation process, acidic formulations (pH 2 to pH 4) are commonly used, and glucagon may be maintained in a relatively non-aggregated state for a short period of time. However, glucagon forms fibrils very rapidly at low pH, and thus theses acidic formulations need to be injected immediately upon preparation.

The glucagon derivative of the present invention includes those developed to have extended action profiles by altering the pi value of native glucagon via substitution of amino acid residues having negative charges and positive charges. These derivatives may have improved solubility and/or high stability at neutral pH compared to native glucagon due to the altered pi value.

In a specific embodiment, the glucagon derivative may be a peptide including an amino acid sequence of General Formula 1 below.

X1-X2-QGTF-X7-SD-X10-S-X12-X13-X14-X15-X16-X17-X18-X19-X20-X21-
F-X23-X24-W-L-X27-X28-X29-X30 (General Formula 1, SEQ ID NO: 45)

In General Formula 1 above,
X1 is histidine, desamino-histidyl, *N*-dimethyl-histidyl, β-hydroxy imidazopropionyl, 4-imidazoacetyl, β-carboxy imidazopropionyl, tryptophan, or tyrosine, or is absent;
X2 is α-methyl-glutamic acid, aminoisobutyric acid (Aib), D-alanine, glycine, N-methylglycine (Sar), serine, or D-serine;
X7 is threonine, valine, or cysteine;
X10 is tyrosine or cysteine;
X12 is lysine or cysteine;
X13 is tyrosine or cysteine;
X14 is leucine or cysteine;
X15 is aspartic acid, glutamic acid or cysteine;
X16 is glutamic acid, aspartic acid, serine, α-methyl-glutamic acid, or cysteine, or is absent;
X17 is aspartic acid, glutamine, glutamic acid, lysine, arginine, serine, cysteine, or valine, or is absent;
X18 is alanine, aspartic acid, glutamic acid, arginine, valine, or cysteine, or is absent;
X19 is alanine, arginine, serine, valine, or cysteine, or is absent;
X20 is lysine, histidine, glutamine, aspartic acid, arginine, α-methyl-glutamic acid, or cysteine, or is absent;
X21 is aspartic acid, glutamic acid, leucine, valine, or cysteine, or is absent;
X23 is isoleucine, valine, or arginine, or is absent;
X24 is valine, arginine, alanine, cysteine, glutamic acid, lysine, glutamine, α-methyl-glutamic acid, or leucine, or is absent;
X27 is isoleucine, valine, alanine, lysine, methionine, glutamine, or arginine, or is absent;
X28 is glutamine, lysine, asparagine, or arginine, or is absent;
X29 is lysine, alanine, glycine, or threonine, or is absent; and
X30 is cysteine, or is absent
(excluding a case where the amino acid sequence of General Formula 1 is identical to SEQ ID NO: 1).

More specifically, in General Formula 1 above,
X1 is histidine, tryptophan, or tyrosine, or is absent;
X2 is serine or aminoisobutyric acid (Aib);
X7 is threonine, valine, or cysteine;
X10 is tyrosine or cysteine;
X12 is lysine or cysteine;
X13 is tyrosine or cysteine;
X14 is leucine or cysteine;
X15 is aspartic acid or cysteine;
X16 is glutamic acid, serine, or cysteine;
X17 is aspartic acid, glutamic acid, lysine, arginine, serine, cysteine, or valine;
X18 is aspartic acid, glutamic acid, arginine, or cysteine;
X19 is alanine or cysteine;
X20 is glutamine, aspartic acid, lysine, or cysteine;
X21 is aspartic acid, glutamic acid, leucine, valine, or cysteine;
X23 is isoleucine, valine or arginine;
X24 is valine, arginine, alanine, glutamic acid, lysine, glutamine, or leucine;
X27 is isoleucine, valine, alanine, methionine, glutamine or arginine;
X28 is glutamine, lysine, asparagine or arginine;
X29 is threonine; and
X30 is cysteine, or is absent (excluding a case where the amino acid sequence of General Formula 1 is identical to SEQ ID NO: 1).

Examples of the peptide include a peptide including an amino acid sequence selected from the group consisting of SEQ ID NOS: 2 to 44, specifically (essentially) consisting of an amino acid sequence selected from the group consisting of SEQ ID NOS: 2 to 44, without being limited thereto.

Also, although described as "a peptide consisting of a particular SEQ ID NO:" in the present invention, such description does not exclude a mutation that may occurring naturally or by addition of a meaningless sequence upstream or downstream of the amino acid sequence of the SEQ ID NO, or a silent mutation thereof, as long as the peptide has an activity identical or equivalent to that of the peptide consisting of the amino acid sequence, and even when the sequence addition or mutation is present, it obviously belongs to the scope of the present invention.

Descriptions provided above may also be applied to other specific embodiments or aspects of the present invention, without being limited thereto.

Specifically, in General Formula 1 above,
X1 is histidine, tryptophan, or tyrosine;
X2 is serine or aminoisobutyric acid (Aib);
X7 is cysteine, threonine, or valine;
X10 is tyrosine or cysteine;
X12 is lysine or cysteine;
X13 is tyrosine or cysteine;
X14 is leucine or cysteine;
X15 is aspartic acid or cysteine;
X16 is glutamic acid, serine, or cysteine;
X17 is glutamic acid, lysine, arginine, cysteine, or valine;
X18 is arginine or cysteine;
X19 is alanine or cysteine;
X20 is glutamine or lysine;
X21 is aspartic acid, glutamic acid, valine, or cysteine;
X23 is valine;
X24 is valine or glutamine;
X27 is methionine;
X28 is asparagine or arginine;
X29 is threonine; and
X30 is cysteine, or is absent.

Examples of the peptide may include a peptide including an amino acid sequence selected from the group consisting of SEQ ID NOS: 3, 11 to 17, 19 to 27, 29, 31, 33, and 35 to 44, specifically a peptide (essentially) consisting of an amino acid sequence selected from the group consisting of SEQ ID NOS: 3, 11 to 17, 19 to 27, 29, 31, 33, and 35 to 44, without being limited thereto.

Specifically, in General Formula 1 above,
X1 is tyrosine;
X2 is aminoisobutyric acid (Aib);
X7 is cysteine, threonine, or valine;
X10 is tyrosine or cysteine;
X12 is lysine;
X13 is tyrosine or cysteine;
X14 is leucine or cysteine;
X15 is aspartic acid or cysteine;
X16 is glutamic acid, serine, or cysteine;
X17 is lysine, arginine, cysteine, or valine;
X18 is arginine or cysteine;
X19 is alanine or cysteine;
X20 is glutamine or lysine;
X21 is aspartic acid, glutamic acid, or cysteine;
X23 is valine;
X24 is glutamine;
X27 is methionine;
X28 is asparagine or arginine;
X29 is threonine; and
X30 is cysteine, or is absent (excluding a case where the amino acid sequence of General Formula 1 is identical to SEQ ID NO: 1).

Examples of the peptide may include a peptide including an amino acid sequence selected from the group consisting of SEQ ID NOS: 12, 14, 17, 19 to 25, 27, 29, 33, 35 to 38, 40 to 42, and 44, specifically a peptide (essentially) consisting of an amino acid sequence selected from the group consisting of SEQ ID NOS: 12, 14, 17, 19 to 25, 27, 29, 33, 35 to 38, 40 to 42, and 44, without being limited thereto.

Specifically, in General Formula 1 above,
X1 is histidine, tryptophan, or tyrosine, or is absent;
X2 is serine or aminoisobutyric acid (Aib);
X7 is threonine, valine, or cysteine;
X10 is tyrosine or cysteine;
X12 is lysine or cysteine;
X13 is tyrosine or cysteine;
X14 is leucine or cysteine;
X15 is aspartic acid or cysteine;
X16 is glutamic acid, serine, or cysteine;
X17 is aspartic acid, glutamic acid, lysine, arginine, serine, cysteine, or valine;
X18 is aspartic acid, glutamic acid, arginine, or cysteine;
X19 is alanine or cysteine;
X20 is glutamine, aspartic acid, or lysine;
X21 is aspartic acid or glutamic acid;
X23 is valine;
X24 is valine or glutamine;
X27 is isoleucine or methionine;
X28 is asparagine or arginine;
X29 is threonine; and
X30 is cysteine, or is absent (excluding a case where the amino acid sequence of General Formula 1 is identical to SEQ ID NO: 1).

Examples of the peptide may include a peptide including an amino acid sequence selected from the group consisting of SEQ ID NOS: 2 to 13, 15, 17, 20 to 24, 26 to 30, and 32 to 44, specifically a peptide (essentially) consisting of an amino acid sequence selected from the group consisting of SEQ ID NOS: 2 to 13, 15, 17, 20 to 24, 26 to 30, and 32 to 44, without being limited thereto.

Specifically, in General Formula 1 above,
X1 is tyrosine;
X2 is aminoisobutyric acid (Aib);
X7 is threonine;
X10 is tyrosine;
X12 is lysine;
X13 is tyrosine;
X14 is leucine;
X15 is aspartic acid or cysteine;
X16 is glutamic acid, serine, or cysteine;
X17 is lysine or arginine;
X18 is arginine;
X19 is alanine;
X20 is glutamine, cysteine, or lysine;
X21 is aspartic acid, cysteine, valine, or glutamic acid;
X23 is valine or arginine;
X24 is glutamine or leucine;
X27 is methionine;
X28 is asparagine or arginine;
X29 is threonine; and
X30 is absent.

Examples of the peptide may include a peptide including an amino acid sequence selected from the group consisting of SEQ ID NOS: 14, 16, 18, 19, 25, and 31, specifically a peptide (essentially) consisting of an amino acid sequence selected from the group consisting of SEQ ID NOS: 14, 16, 18, 19, 25, and 31, without being limited thereto.

More specifically, the peptide may be a peptide including an amino acid sequence of General Formula 2 below:

Y-Aib-QGTF-X7-SD-X10-S-X12-Y-L-X15-X16-X17-R-A-X20-X21-F-V-X24-W
-L-M-N-T-X30 (General Formula 2, SEQ ID NO: 46)

In General Formula 2 above,
X7 is threonine, valine, or cysteine;
X10 is tyrosine or cysteine;
X12 is lysine or cysteine;
X15 is aspartic acid or cysteine;
X16 is glutamic acid or serine;
X17 is lysine or arginine;
X20 is glutamine or lysine;
X21 is aspartic acid or glutamic acid;
X24 is valine or glutamine; and
X30 is cysteine, or is absent.

Examples of the peptide may include a peptide including an amino acid sequence selected from the group consisting of SEQ ID NOS: 12, 13, 15, and 36 to 44, specifically a peptide (essentially) consisting of an amino acid sequence selected from the group consisting of SEQ ID NOS: 12, 13, 15, and 36 to 44, without being limited thereto. Even more specifically, the peptide may include a peptide including an amino acid sequence of SEQ ID NOS: 12, 20, or 37 or (essentially) consisting of the amino acid sequence. However, the present invention is not limited thereto.

Specifically, in General Formula 2 above,
X7 is threonine, valine, or cysteine;
X10 is tyrosine or cysteine;
X12 is lysine;
X15 is aspartic acid;
X16 is glutamic acid or serine;
X17 is lysine or arginine;
X20 is glutamine or lysine;
X21 is aspartic acid or glutamic acid;
X24 is glutamine; and
X30 is cysteine, or is absent, without being limited thereto.

Examples of the peptide may include a peptide including an amino acid sequence selected from the group consisting of SEQ ID NOS: 12, 36 to 38, 40 to 42, and 44, specifically a peptide (essentially) consisting of an amino acid sequence selected from the group consisting of SEQ ID NOS: 12, 36 to 38, 40 to 42, and 44, without being limited thereto.

However, among the isolated peptides, the peptides corresponding to SEQ ID NOS: 2 to 11, 14, 16 to 35, 49, and 50, specifically SEQ ID NOS: 19, 33, 49, and 50 may have a combination out of the claimed range, without being limited thereto, and all peptides disclosed in the claims are within the scope of the present invention unless otherwise stated in the claims.

The above-described glucagon derivative may include an intramolecular bridge (e.g., covalent crosslinking or non-covalent crosslinking), and specifically, is in a form including a ring. For example, the glucagon derivative may be in a form where a ring is formed between the 16^{th} and 20^{th} amino acids of the peptide, without being limited thereto.

Non-limiting examples of the ring may include a lactam bridge (or a lactam ring).

In addition, the glucagon derivative includes all of those modified to include a ring or include an amino acid capable of forming a ring at a target position.

The ring may be formed between amino acid side chains in the glucagon derivative, e.g., a lactam ring may be formed between a side chain of lysine and a side chain of glutamic acid, without being limited thereto.

For example, in the peptide including the amino acid sequence of General Formula 1 or 2, each amino acid of at least one amino acid pair among amino acid pairs of X10 and X14, X12 and X16, X16 and X20, X17 and X21, X20 and X24, and X24 and X28 of General Formula 1 or 2 may be substituted with glutamic acid or lysine, without being limited thereto. In the Xn (where n is a natural number), n indicates a site of the amino acid from the N-terminus of the amino acid sequence.

Also, in the peptide including the amino acid sequence of General Formula 1 or 2, each amino acid of the amino acid pair of X12 and X16, the amino acid pair of X16 and X20, or the amino acid pair of X17 and X21 may be substituted with glutamic acid or lysine capable of forming a ring, without being limited thereto.

Also, in General Formula 1 or 2 above, a ring (e.g., lactam ring) may be formed between amino acids of at least one amino acid pair, among the amino acid pairs of X10 and X14, X12 and X16, X16 and X20, X17 and X21, X20 and X24, and X24 and X28, without being limited thereto.

Also, in General Formula 1 or 2 above, X16 may be glutamic acid, and X20 may be lysine, and a lactam ring may be formed between a side chain of X16 and a side chain of X20, without being limited thereto.

In addition, although the peptide according to the present invention may be those whose N-terminus and/or C-terminus are not modified, any peptide in the form of a variant where the N-terminus and/or C-terminus is chemically modified or protected by organic groups or amino acids may be added to the termini of the peptide for protection from proteases *in vivo* while increasing stability thereof, and may also be within the peptide of the present invention. When the C-terminus is not modified, the terminus of the peptide according to the present invention includes a carboxyl group, without being limited thereto.

In particular, since the N- and C-termini of chemically synthesized peptides are electrically charged, the N-terminus may be acetylated and/or the C-terminus may be amidated to remove the charges, but the embodiment is not limited thereto.

Unless otherwise stated, detailed descriptions about the "peptide" of the present invention or the "conjugate" in which the peptide is covalently linked to a biocompatible material disclosed in the specification or techniques of the claims may be applied not only to the peptide or conjugate but also a salt of the peptide or conjugate (*e.g*., a pharmaceutically acceptable salt of the peptide), or a solvate form thereof. Thus, although only "peptide" or "conjugate" is described in the specification, descriptions thereof may also be applied to particular salts thereof, particular solvates thereof, and solvates of the particular salts. Such salts may be, for example, in the form of a pharmaceutically acceptable salt. Types of the salts are not particularly limited. However, salts may be in a safe and effective form in mammals, without being limited thereto.

The term, "pharmaceutically acceptable" refers to a substance that may be effectively used for the intended use within the scope of a pharmaco-medical decision without inducing excessive toxicity, irritation, allergic responses, and the like.

As used herein, the term "pharmaceutically acceptable salt" refers to a salt derived from a pharmaceutically acceptable inorganic acid, organic acid, or base. Examples of a suitable acid may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, and benzenesulfonic acid. Examples of the salt derived from a suitable base may include alkali metals such as sodium and potassium, alkaline earth metals such as magnesium, and ammonium.

In addition, as used herein, the term "solvate" refers to a complex of the peptide, conjugate, or a salt thereof according to the present invention and molecules of a solvent.

In addition, the glucagon derivative peptide of the present invention may be synthesized according to the length by way of a method well known in the art, e.g., by an automatic peptide synthesizer, and may be produced by way of genetic engineering technology.

Specifically, the peptide of the present invention may be prepared by way of a standard synthesis method, a recombinant expression system, or any other method known in the art. Thus, the glucagon derivative according to the present invention may be synthesized by way of a plurality of methods including the following methods:
(a) a method of synthesizing a peptide in a stepwise or fragment assembling manner by way of a solid-phase or liquid-phase method, followed by isolation and purification of a final peptide product;
(b) a method of expressing a nucleic acid construct encoding a peptide in a host cell and recovering an expression product from a host cell culture;
(c) a method of performing *in vitro* cell-free expression of a nucleic acid construct encoding a peptide and recovering an expression product therefrom; or
   a method of obtaining peptide fragments by way of any combination of the methods (a), (b), and (c), obtaining the peptide by linking the peptide fragments, and then recovering the peptide.

In a specific embodiment, desired glucagon derivatives may be produced by preparing a fusion gene encoding a fusion protein including a fusion partner and a glucagon derivative via genetic modification, transforming a host cell with the fusion gene, expressing the fusion protein, and cleaving and isolating the glucagon derivative from the fusion protein by using a protease or compound. To this end, for example, a DNA sequence encoding an amino acid residue cleaved by a protease such as Factor Xa or enterokinase or a compound such as CNBr or hydroxylamine may be inserted between polynucleotides respectively encoding the fusion partner and the glucagon derivative.

In a more specific embodiment, the substance with activity to a glucagon receptor (*e.g.*, a glucagon derivative, for example, a peptide including an amino acid sequence of General Formula 1 or 2 above) may be in the form of a long-acting conjugate in which the substance is liked to a biocompatible material capable of increasing *in vivo* half-life, without being limited thereto. The biocompatible material may be used interchangeably with a carrier.

Specifically, in the conjugate, the substance with activity to a glucagon receptor is in the form of a peptide, and the biocompatible material may be linked to the peptide site with activity to a glucagon receptor therein, without being limited thereto.

More specifically, the conjugate includes the peptide site and the biocompatible material covalently linked to the peptide site, wherein the peptide site may have a sequence identical to or including the amino acid sequence of General Formula 1 or 2.

As used herein, the term "long-acting conjugate" refers to a conjugate having a structure including a biologically active substance (*e.g*., a substance with activity to a glucagon receptor or insulinotropic peptide) linked to a biocompatible material or carrier and exhibiting an extended duration of increased effects (*e.g.*, extending *in vivo* half-life) compared with the biologically active substance to which the biocompatible material or carrier is not linked. In the long-acting conjugate, the biocompatible material or carrier may be covalently linked to the biologically active substance, without being limited thereto.

In a specific embodiment of the present invention, the conjugate of the glucagon derivative may have extended duration of increased effects compared with native glucagon or glucagon derivatives to which the carrier is not linked.

As used herein, the term, "biocompatible material" refers to a material linked to the biologically active substance (*e.g*., the substance with activity to a glucagon receptor or insulinotropic peptide) to extend the duration of effects of the biologically active substance compared with the biologically active substance to which the biocompatible material or carrier is not linked. The biocompatible material may be covalently linked to the biologically active substance, without being limited thereto.

Examples of the biocompatible material may be a polymer, a fatty acid, cholesterol, albumin and fragments thereof, an albumin binding material, a polymer of a repeating unit with a particular amino acid sequence, an antibody, an antibody fragment, an FcRn binding material, *in vivo* connective tissue or a derivative thereof, a nucleotide, fibronectin, transferrin, a saccharide, heparin, or elastin, without being limited thereto.

Examples of the polymer may be a polymer selected from the group consisting of polyethylene glycol (PEG), polypropylene glycol, an ethylene glycol-propylene glycol copolymer, polyoxyethylated polyol, polyvinylalcohol, a polysaccharide, dextran, polyvinylethylether, a biodegradable polymer, a lipid polymer, chitin, hyaluronic acid, an oligonucleotide, and any combination thereof, without being limited thereto.

The polyethylene glycol is a term including all of an ethylene glycol homopolymer, a PEG copolymer, or a monomethyl-substituted PEG polymer (mPEG), without being limited thereto.

Also, the biocompatible material includes a poly-amino acid such as poly-lysine, poly-aspartic acid, and poly-glutamic acid, without being limited thereto.

In addition, the fatty acid may have a binding affinity for albumin in a living body, without being limited thereto.

More specifically, the FcRn binding material may be an immunoglobulin Fc region, even more specifically an IgG Fc region, without being limited thereto.

At least one amino acid side chain within the substance with activity to a glucagon receptor of the present invention may be attached to the biocompatible material in order to increase *in vivo* solubility and/or half-life, and/or increase bioavailability thereof. These modifications may reduce the clearance of therapeutic proteins and peptides.

The biocompatible material may be soluble (amphipathic or hydrophilic), non-toxic, and/or pharmaceutically acceptable.

It is a known fact to a skilled person in the art that the thus-modified glucagon derivative would have a superior therapeutic effect compared to native glucagon. Thus, variants of the above-described glucagon derivative are also within the scope of the glucagon derivative of the present invention.

The biocompatible material may be linked to the substance with activity to a glucagon receptor directly or via a linker. The biocompatible material may be covalently linked to the substance with activity to a glucagon receptor directly or via a linker.

Specifically, the linker may be a peptidyl linker or non-peptidyl linker.

The peptidyl linker may include one or more amino acids, *e.g*., 1 to 1000 amino acids, but is not limited thereto. Various peptide linkers known in the art may be used in the present invention and may include, for example, [GS]ₓ linker, [GGGS]ₓ linker, and [GGGGS]ₓ linker, where x is a natural number of 1 or greater. However, the present invention is not limited thereto.

In the present invention, the "non-peptidyl linker" includes a biocompatible polymer composed of two or more repeating units linked to each other. The repeating units are linked to each other via any covalent bond other than a peptide bond. This non-peptidyl linker may be a component constituting the conjugate of the present invention.

In the present invention, the non-peptidyl linker may be used interchangeably with a non-peptidyl polymer.

In a specific embodiment, the biocompatible material may be covalently linked to the peptide via the non-peptidyl linker including reactive groups at both ends respectively linked to the biocompatible material (specifically immunoglobulin Fc region) and a peptide drug.

Specifically, the non-peptidyl linker may be selected from the group consisting of a fatty acid, a saccharide, a polymer, a low-molecular-weight compound, a nucleotide, and any combination thereof.

Although not particularly limited thereto, the polymer of the present invention may have a molecular weight greater than 0 kDa and equal to or less than about 100 kDa, specifically in the range of about 1 kDa to about 100 kDa, more specifically about 1 kDa to about 20 kDa, without being limited thereto.

Although not particularly limited thereto, the non-peptidyl linker may be selected from the group consisting of a biodegradable polymer such as polyethylene glycol, polypropylene glycol, an ethylene glycol-propylene glycol copolymer, polyoxyethylated polyol, polyvinylalcohol, a polysaccharide, dextran, polyvinylethylether, polylactic acid (PLA), and polylactic-glycolic acid (PLGA); a lipid polymer; chitin; hyaluronic acid; oligonucleotide; and any combination thereof.

In a more specific embodiment, the non-peptidyl polymer may be polyethylene glycol, without being limited thereto. Also, derivatives thereof known in the art and derivatives that may be easily prepared by way of techniques known in the art also fall within the scope of the present invention.

The non-peptidyl polymer available in the present invention is not limited as long as it has *in vivo* resistance to a protease. A molecular weight of the non-peptidyl polymer may be greater than 0 kDa and equal to or less than about 100 kDa, specifically in the range of about 1 kDa to 100 kDa, more specifically about 1 kDa to about 20 kDa, without being limited thereto. In addition, the non-peptidyl linker according to the present invention linked to the polypeptide including the immunoglobulin Fc region may be not only a polymer of one type but also a combination different types of polymers.

As used herein, the term "about" refers to a range including all of ±0.5, ±0.4, ±0.3, ±0.2, ±0.1, or the like and includes all values equivalent to those which come immediately after the term "about" or those in a similar range, without being limited thereto.

Specifically, the linker may be linked to both the peptide site and the biocompatible material via covalent bonds respectively formed by reactions between one end of the linker and an amine or thiol group of the biocompatible material and between the other end of the linker and an amine or thiol group of the peptide site, specifically the amino acid sequence of General Formula 1 or 2.

In a specific embodiment, one end of the non-peptidyl linker may be covalently linked to an amine group or a thiol group of the immunoglobulin Fc region, and the other end of the linker may be covalently linked to an amine group or a thiol group of the glucagon derivative. Specifically, the non-peptidyl polymer may include reactive groups linked to the biocompatible material, specifically the immunoglobulin Fc region, and the glucagon derivative at both ends. For example, the reactive groups may form covalent bonds to the biocompatible material and the glucagon derivative via reaction with an amine group of the N-terminus or lysine or a thiol group of cysteine of the glucagon derivative and reaction with an amine group of an amine group of the N-terminus or lysine or a thiol group of cysteine of the biocompatible material (*e.g*., specifically immunoglobulin Fc region), without being limited thereto.

In addition, the reactive groups of the non-peptidyl polymer to be linked to the biocompatible material, specifically immunoglobulin Fc region, and glucagon derivative, may be selected from the group consisting of an aldehyde group, a maleimide group, and a succinimide derivative, but are not limited thereto.

In the above description, the aldehyde group may be a propionaldehyde group or a butyraldehyde group, without being limited thereto.

In the above description, the succinimide derivative may be succinimidyl valerate, succinimidyl methyl butanoate, succinimidyl methylpropionate, succinimidyl butanoate, succinimidyl propionate, *N*-hydroxysuccinimide, hydroxy succinimidyl, succinimidyl carboxymethyl, or succinimidyl carbonate, without being limited thereto.

Also, a final product produced by reductive amination via aldehyde bonds is more stable than a linkage formed by an amide bond. The aldehyde reactive group selectively reacts with the N-terminus at low pH while forming a covalent bond with a lysine residue at high pH, *e.g*., at a pH of 9.0.

In addition, the reactive groups of the two ends may be the same or different, for example, a maleimide group may be provided at one end and an aldehyde group, a propionaldehyde group, or a butyraldehyde group may be provided at the other end. However, the reactive groups are not particularly limited as long as the immunoglobulin Fc region and the glucagon derivative may be linked to the respective ends of the non-peptidyl linker.

For example, the non-peptidyl linker may include a maleimide group at one end and an aldehyde group, a propionaldehyde group, or a butyraldehyde group at the other end, as reactive groups.

When polyethylene glycol having hydroxyl groups as reactive groups at both ends is used as the non-peptidyl polymer, the long-acting protein conjugate according to the present invention may be prepared by activating the hydroxyl groups to various reactive groups by known chemical reactions, or using commercially available polyethylene glycol having modified reactive groups.

In a specific embodiment, the non-peptidyl polymer may be linked to a cysteine residue, more specifically a -SH group of cysteine, of the glucagon derivative without being limited thereto. In this regard, the non-peptidyl polymer may be polyethylene glycol, without being limited thereto, and may also be any other types of non-peptidyl polymers as described above.

In a specific embodiment, the conjugate may be one in which the peptide including an amino acid sequence of SEQ ID NO: 12, 20, or 37 is linked to the immunoglobulin Fc region via the non-peptidyl polymer, wherein the non-peptidyl polymer may be linked to a cysteine residue located at the 30^{th} position of the amino acid sequence of SEQ ID NO: 12, a cysteine residue located at the 17^{th} position of the amino acid sequence of SEQ ID NO: 20, or a cysteine residue located at the 30^{th} position of the amino acid sequence of SEQ ID NO: 37. However, the present invention is not limited thereto. In this regard, the non-peptidyl polymer may be polyethylene glycol, without being limited thereto, and may include any different types of the non-peptidyl polymer as described above.

When maleimide-PEG-aldehyde is used, the maleimide group may be linked to the -SH group of the glucagon derivative via a thioether bond, and the aldehyde group may be linked to the -NH₂ group of the immunoglobulin Fc region via reductive amination, but this is merely an example, and the present invention is not limited thereto.

Also, in the conjugate, the reactive group of the non-peptidyl polymer may be linked to the -NH₂ group located at the N-terminus of the immunoglobulin Fc region, but this is merely an example.

As used herein, the term "immunoglobulin Fc region" refers to a region including a heavy chain constant region 2 (CH2) and/or a heavy chain constant region 3 (CH3) excluding the heavy chain and light chain variable regions of the immunoglobulin. The immunoglobulin Fc region may be a component constituting a moiety of the protein conjugate of the present invention.

The immunoglobulin Fc region may include a hinge region in the heavy chain constant region, without being limited thereto. Also, the immunoglobulin Fc region of the present invention may be an extended Fc region including a part or the entirety of a heavy chain constant region 1 (CH1) and/or a light chain constant region 1 (CL1) excluding the heavy chain and the light chain variable regions of the immunoglobulin, as long as the immunoglobulin Fc region has substantially identical or enhanced effects compared to the native type. Also, the immunoglobulin Fc region may be a region from which a considerably long part of the amino acid sequence corresponding to the CH2 and/or CH3 is removed.

For example, the immunoglobulin Fc region of the present invention may include 1) CH1 domain, CH2 domain, CH3 domain, and CH4 domain, 2) CH1 domain and CH2 domain, 3) CH1 domain and CH3 domain, 4) CH2 domain and CH3 domain, 5) a combination of one or more domains selected from the CH1 domain, CH2 domain, CH3 domain, and CH4 domain and an immunoglobulin hinge region (or a part of the hinge region) or, 6) a dimer of each domain of the heavy chain constant region and the light chain constant region. However, the embodiment is not limited thereto.

Also, in a specific embodiment, the immunoglobulin Fc region may be in a dimeric form, and one glucagon derivative molecule is covalently linked to one Fc region in the dimeric form, wherein the immunoglobulin Fc may be linked to the glucagon derivative via a non-peptidyl polymer. Meanwhile, two glucagon derivative molecules may also be symmetrically linked to one Fc region in the dimeric form. In this case, the immunoglobulin Fc may be linked to the glucagon derivatives via non-peptidyl linkers. However, the embodiment is not limited to the above-described examples.

In addition, the immunoglobulin Fc region of the present invention includes not only native amino acid sequences but also sequence derivatives thereof. The sequence derivatives refer to amino acid sequences having a difference in at least one amino acid residue of a native amino acid sequence by deletion, addition, or non-conservative or conservative substitution, or any combination thereof.

For example, in the case of IgG Fc, amino acid residues at positions 214 to 238, 297 to 299, 318 to 322, or 327 to 331, which are known to be important in the binding, may be used as suitable sites for modification.

Also, various other types of derivatives may be prepared, for example, by removing a region capable of forming a disulfide bond, removing several amino acids from the N-terminus of the native Fc, or adding a methionine residue to the N-terminus of the native Fc. Also, to remove effector functions, a complement-binding site, *e.g*., a C1q-binding site, may be removed, or an antibody-dependent cell-mediated cytotoxicity (ADCC) site may be removed. Techniques of preparing these sequence derivatives of the immunoglobulin Fc region are disclosed in International Patent Application Publication Nos. WO 97/34631, WO 96/32478, *etc.*

Amino acid exchanges in proteins and peptides, which do not alter the activity thereof are well known in the art (H. Neurath, R.L. Hill, The Proteins, Academic Press, New York, 1979). The most commonly occurring exchanges are exchanges between amino acid residues, *e.g*., Ala/Ser, Val/lle, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. If required, the Fc region may be modified by phosphorylation, sulfation, acrylation, glycosylation, methylation, farnesylation, acetylation, amidation, and the like.

The above-described Fc derivatives may have biological activity identical to that of the Fc region of the present invention and improved structural stability against heat, pH, or the like.

In addition, the Fc region may be obtained from native forms isolated *in vivo* from humans or animals such as cows, goats, pigs, mice, rabbits, hamsters, rats, or guinea pigs, or may be recombinants or derivatives thereof, obtained from transformed animal cells or microorganisms. In this regard, the Fc region may be obtained from a native immunoglobulin by isolating whole immunoglobulin from a living body of a human or animal and treating the isolated immunoglobulin with protease. When the whole immunoglobulin is treated with papain, it is cleaved into Fab and Fc regions, whereas when the whole immunoglobulin is treated with pepsin, it is cleaved into pF'c and F(ab)₂ fragments. Fc or pF'c may be isolated therefrom using size-exclusion chromatography, or the like. In a more specific embodiment, a human-derived Fc region is a recombinant immunoglobulin Fc region obtained from a microorganism.

In addition, the immunoglobulin Fc region may have natural glycans or increased or decreased glycans compared to the natural type or be in a deglycosylated form. The increase, decrease, or removal of glycans of the immunoglobulin Fc may be achieved by way of any methods commonly used in the art such as a chemical method, an enzymatic method, and a genetic engineering method using a microorganism. In this regard, the immunoglobulin Fc region obtained by removing glycans shows a significant decrease in binding affinity to a complement c1q and a decrease in or loss of antibody-dependent cytotoxicity or complement-dependent cytotoxicity, and thus unnecessary immune responses are not induced thereby in living organisms. Based thereon, a deglycosylated or aglycosylated immunoglobulin Fc region may be more suitable as a drug carrier in view of the objects of the present invention.

As used herein, the term "deglycosylation" refers to an Fc region from which glycan is removed using an enzyme and the term "aglycosylation" refers to an Fc region that is not glycosylated and produced in prokaryotes, more specifically *E. coli.*

Meanwhile, the immunoglobulin Fc region may be derived from humans or animals such as cows, goats, pigs, mice, rabbits, hamsters, rats, or guinea pigs. In a more specific embodiment, the immunoglobulin Fc region may be derived from humans.

In addition, the immunoglobulin Fc region may be derived from IgG, IgA, IgD, IgE, or IgM, or any combination or hybrid thereof. In a more specific embodiment, it is derived from IgG or IgM, which are the most abundant proteins in human blood, and in an even more specific embodiment, it is derived from IgG, which is known to enhance the half-lives of ligand-binding proteins. In a yet even more specific embodiment, the immunoglobulin Fc region is an IgG4 Fc region, and in the most specific embodiment, the immunoglobulin Fc region is an aglycosylated Fc region derived from human IgG4, without being limited thereto.

Meanwhile, as used herein, the term "combination" refers to formation of a linkage between a polypeptide encoding a single-chain immunoglobulin Fc region of the same origin and a single-chain polypeptide of a different origin when a dimer or a multimer is formed. That is, a dimer or multimer may be prepared using two or more Fc fragments selected from the group consisting of IgG Fc, IgA Fc, IgM Fc, IgD Fc, and IgE Fc fragments.

The combination, composition, or kit of the present invention may be used to prevent or treat a metabolic syndrome.

As used herein, the term "prevention" refers to all actions intended to inhibit or delay development of a target disease, *e.g*., a metabolic syndrome, by administering a substance with glucagon activity or a conjugate thereof, a compound or substance with therapeutic activity against a metabolic syndrome, or a combination or composition including the same. The term "treatment" refers to all actions to alleviate or beneficially change symptoms associated with a target disease, *e.g*., a metabolic syndrome, by administering a substance with glucagon activity or a conjugate thereof, a compound or substance with therapeutic activity against a metabolic syndrome, or a combination or composition including the same.

As used herein, the "administration" refers to introduction of a particular substance into a patient by way of any appropriate method, and an administration route of the composition may be, but is not limited to, any conventional route that enables delivery of the composition to the target in living organisms, for example, intraperitoneal administration, intravenous administration, intramuscular administration, subcutaneous administration, intradermal administration, oral administration, topical administration, intranasal administration, intrapulmonary administration, or intrarectal administration.

The glucagon derivative or a conjugate including the same may be used alone or in combination with the compound or substance with therapeutic activity against a metabolic syndrome for prevention or treatment of a metabolic syndrome.

In addition, the substance with glucagon activity or a conjugate thereof of the present invention may be used as a medicament for preventing weight gain, accelerating weight loss, reducing overweight, and treating disease and health status including, but not limited to, obesity including morbid obesity (*e.g*., by controlling appetite, diet, food ingestion, calorie intake, and/or energy consumption), obesity-related inflammation, obesity-related gallbladder disease, and obesity-induced sleep apnea. The substance with glucagon activity or a conjugate thereof of the present invention may also be used for treating a metabolic syndrome other than obesity, *i.e*., impaired glucose tolerance, hypercholesterolemia, dyslipidemia, obesity, diabetes, hypertension, non-alcoholic steatohepatitis (NASH), arteriosclerosis caused by dyslipidemia, atherosclerosis, arteriosclerosis, coronary heart disease, stroke, and the like. However, in these conditions, the effects of the peptide according to the present invention may be entirely or partially mediated by the above-described weight-related effect or may be independent thereof.

As used herein, the term "metabolic syndrome" refers to a symptom in which various diseases caused by chronic metabolic disorders occur singly or in combination. In particular, examples of the diseases belonging to the metabolic syndrome may include impaired glucose tolerance, hypercholesterolemia, dyslipidemia, obesity, diabetes, hypertension, non-alcoholic steatohepatitis (NASH), arteriosclerosis caused by dyslipidemia, atherosclerosis, arteriosclerosis, coronary heart disease, and stroke, but are not limited thereto.

As used herein, the term "obesity" refers to a health condition in which adipose tissue is excessive in the body. When an obesity index (body mass index: a value obtained by dividing a person's body weight (kg) over the square of his/her height (m²) is 25 or higher, the person is defined as being obese. Obesity is generally caused by energy imbalance when an excess energy intake over energy expenditure continues for a long time.

Obesity is a metabolic disease that affects the whole body, increases the likelihood of getting diabetes and hyperlipidemia, increases the risk of developing sexual dysfunction, arthritis, and cardiovascular disease, and is related to occurrence of cancer.

Since the glucagon derivative according to the present invention has improved solubility and high stability at neutral pH due to an altered pi value different from that of native glucagon, and also has activity to a glucagon receptor, the glucagon derivative may be effectively used for prevention or treatment of a target disease such as a metabolic syndrome.

The pharmaceutical composition of the present invention may further include a pharmaceutically acceptable carrier, excipient, or diluent. As used herein, the term "pharmaceutically acceptable" refers to an amount sufficient for exhibiting therapeutic effects without causing side-effects and may be easily determined by those of ordinary skill in the art based on factors well known in the medical field such as the type of disease, age, body weight, health status, gender, and sensitivity to drug of a patient, administration route, administration method, frequency of administration, duration of treatment, and a drug used in combination or concurrently.

The pharmaceutical composition according to the present invention including the substance with glucagon activity and a conjugate thereof may further include a pharmaceutically acceptable carrier. Although the pharmaceutically acceptable carrier is not particularly limited, a binder, a lubricant, a disintegrator, an excipient, a solubilizer, a dispersant, a stabilizer, a suspending agent, a coloring agent, and a flavoring agent may be used for oral administration, a buffer, a preservative, an analgesic, a solubilizer, an isotonic agent, and a stabilizer may be used in combination for injectable preparations, and a base, an excipient, a lubricant, a preservative, and the like may be used for preparation for topical administration.

The pharmaceutical composition of the present invention may be formulated into various forms in combination with the above-mentioned pharmaceutically acceptable carrier. For example, for oral administration, the pharmaceutical composition may be formulated into tablets, troches, capsules, elixirs, suspensions, syrups, wafers, and the like. For injectable preparations, the pharmaceutical composition may be formulated into a single-dose ampoule or multidose form. The pharmaceutical composition may also be formulated into solutions, suspensions, tablets, pills, capsules, sustained-release preparations, and the like.

Meanwhile, examples of the carrier, excipient, and diluent suitable for formulation may include lactose, dextrose, sucrose, sorbitol, mannitol, xylitol, erythritol, maltitol, starch, Acacia rubber, alginate, gelatin, calcium phosphate, calcium silicate, cellulose, methyl cellulose, amorphous cellulose, polyvinyl pyrrolidone, water, methylhydroxy benzoate, propylhydroxy benzoate, talc, magnesium stearate, or mineral oils. Also, the pharmaceutical composition may further include a filler, an anti-coagulant, a lubricant, a humectant, a flavoring agent, a preservative, and the like.

In addition, the pharmaceutical composition of the present invention may be formulated in a formulation selected from the group consisting of tablets, pills, powders, granules, capsules, suspensions, formulations for internal use, emulsions, syrups, sterilized aqueous solutions, non-aqueous solvents, lyophilized preparations, and suppositories.

Also, the composition may be formulated in a unit dosage form suitable for administration into the body of a patient, specifically in a form useful for administration of peptide medicines, according to a method commonly used in the art and administered via an oral administration route or a parenteral administration route such as an intradermal, intravenous, intramuscular, intraarterial, intramedullary, intrathecal, intraventricular, intrapulmonary, transdermal, subcutaneous, intraperitoneal, intranasal, intestinal, topical, sublingual, vaginal, or rectal route using an administration method commonly used in the art, but is not limited thereto.

In addition, the substance with glucagon activity or a conjugate thereof may be used in combination with various carriers permitted as medicaments such as a saline solution or an organic solvent. As the medicaments, carbohydrates such as glucose, sucrose, or dextran, antioxidants such as ascorbic acid or glutathione, chelating agents, low-molecular-weight proteins, or other stabilizers may be used to improve stability or absorbability.

An administration dose and frequency of the pharmaceutical composition of the present invention may be determined depending on a type of a drug, as an active ingredient, together with various related factors such as a disease to be treated, administration route, age, gender, and body weight of a patient, and severity of the disease.

Although not particularly limited thereto, the pharmaceutical composition of the present invention may include the component (active ingredient) in an amount of 0.01% to 99% by weight.

A total effective amount of the composition of the present invention may be administered to a patient in a single dose or in multiple doses using a fractional treatment protocol in which administration is performed for a prolonged period of time. The amount of an effective ingredient of the pharmaceutical composition of the present invention may vary according to severity of a disease. Specifically, a preferred daily dosage of the glucagon derivative peptide or a conjugate thereof of the present invention may be from about 0.0001 µg to about 500 mg per 1 kg of the body weight of the patient. However, since the dosage of the peptide or conjugate is determined as an effective dosage for the patient in consideration of various factors such as age, body weight, health status, and gender of the patient, severity of the disease, diet, and excretion rate as well as route and frequency of administration of the pharmaceutical composition, an appropriate effective dosage for a particular use of the composition of the present invention may be determined by one of ordinary skill in the art by considering these factors. Formulation, administration route, and administration method of the pharmaceutical composition are not particularly limited as long as the effects of the present invention are obtained.

Since the pharmaceutical composition of the present invention has excellent *in vivo* persistence and potency, the number and frequency of administration of the pharmaceutical composition according to the present invention may be significantly reduced, without being limited thereto.

In particular, since the pharmaceutical composition of the present invention includes the glucagon derivative having an altered pi value different from that of native glucagon as an active ingredient, improved solubility and/or high stability may be obtained at neutral pH, and thus the pharmaceutical composition may be effectively used for preparation of a stable glucagon formulation for treating a target disease such as a metabolic syndrome.

The pharmaceutical composition for preventing or treating a metabolic syndrome in a therapy for preventing or treating a metabolic syndrome may further include a compound or substance with therapeutic activity against the metabolic syndrome in addition to the substance with activity to a glucagon receptor or a conjugate thereof, and the therapy may further include additional use of the compound or substance.

Examples of the compound or substance with therapeutic activity against a metabolic syndrome contained in co-administration or composition of the present invention may include an insulinotropic peptide, a glucagon-like peptide-1 (GLP-1) receptor agonist, a Leptin receptor agonist, a dipeptidyl peptidase-IV (DPP-IV) inhibitor, a Y5 receptor antagonist, a melanin-concentrating hormone (MCH) receptor antagonist, a Y2/4 receptor agonist, a melanocortin 3/4 (MC3/4) receptor agonist, a gastric/pancreatic lipase inhibitor, a 5-hydroxytryptamine receptor 2C (5HT2c), a G protein-coupled) agonist, a β3A receptor agonist, an amylin receptor agonist, a ghrelin antagonist, a ghrelin receptor antagonist, a peroxisome proliferator-activated receptor alpha (PPARα) agonist, a peroxisome proliferator-activated receptor delta (PPARδ) agonist, a farnesoid X receptor (FXR) agonist, an acetyl-CoA carboxylase inhibitor, peptide YY, cholecystokinin (CCK), Xenin, glicentin, obestatin, secretin, nesfatin, insulin, a glucose-dependent insulinotropic peptide (GIP), biguanides, sulfonylureas, meglitinide, thiazolidinedione (TZD), a sodium-glucose cotransporter (SGLT2) inhibitor, and/or an α-glucosidase inhibitor, without being limited thereto. More specifically, the compound or substance may be a DPP-4 inhibitor, an SGLT2 inhibitor, or a glucagon-like peptide-1 (GLP-1) receptor agonist, without being limited thereto. In addition, the compound or substance may include all drugs with a therapeutic effect on obesity and drugs inhibiting inflammation and fibrosis of the liver.

Specifically, as used herein, the term "GLP-1 receptor agonist" refers to a substance (compound, peptide, amino acid, and the like) with activity to the GLP-1 receptor, as one of the substances having therapeutic activity against the metabolic syndrome, and may be used interchangeably with "GLP-1 analog". The GLP-1 receptor agonist may have *in vitro* activity of 0.1% or more, 1% or more, 2% or more, 3% or more, 4% or more, 5% or more, 6% or more, 7% or more, 8% or more, 9% or more, 10% or more, 20% or more, 30% or more, 40% or more, 50% or more, 60% or more, 70% or more, 80% or more, 90% or more, or 100% or more, to the GLP-1 receptor, compared to that of a native ligand (GLP-1) of the receptor.

The DPP-4 inhibitor may be selected from the group consisting of Sitagliptin (e.g., Januvia®), Vildagliiptin, Saxagliptin, Alogliptin, Linagliptin, Trayenta®, Anagliptin, Teneligliptin, Trelagliptin, migliptin, Omarigliptin, Evogliptin, and Dutogliptin; and
the SGLT2 inhibitor may be selected from the group consisting of Empagliflozin (e.g., Jardiance®), Dapagliflozin, Canagliflozin, Remogliflozin, Remogliflozin Etabonate, Sergliflozin, Ipragliflozin, Tofogliflozin, Luseogliflozin, and Ertugliflozin, but these are not limited thereto as long as they may be co-administered with the substance with activity to a glucagon receptor or a conjugate thereof according to the present invention and have therapeutic activity.

Another aspect of the present invention provides a pharmaceutical composition for preventing or treating a metabolic syndrome including the substance with activity to a glucagon receptor or a conjugate thereof characterized in that it is likely to be likely to be co-administered with the compound or substance with therapeutic activity against the metabolic syndrome.

The substance with activity to a glucagon receptor, the conjugate including the same, the composition including the same, the metabolic syndrome, prevention, treatment, and the pharmaceutical composition are as described above.

Another aspect of the present invention provides a method of preventing or treating a metabolic syndrome, the method including administering (i) a substance with activity to a glucagon receptor or a conjugate thereof and (ii) a compound or substance with therapeutic activity against a metabolic syndrome, to an individual in need thereof.

The substance with activity to a glucagon receptor, the conjugate including the same, the composition including the same, the metabolic syndrome, prevention, and treatment are as described above.

In the present invention, the individual refers to a subject suspected of having a metabolic syndrome, and the subject suspected of having a metabolic syndrome refers to mammals such as rats and livestock including humans with a metabolic syndrome or at the risk of developing the disease, but any individual that may be treated with the glucagon derivative or the composition including the same according to the present invention may be included without limitation. In addition, by administering the pharmaceutical composition including the glucagon derivative according to the present invention to an individual suspected of having a metabolic syndrome, the individual may be effectively treated. The metabolic syndrome is as described above.

The method of the present invention may include administering a combination or pharmaceutical composition including (i) the substance with activity to a glucagon receptor or a conjugate thereof and (ii) the compound or substance with therapeutic activity against a metabolic syndrome, to the individual in a pharmaceutically effective amount. The method of the present invention may include administering (i) the substance with activity to a glucagon receptor or a conjugate thereof and (ii) the compound or substance with therapeutic activity against a metabolic syndrome, in a single formulation or in separate formulations simultaneously, individually, sequentially, or in reverse order, without being limited thereto.

An appropriate daily dose may be determined by a doctor within the scope of sound medical judgment in a bolus or in multiple doses. For the purpose of the present invention, it is preferred that a specific therapeutically effective amount for a specific patient is differently applied depending on various factors including the type and extent of a response to be achieved, a specific composition including whether other formulations are used according to the case, age, body weight, general health status, gender, and diet of the patient, administration time, administration route, excretion rate of the composition, duration of treatment, a drug used in combination or concurrently with the specific composition and similar factors well known in the medical field.

Meanwhile, although not particularly limited thereto, the method of preventing or treating a metabolic syndrome may be a co-administration method further including administering at least one compound or substance with therapeutic activity against a metabolic syndrome.

Another aspect of the present invention provides a composition, specifically a pharmaceutical composition for preventing or treating a metabolic syndrome, hypoglycemia, or congenital hyperinsulinism including a peptide with activity to a glucagon receptor or a conjugate including the same.

The metabolic syndrome may be at least one disease selected from the group consisting of impaired glucose tolerance, hypercholesterolemia, dyslipidemia, obesity, diabetes, hypertension, non-alcoholic steatohepatitis (NASH), arteriosclerosis caused by dyslipidemia, atherosclerosis, arteriosclerosis, coronary heart disease, and stroke, without being limited thereto.

The peptide with activity to the glucagon receptor includes a substance in the form of a peptide having a significant level of activity to the glucagon receptor and refers to the substance with activity to a glucagon receptor.

The substance with activity to a glucagon receptor, the conjugate including the same, the composition including the same, the metabolic syndrome, prevention, and treatment are as described above.

The pharmaceutical composition of the present invention may be used to prevent or treat a metabolic syndrome, hypoglycemia, or congenital hyperinsulinism.

As used herein, the term "hypoglycemia" refers to a condition when a blood glucose level is abnormally low. A blood glucose level below 50 mg/dL is generally regarded as hypoglycemia, without being limited thereto. Hypoglycemia is commonly caused when a person who takes a medication such as an oral hypoglycemic agent or insulin consumes less food or exercises more than usual. In addition, hypoglycemia may be caused by drinking alcohol, use of a drug lowering blood glucose level, severe physical illness, deficiency of hormones such as adrenocortical hormones or glucagon, tumors of pancreas that produces insulin, autoimmune disease against insulin, gastric resection, inherited disorders of carbohydrate metabolism, or the like.

In the present invention, the hypoglycemia includes both acute hypoglycemia and chronic hypoglycemia.

Symptoms of hypoglycemia include weakness, shakiness, pallor, sweating, giddiness, anxiety, nervousness, palpitation, feeling of hunger, headache, fatigue, and the like. When hypoglycemia continues for a long time, convulsions or seizures may be caused, resulting in shock and loss of consciousness.

More specifically, the hypoglycemia may be caused by persistent hyperinsulinism due to genetic defects. Causes of hyperinsulinism due to genetic defects may include a mutation of SUR or Kir6.2 gene on the 11p15.1 chromosome, an increase in GK activity due to a mutation of the glucokinase (GK) gene on the 7p15-p13 chromosome, and an increase in increasing ATP in beta cells in the pancreatic islets due to glutamate dehydrogenase (GDH) activated by a mutation of GDH.

Meanwhile, congenital hyperinsulinism is one of the causes of severe and persistent hypoglycemia in newborns and children. Congenital hyperinsulinism may be caused by a temporary increase in insulin secretion in babies born with low birth weight or born from diabetic mothers and abnormal functioning of pancreatic cells due to genetic mutation. It is known that glucagon may be used to treat the congenital hyperinsulinism.

In addition, the peptide with activity to a glucagon receptor or a conjugate including the same of the present invention may be used as a medicament for preventing weight gain, accelerating weight loss, reducing overweight, and treating disease and health status including, but not limited to, obesity including morbid obesity (e.g., by controlling appetite, diet, food ingestion, calorie intake, and/or energy consumption), obesity-related inflammation, obesity-related gallbladder disease, and obesity-induced sleep apnea. The peptide with activity to a glucagon receptor or a conjugate including the same according to the present invention may be used to treat a metabolic syndrome as well as obesity, i.e., associated diseases such as impaired glucose tolerance, hypercholesterolemia, dyslipidemia, obesity, diabetes, hypertension, non-alcoholic steatohepatitis (NASH), arteriosclerosis caused by dyslipidemia, atherosclerosis, arteriosclerosis, coronary heart disease, and stroke. However, in these conditions, the effects of the peptide according to the present invention may be entirely or partially mediated by the above-described weight-related effect or may be independent thereof.

As used herein, the term "obesity" refers to a health condition in which adipose tissue is excessive in the body. When an obesity index (body mass index: a value obtained by dividing a person's body weight (kg) over the square of his/her height (m²) is 25 or higher, the person is defined as being obese. Obesity is generally caused by energy imbalance when an excess energy intake over energy expenditure continues for a long time. Obesity is a metabolic disease that affects the whole body, increases the likelihood of getting diabetes and hyperlipidemia, increases the risk of developing sexual dysfunction, arthritis, and cardiovascular disease, and is related to occurrence of cancer.

The peptide with activity to a glucagon receptor of the present invention may also be effectively used alone to prevent or treat a metabolic syndrome, hypoglycemia, or congenital hyperinsulinism, and the glucagon derivative may exhibit improved solubility and high stability at neutral pH due to an altered pi value different from that of native glucagon and have activity to the glucagon receptor, thereby being effectively used to prevent or treat a target disease such as hypoglycemia, obesity, a metabolic syndrome, and congenital hyperinsulinism.

Another aspect of the present invention provides a peptide with activity to a glucagon receptor, i.e., a glucagon derivative.

The peptide with activity to the glucagon receptor is as described above.

More specifically, it is characterized in that the derivative is an isolated peptide including the amino acid sequence of General Formula 1 represented by SEQ ID NO: 45. Descriptions and combinations of the isolated peptide including the amino acid sequence of General Formula 1 above are as described above.

It is characterized in that the derivative is an isolated peptide including an amino acid sequence of General Formula 2 below.

Y-Aib-QGTF-X7-SD-X10-S-X12-Y-L-X15-X16-X17-R-A-X20-X21-F-V-X24-W
-L-M-N-T-X30 (General Formula 2, SEQ ID NO: 46)

In General Formula 2 above,
X7 is threonine, valine, or cysteine;
X10 is tyrosine or cysteine;
X12 is lysine or cysteine;
X15 is aspartic acid or cysteine;
X16 is glutamic acid or serine;
X17 is lysine or arginine;
X20 is glutamine or lysine;
X21 is aspartic acid or glutamic acid;
X24 is valine or glutamine; and
X30 is cysteine, or is absent.

More specifically, the peptide including the amino acid sequence of General Formula 2 above may have a structure in which X16 is glutamic acid, X20 is lysine, and a lactam ring is formed between side chains of X16 and X20 of General Formula 2, without being limited thereto.

In addition, the C-terminus of the peptide including the amino acid sequence of General Formula 2 above may be amidated or may not be modified, without being limited thereto.

In addition, the peptide may be a glucagon derivative capable of activating the glucagon receptor, without being limited thereto.

More specifically, the peptide may include an amino acid sequence selected from the group consisting of SEQ ID NOS: 12, 13, 15, and 36 to 44, without being limited thereto.

Another aspect of the present invention provides an isolated polynucleotide encoding the glucagon derivative, a vector including the polynucleotide, and isolated cells including the polynucleotide or vector.

The glucagon derivative is as described above.

Specifically, the derivative may be an isolated peptide including an amino acid sequence of General Formula 1 represented by SEQ ID NO: 45. For descriptions and combinations with regard to the isolated peptide including the amino acid sequence of General Formula 1, all of those described above will be applied thereto. Also, specifically, the derivative may be an isolated peptide including an amino acid sequence of General Formula 1 represented by SEQ ID NO: 46. For descriptions and combinations with regard to the isolated peptide including the amino acid sequence of General Formula 2, all of those described above will be applied thereto.

As used herein, the term "homology" indicates sequence similarity with a wild-type amino acid sequence or wild-type nucleotide sequence, and the homology comparison may be done by visual observation or using a commercially available comparison program. Using a commercially available computer program, the homology between two or more sequences may be expressed as a percentage (%), and the homology (%) between adjacent sequences may be calculated.

As used herein, the term "recombinant vector" refers to a DNA construct including a gene encoding a target peptide, e.g., a glucagon derivative, and operably linked to an appropriate regulatory sequence to express the target peptide, e.g., the glucagon derivative, in a suitable host cell.

The regulatory sequence may include a promoter initiating transcription, an operator sequence for regulating the transcription, a sequence encoding a suitable mRNA ribosome binding site, and a sequence regulating termination of transcription and translation. After the suitable host cell is transformed with the recombinant vector, it may replicate or function independently of the host genome and may be integrated into the genome.

The recombinant vector used herein is not particularly limited as long as the recombinant vector replicate in host cells, and any vector known in the art may be used. Examples of the vector known in the art may include a natural or recombinant plasmid, cosmid, virus, and bacteriophage. The vector available in the present invention is not particularly limited, and any known expression vectors may be used.

The recombinant vector is used to transform host cells to produce the glucagon derivative of the present invention. Also, as a part of the present invention, such transformed cells may be cells or cell lines used to proliferate nucleic acid fragments and vectors of the present invention or used in cultivation for recombinant production of the glucagon derivative of the present invention.

As used herein, the term "transformation" refers to a process of introducing the recombinant vector including a polynucleotide encoding a target protein into a host cell in such a way that the protein encoded by the polynucleotide is expressed in the host cell. The transformed polynucleotide may be either in a form inserted into the chromosome of the host cell or in a form located outside the chromosome as long as the protein is expressed in the host cell.

In addition, the polynucleotide includes DNA and RNA encoding the target protein. The polynucleotide may be introduced into the host cell in any form as long as the polynucleotide is introduced into the host cell and the protein is expressed therein. For example, the polynucleotide may be introduced into the host cell in the form of an expression cassette, which is a gene construct including all of the essential elements required for self-replication. The expression cassette may generally include a promoter operably linked to the polynucleotide, a transcription termination signal, a ribosome binding site, and a translation termination signal. The expression cassette may be in the form of a self-replicable expression vector. Also, the polynucleotide may be introduced into the host cell in its original form and operably linked to a sequence required for the expression in the host cell, without being limited thereto.

In addition, as used herein, the term "operably linked" means a functional linkage between a gene sequence encoding the polypeptide of the present invention and a promoter sequence which initiates and mediates transcription of the nucleotide sequence.

Hosts suitable for the present invention are not particularly limited as long as the polynucleotide of the present invention is expressed therein. Specific examples of the hosts available in the present invention may include bacteria belonging to the genus *Escherichia* such as *E. coli;* bacteria belonging to the genus *Bacillus* such as *Bacillus subtilis;* bacteria belonging to the genus *Pseudomonas* such as *Pseudomonas putida;* yeast such as *Pichia pastoris, Saccharomyces cerevisiae,* and *Schizosaccharomyces pombe;* insect cells such as *Spodoptera frugiperda* (Sf9); and animal cells such as CHO, COS, and BSC.

Another aspect of the present invention provides an isolated conjugate in which a peptide with activity to a glucagon receptor is linked to a biocompatible material increasing *in vivo* half-life. The conjugate may be a long-acting conjugate.

Specifically, as the isolated conjugate including the peptide site and the biocompatible material linked to the peptide site via a covalent bond, an isolated conjugate is provided in which the peptide site includes an amino acid sequence identical to or including the amino acid sequence of General Formula 1 or 2.

The glucagon derivative, the amino acid sequence of General Formula 1 or 2, the biocompatible material, and the conjugate are as described above.

Specifically, the derivative may be an isolated peptide including the amino acid sequence of General Formula 1 represented by SEQ ID NO: 45. For descriptions and combinations with regard to the isolated peptide including the amino acid sequence of General Formula 1, all of those described above will be applied thereto.

More specifically, it is characterized in that the derivative is an isolated peptide including the amino acid sequence of General Formula 2 represented by SEQ ID NO: 46. For descriptions and combinations with regard to the isolated peptide including the amino acid sequence of General Formula 2, all of those described above will be applied thereto.

Another aspect of the present invention provides a method of preventing or treating congenital hyperinsulinism, hypoglycemia, obesity, or a metabolic syndrome, the method including administering a peptide with activity to the glucagon receptor, a conjugate including the same, or a composition including the same, to an individual.

The peptide with activity to a glucagon receptor, the conjugate including the same, the composition including the same, congenital hyperinsulinism, hypoglycemia, obesity, the metabolic syndrome, prevention, and treatment are as described above.

In the present invention, the individual refers to a subject suspected of having congenital hyperinsulinism, hypoglycemia, obesity, or a metabolic syndrome, and the subject suspected of having congenital hyperinsulinism, hypoglycemia, obesity, or a metabolic syndrome refers to mammals such as rats and livestock including humans with the disease or at risk of developing the disease, but any individual that may be treated with the glucagon derivative or the composition including the same according to the present invention may be included without limitation. In addition, by administering the pharmaceutical composition including the peptide with activity to the glucagon receptor according to the present invention to an individual suspected of having congenital hyperinsulinism, hypoglycemia, or obesity, the individual may be effectively treated. The congenital hyperinsulinism, hypoglycemia, obesity, and metabolic syndrome are as described above.

The method of the present invention may include administering the pharmaceutical composition including the peptide in a pharmaceutically effective amount. An appropriate daily dose may be determined by a doctor within the scope of sound medical judgment in a bolus or in multiple doses. However, for the purpose of the present invention, it is preferred that a specific therapeutically effective amount for a specific patient is differently applied depending on various factors including the type and extent of a response to be achieved, a specific composition including whether other formulations are used according to the case, age, body weight, general health status, gender, and diet of the patient, administration time, administration route, excretion rate of the composition, duration of treatment, a drug used in combination or concurrently with the specific composition and similar factors well known in the medical field.

Meanwhile, although not particularly limited thereto, the method of preventing or treating a metabolic syndrome may be a co-administration method further including administering at least one compound or substance with therapeutic activity against a metabolic syndrome.

When the term "co-administration" is used, it should be understood that the components are administered simultaneously, individually, or sequentially. When administration is performed sequentially or individually, an interval between two components should be set not to lose beneficial effects of co-administration.

Another aspect of the present invention provides a use of the peptide with activity to a glucagon receptor or the isolated conjugate, or the composition for preparation of medicaments (or pharmaceutical composition) for preventing or treating congenital hyperinsulinism, hypoglycemia, obesity, or a metabolic syndrome.

The peptide with activity to a glucagon receptor, the isolated conjugate, the composition, congenital hyperinsulinism, hypoglycemia, obesity, and the metabolic syndrome are as described above.

Hereinafter, the present invention will be described in more detail with reference to the following examples. However, the following examples are merely presented to exemplify the present invention, and the scope of the present invention is not limited thereto.

### Example 1: Production of cell line exhibiting cAMP response to glucagon

PCR was performed using a region of human glucagon receptor gene corresponding to Open Reading Frame (ORF) in the cDNA (OriGene Technologies, Inc., USA) as a template and forward and reverse primers of SEQ ID NOS: 47 and 48 including EcoRI and Xhol restriction sites, respectively.

In this regard, PCR was performed for a total of 30 cycles under the following conditions: at 95°C for 60 seconds for denaturation, at 55°C for 60 seconds for annealing, and at 68°C for 30 seconds for elongation. The amplified PCR products were subjected to electrophoresis in a 1.0% agarose gel, followed by elution, to obtain a 450 bp band.

Forward primer (SEQ ID NO: 47):
5'-CAGCGACACCGACCGTCCCCCCGTACTTAAGGCC-3'

Reverse primer (SEQ ID NO: 48):
5'-CTAACCGACTCTCGGGGAAGACTGAGCTCGCC-3'

The PCR product was cloned into a known animal cell expression vector, xOGC/dhfr, to prepare a recombinant vector xOGC/GCGR.

CHO DG44 cells cultured in DMEM/F12 supplemented with 10% FBS medium were transformed with the prepared recombinant vector xOGC/GCGR using Lipofectamine, and cultured in a selection medium containing 1 mg/mL G418 and 10 nM methotrexate. Single clone cells were selected therefrom by a limiting dilution technique, and a cell line exhibiting excellent cAMP response to glucagon in a concentration-dependent manner was finally selected therefrom.

### Example 2: Synthesis of glucagon derivative

In order to prepare a glucagon derivative having improved physical properties, the amino acid sequence (SEQ ID NO: 1) of native glucagon was substituted with amino acid residues with negative and positive charges to synthesize glucagon derivatives as shown in Table 1 below. Relative *in vitro* activities described therein were measured by way of a method described in Example 4 below.

**Table 1**

| Amino acid sequences of native glucagon and glucagon derivatives | | | | |
|---|---|---|---|---|
| SEQ ID NO | Peptide sequence | Ring | pI | *in vitro activity* (%, *relative to SEQ ID NO: 1)* |
| 'SEQ ID NO: 1 | HSQGTFTSDYSKYLDSRRAQDFVQWLMNT | - | 6.8 | 100 |
| SEQ ID NO: 2 | HSQGTFTSDYSKYLDCDRAQDFVQWLMNT | - | 4.56 | 0.6 |
| SEQ ID NO: 3 | HSQGTFTSDYSKYLDCERAQDFVQWLHNT | - | 4.66 | 6.1 |
| SEQ ID NO: 4 | HSQGTFTSDYSKYLDSCDAQDFVQWLMHT | - | 4.13 | < 0.1 |
| SEQ ID NO: 5 | HSQGTFTSDYSKYLDSCEAQDFVQWLMNT | - | 4.22 | 0.3 |
| SEQ ID NO: 6 | HSQGTFTSDYSKYLDSCEADDFVQWLMNT | - | 4.03 | < 0.1 |
| SEQ ID NO: 7 | YSCTGTFTSDYSKYLDSCEADDFVQWLMT | - | 3.71 | < 0.1 |
| SEQ ID NO: 8 | YXQGTFTSDYSKYLDSCDAQDFVQWLINT | - | 3.77 | < 0.1 |
| SEQ ID NO: 9 | YXQGTFTSDYSKYLDSCDAQDFVVWLINT | - | 3.77 | < 0.1 |
| SEQ ID NO: 10 | YXQGTFTSDYSKYLDSCDADDFVVWLINT | - | 3,66 | < 0. 1 |
| SEQ ID NO: 11 | YXQGTFTSDYSKYLDEKCAKEFVQWLMNT | - | 4.78 | 4.6 |
| SEQ ID NO: 12 | YXQGTFTSDYSKYLD**E**KRA**K**EFVQWLMNTC | ring formed | 6.20 | 56. 3 |
| SEQ ID NO: 13 | YXQGTFTSDYSCYLDSRRAQDFVQWLMNT | - | 4.43 | 5.2 |
| SEQ ID NO: 14 | YXQGTFTSDYSKYLDCKRAKEFVQWLMNT | - | 8.12 | 18.1 |
| SEQ ID NO: 15 | YXQGTFTSDYSKYLCEKRAQDFVVWLMNT | - | 6,11 | 1.1 |
| SEQ ID NO: 16 | YXQGTFTSDYSKYLDCRRAQVFVQWLWLMRT | - | 9.11 | 4.2 |
| SEQ ID NO: 17 | YXQGTFTSDYSKYLDCVRAQDFVQWLMRT | - | 6.03 | 23.2 |
| SEQ ID NO: 18 | YXQGTFTSDYSKYLDSRRACDFRLWLMNT | - | 8.15 | < 0.1 |
| SEQ ID NO: 19 | YXQGTFTSDYSKYLC**E**KRA**K**EFVQWLMNT | ring formed | 8.12 | 12.1 |
| SEQ ID NO: 20 | YXQGTFTSDYSKYLD**E**CRA**K**EFVQWLMNT | ring formed | 4.78 | 299.7 |
| SEQ ID NO: 21 | YXQGTFTSDYSKYLD**E**KA**K**EFVQWLMNT | ring formed | 4.78 | 57.8 |
| SEQ ID NO: 22 | YXQGTFTSDYSKYLD**E**KRC**K**EFVQWLMNT | ring formed | 6.20 | 147, 8 |
| SEQ ID NO: 23 | YXQGTFTSDYSKYCD**E**KRA**K**EFVQWLMNT | ring formed | 6.20 | 76,8 |
| SEQ ID NO: 24 | YXQGTFTSDYSKCLD**E**KRA**K**EFVQWLMNT | ring formed | 6.21 | 58.0 |
| SEQ ID NO: 25 | YXQGTFTSDYSKYLD**E**KRA**K**CFVQWLMNT | ring formed | 8.12 | 46.9 |
| SEQ ID NO: 26 | WXQGTFTSDYSKYLD**E**CRA**K**DFVQWLMNT | ring formed | 4.68 | 1.0 |
| SEQ ID NO: 27 | YXQGTFVSDYSKYLD**E**CRA**K**DFVQWLMNT | ring formed | 4.68 | 93.6 |
| SEQ ID NO: 28 | WXQGTFVSDYSKYLD**E**CRA**K**DFVQWLMNT | ring formed | 4.68 | < 0.1 |
| SEQ ID NO: 29 | YXQGTFTSDYSKCLD**E**RRA**K**DFVQWLMNT | ring formed | 6.15 | 61.3 |
| SEQ ID NO: 30 | WXQGTFTSDYSKCLD**E**RRA**K**DFVQWLMNT | ring formed | 4.44 | 0.3 |
| SEQ ID NO: 31 | YXQGTFTSDYSKYLDCKRAKEFVQWLMNT | ring formed | 8.12 | 6.3 |
| SEQ ID NO: 32 | -SQGTFTSDYSKYLD**E**CRA**K**EFVQWLMHT | ring formed | 4.78 | 0.7 |
| SEQ ID NO: 33 | YXQGTFTSDYSKYLDSRRAQDFVQWLMHT | - | 6.04 | 108.2 |
| SEQ ID NO: 34 | WXQGTFTSDYSKYCD**E**RRA**K**EFVQWLMHT | ring formed | 6.21 | 0.2 |
| SEQ ID NO: 35 | YXQGTFTSDYSKYCD**E**RRA**K**EFVQWLMHT | ring formed | 6.2 | 17.7 |
| SEQ ID NO: 36 | YXQGTFTSDCSKYLD**E**RRA**K**EFVQWLMNT | ring formed | 6.21 | 9.9 |
| SEQ ID NO: 37 | YXQGTFTSDYSKYLD**E**RRA**K**EFVQWLMNTC | ring formed | 6.21 | 225.5 |
| SEQ ID NO: 38 | YXQGTFCSDYSKYLD**E**RRA**K**EFVQWLMNT | ring formed | 6,15 | 167.3 |
| SEQ ID NO: 39 | YXQGTFVSDCSKYLD**E**RRA**K**DFVQWLMHT | ring formed | 6.15 | 3.7 |
| SEQ ID NO: 40 | YXQGTFVSDYSKYLD**E**RRA**K**DFVQWLMNTC | ring formed | 6.15 | 40.8 |
| SEQ ID NO: 41 | YXQGTFCSDYSKYLD**E**RRA**K**DFVQWLMNT | ring formed | 6.03 | 45.2 |
| SEQ ID NO: 42 | YXQGTFCSDYSKYLDSRRAQDFVGWLMNT | - | 6.03 | 37.9 |
| SEQ ID NO: 43 | YXGGTFTSDCSKYLDSRRAQDFVQWLMNT | - | 6.03 | 1.6 |
| SEQ ID NO: 44 | YXQGTFTSDYSKYLDSRRAQDFVQWLMNTC | - | 6.21 | 75.4 |

In the amino acid sequences described in Table 1, the amino acid marked with X represents a non-native amino acid, aminoisobutyric acid (Aib), the underlined amino acid residues represent formation of a lactam ring between side chains of the amino acid pair, "-" in the amino acid sequence indicates that no amino acid residue is present on the corresponding position, and "-" in the ring indicates that no ring is formed.

### Example 3: Measurement of pi of glucagon derivative

In order to identify improved physical properties of the glucagon derivatives synthesized in Example 2, pi values were calculated based on the amino acid sequences using the pl/Mw tool (http://expasy.org/tools/pi_tool.html; Gasteiger *et al.,* 2003) in the ExPASy server.

As shown in Table 1 above, while the native glucagon of SEQ ID NO: 1 had a pi of 6.8, some glucagon derivatives according to the present invention showed pi values in the range of about 4 to about 6. Since the glucagon derivatives have pi values lower or higher than that of native glucagon, they may exhibit improved solubility and higher stability at neutral pH condition compared to native glucagon.

When the glucagon derivatives according to the present invention are used as a therapeutic agent for treating a target disease such as a metabolic syndrome, patient compliance therewith may be improved, and the glucagon derivatives are also suitable for administration in combination with other anti-obesity agents or anti-diabetes agents, and thus the glucagon derivatives of the present invention may be effectively used as a therapeutic agent for treating a metabolic syndrome as well as obesity, diabetes, non-alcoholic steatohepatitis (NASH), dyslipidemia, and coronary heart disease.

### Example 4: Measurement of cAMP activity of glucagon derivative

Activity of the glucagon derivatives synthesized in Example 2 was measured in cell lines having human glucagon receptors produced in Example 1. Specifically, the transformed cell lines were subcultured 3 to 4 times a week, aliquoted into a 384-well plate in an amount of 6×10³ cell lines/well, and cultured for 24 hours. Native glucagon and glucagon derivatives were suspended in Hank's balanced salt solution (HBSS) buffer containing 0.5 mM 3-isobuty1-1-methylxanthine (IBMX), 0.1% bovine serum albumin (BSA), and 5 mM 4-(2-hydroxyethyl)-1-piperazineethanesulfonic acid (HEPES) with the cultured cells, at concentrations of 200 nM and 1600 nM, respectively, continuously subjected into a 4-fold dilution 10 times, applied to a cAMP assay kit (LANCE cAMP 384 kit, PerkinElmer), and added to the cultured cells, and their fluorescence values were measured. Upon measurement, the highest fluorescence value was set to 100%, and then EC₅₀ values of the glucagon derivative were calculated based thereon and each compared with that of native glucagon. The results are shown in Table 1 above.

### Example 5: Preparation of conjugate including glucagon derivative and immunoglobulin Fc (glucagon derivative-immunoglobulin Fc region conjugate)

A conjugate was prepared by selecting the glucagon derivative prepared in Example 2 above having a pi value of 6 to 7 and an *in vitro* activity of 200% or more as a representative glucagon derivative. Specifically, for pegylation of 10 kDa PEG having a maleimide group and an aldehyde group at both ends respectively, i.e., maleimide-PEG-aldehyde (10 kDa, NOF, Japan) into a cysteine residue of a glucagon derivative, the glucagon derivatives and the maleimide-PEG-aldehyde were reacted at a molar ratio of 1:1 to 5 with a protein concentration of 3 mg/mL to 10 mg/mL at low temperature for 1 to 3 hours. In this case, the reaction was conducted in an environment including 50 mM Tris buffer (pH 7.5) to which 20% to 60% isopropanol was added. Upon completion of the reaction, the reaction solution was applied to SP sepharose HP (GE Healthcare, USA) to purify the glucagon derivatives mono-pegylated on cysteine.

Subsequently, the purified mono-pegylated glucagon derivative and an immunoglobulin Fc were reacted at a molar ratio of 1:2 to 10 with a protein concentration of 10 mg/mL to 50 mg/mL at 4°C to 8°C for 12 hours to 18 hours. The reaction was conducted in an environment in which 10 mM to 50 mM sodium cyanoborohydride, as a reducing agent, and 10% to 20% isopropanol were added to a 100 mM calcium phosphate buffer (pH 6.0). Upon completion of the reaction, the reactant solution was applied to the Butyl sepharose FF purification column (GE Healthcare, USA) and Source ISO purification column (GE Healthcare, USA) to purify the conjugate including the glucagon derivative and the immunoglobulin Fc.

After preparation, purity analyzed by reverse-phase chromatography, size-exclusion chromatography, and ion-exchange chromatography was 95% or more.

In this regard, the conjugate in which the glucagon derivative is linked to the immunoglobulin Fc by PEG was named "conjugate including the glucagon derivative and the immunoglobulin Fc", "glucagon derivative long-acting conjugate", or "long-acting glucagon derivative", which may be used interchangeably herein.

### Experimental Example 1: Effect on losing body weight and increasing insulin sensitivity in mouse with high-fat-diet-induced obesity

Mice with high-fat-diet-induced obesity, which have been widely used as obesity animal models, were used for this experiment. The body weights of the mice were in the range of about 50 g to 55 g. During this experiment, mice were housed 7 mice per each group and were given *ad libitum* access to water. Lights were turned off between 6 P.M. and 6 A.M.

Test groups fed with high-fat diet include: Group 1: administered with an excipient not including long-acting glucagon (5 mL/kg, injection once every 2 days) - control (Vehicle), Group 2: administered with a GLP-1 analog obesity drug (Saxenda®, 50 nmol/kg, injection twice a day), Group 3: administered with the long-acting glucagon derivative (1.2 nmol/kg, injection once every 2 days), and Group 4: administered with a DPP-4 inhibitor (Sitagliptin, 49.2 mg/kg, injection once a day).

The experiment was terminated on the 28^{th} day, and changes in body weight of the mice in each group were measured at 2-day intervals during the progress of the experiment, and changes in blood glucose level were measured before administration and at the 1^{st}, 4^{th}, 7^{th}, 10^{th}, 13^{th}, 16^{th}, 19^{th}, 22^{nd}, 25^{th}, and 28^{th} days after administration. Upon termination of the experiment, the weight of fat and HOMA-IR were measured.

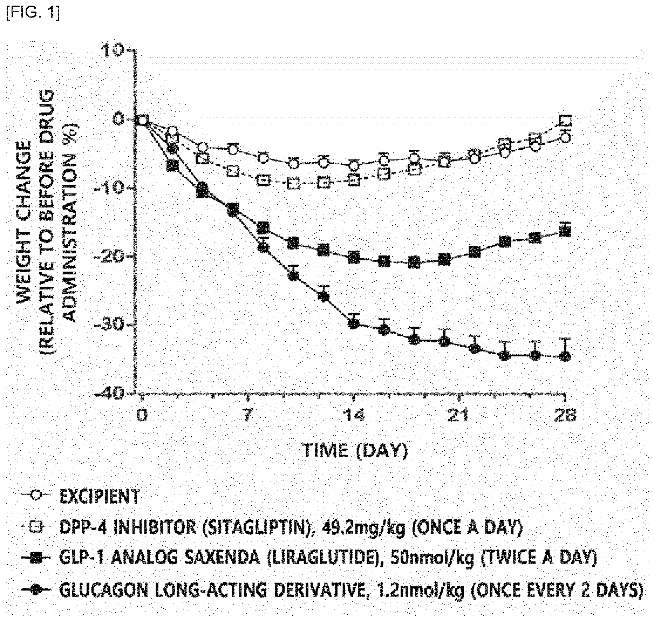

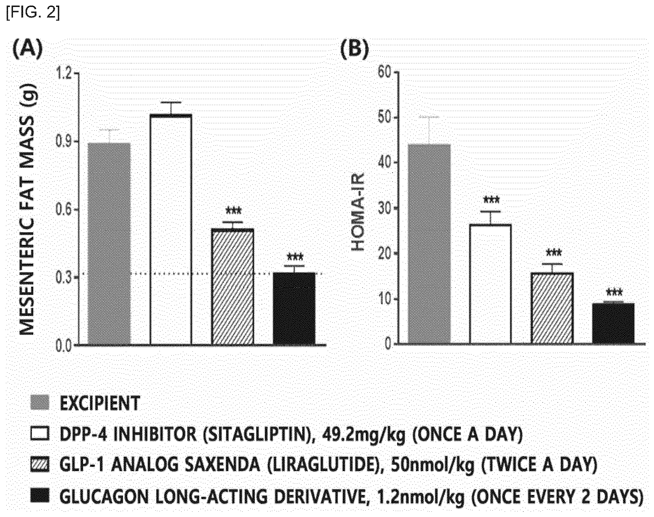

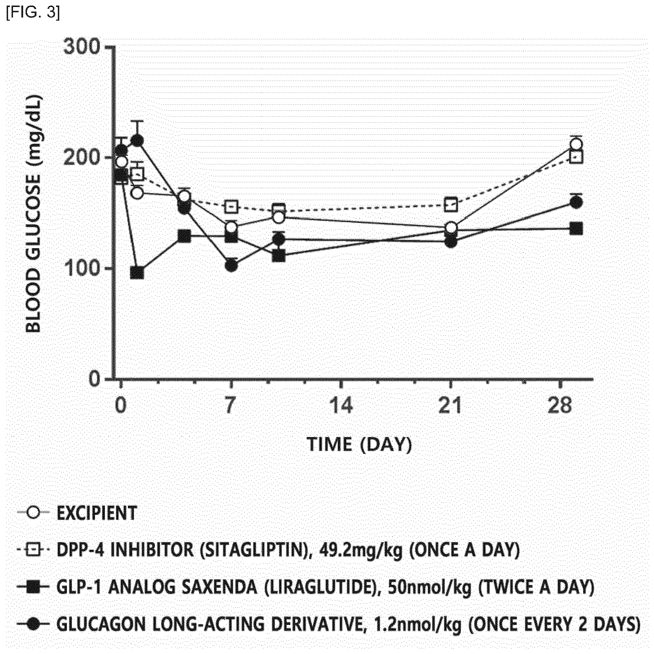

As a result of the measurement of changes in body weight, as shown in FIG. 1, it was confirmed that the group administered with the long-acting glucagon derivative (1.2 nmol/kg, once every 2 days) showed weight loss by -34.5% compared to the body weight before administration, and the effects were greater than the weight loss by -2.6%, -16.3%, and -0.1% obtained using the control (Vehicle), the GLP-1 analog obesity drug (Saxenda®), and the DPP-4 inhibitor (Sitagliptin), respectively. As shown in FIGS. 2(A) and 2(B), it was confirmed that weight loss was caused by a considerable decrease in the amount of fat and insulin sensitivity was improved based on the HOMA-IR measurement results. In addition, as shown in FIG. 3, it was confirmed that the glucagon had greater effects on losing weight than the GLP-1 analog and the DPP-4 inhibitor at a dose with no risk of hyperglycemia based on the fact that no increase was observed in blood glucose levels during the administration period.

Statistical analysis was performed by comparing the excipient group (control) with the test groups by 1-way ANOVA.

The results as shown above indicate that the peptide with activity to a glucagon receptor of the present invention may be used as a medicament for preventing or treating various metabolic syndromes, hypoglycemia, or congenital hyperinsulinism.

### Experimental Example 2: Effect of co-administration of long-acting glucagon derivative and oral drug for diabetes on losing body weight and increasing insulin sensitivity in mouse with high-fat-diet-induced obesity

Mice with high-fat-diet-induced obesity, which have been widely used as obesity animal models, were used for this experiment. The body weights of the mice were in the range of about 50 g to 55 g. During this experiment, mice were housed 7 mice per each group and were given *ad libitum* access to water. Lights were turned off between 6 P.M. and 6 A.M.

Test groups fed with high-fat diet include: Group 1: administered with an excipient not including long-acting glucagon (5 mL/kg, injection once every 2 days) - control (Vehicle), Group 2: administered with a GLP-1 analog obesity drug (Saxenda®, 50 nmol/kg, injection twice a day), Group 3: administered with the long-acting glucagon derivative (1.2 nmol/kg, injection once every 2 days), Group 4: administered with a DPP-4 inhibitor (Sitagliptin, 49.2 mg/kg, injection once a day), Group 5: administered with an SGLT-2 inhibitor (Empagliflozin, 12.3 mg/kg, injection once a day), Group 6: co-administered with Sitagliptin (49.2 mg/kg, injection once a day) and the long-acting glucagon derivative (1.2 nmol/kg, injection once every 2 days), and Empagliflozin (12.3 mg/kg, injection once a day) and the long-acting glucagon derivative (1.2 nmol/kg, injection once every 2 days). The long-acting glucagon derivative used in this experiment was the long-acting derivative according to Example 5.

The experiment was terminated on the 28^{th} day, and changes in body weight of the mice in each group were measured at 2-day intervals during the progress of the experiment, and changes in blood glucose level were measured before administration and at the 1^{st}, 4^{th}, 7^{th}, 10^{th}, 13^{th}, 16^{th}, 19^{th}, 22^{nd}, 25^{th}, and 28^{th} days after administration. Upon termination of the experiment, the weight of fat and HOMA-IR were measured.

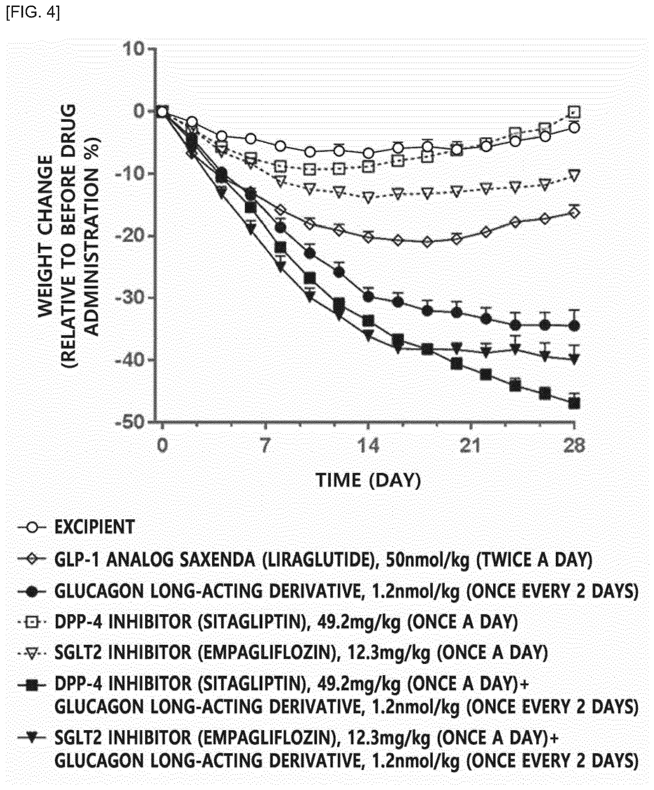

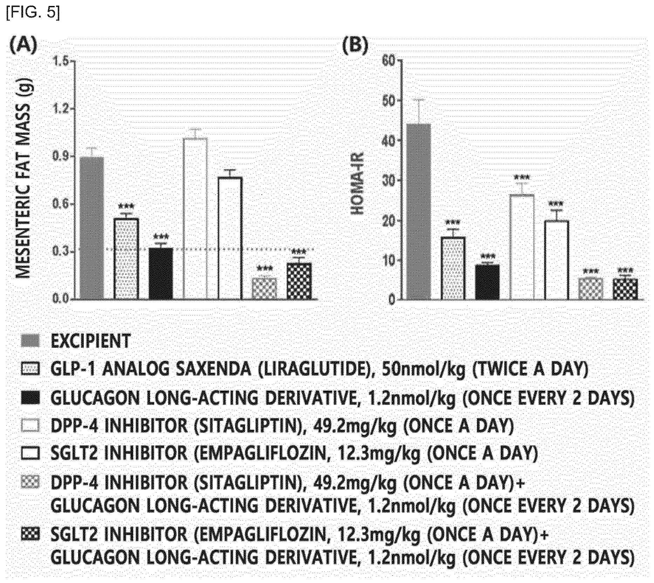

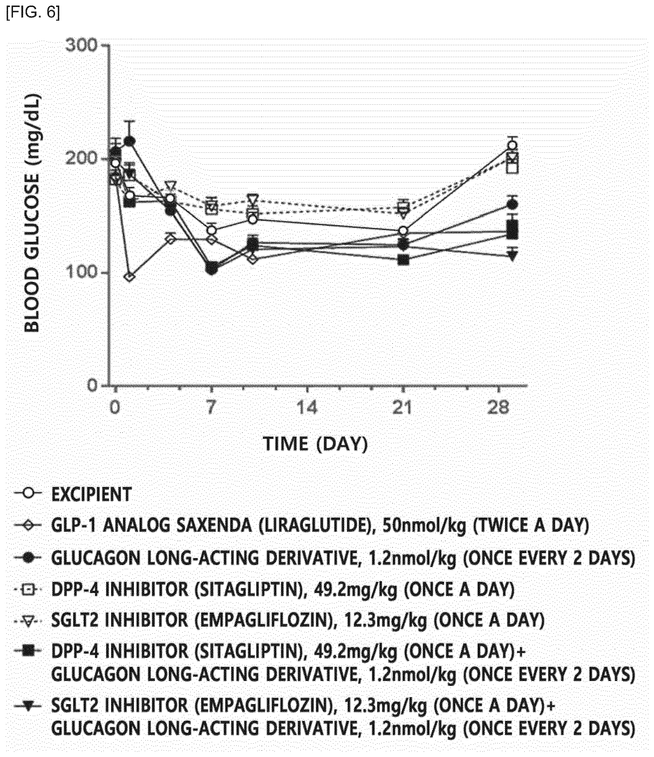

As a result of the measurement of changes in body weight, as shown in FIG. 4, it was confirmed that the groups co-administered with the long-acting glucagon derivative (1.2 nmol/kg, once every 2 days) and the DPP-4 inhibitor (Sitagliptin) or the SGLT-2 inhibitor (Empagliflozin) showed greater effects on weight loss by -46.9% and -39.5%, respectively, compared to weight loss of the group treated only with the long-acting glucagon derivative, and the effects of the co-administration were greater than those of the control (Vehicle) and the groups treated with the GLP-1 analog obesity drug (Saxenda®), the DPP-4 inhibitor (Sitagliptin), and the SGLT-2 inhibitor (Empagliflozin) which showed weight loss by -2.6%, -16.3%, -0.1%, and -10.4%, respectively. As shown in FIGS. 5(A) and 5(B), it was confirmed that weight loss is caused by a considerable decrease in the amount of fat and insulin sensitivity was improved based on the HOMA-IR measurement results. In addition, as shown in FIG. 6, it was confirmed that the co-administration had greater effects on losing weight than the GLP-1 analog and the DPP-4 inhibitor at a dose with no risk of hyperglycemia based on the fact that no increase was observed in blood glucose levels during the administration period. Furthermore, it was confirmed that when the glucagon derivative was co-administered with the oral drug for diabetes such as the DPP-4 inhibitor, Sitagliptin, and the SGLT-2 inhibitor, Empagliflozin, enhanced therapeutic effects on obesity may be expected via additional weight loss and improved effects on controlling blood glucose levels may be expected by improving insulin sensitivity.

Statistical analysis was performed by comparing the excipient group (control) with the test groups by 1-way ANOVA.

### Experimental Example 3: Identification of effect of long-acting glucagon derivative and long-acting GLP-1 analog on weight loss and additional effect of co-administration in mouse with high-fat-diet-induced obesity

Mice with high-fat-diet-induced obesity, which have been widely used as obesity animal models, were used for this experiment. The body weights of the mice were in the range of about 50 g to 55 g. During this experiment, mice were housed 7 mice per each group and were given *ad libitum* access to water. Light was turned off between 6 P.M. and 6 A.M.

Test groups fed with high-fat diet include: Group 1: administered with an excipient not including long-acting glucagon (5 mL/kg, injection once every 2 days) - control (Vehicle), Group 2: administered with a GLP-1 analog (Saxenda®, 50 nmol/kg, injection twice a day), Group 3: administered with a long-acting GLP-1 analog (Ozempic®, 20.5 nmol/kg, injection once in every 2 days, Group 4: administered with a long-acting GLP-1 analog (Trulicity®, 2.7 nmol/kg, injection once every 2 days), Group 5: administered with the long-acting glucagon derivative (2.0 nmol/kg, injection once every 2 days), Group 6: co-administered with the GLP-1 analog (Saxenda®, 50 nmol/kg, injection twice a day) and the long-acting glucagon derivative (2.0 nmol/kg, injection once every 2 days), Group 7: co-administered with the long-acting GLP-1 analog (Ozempic®, 20.5 nmol/kg, injection once every 2 days) and the long-acting glucagon derivative (2.0 nmol/kg, injection once every 2 days), and Group 8: co-administered with the long-acting GLP-1 analog (Trulicity®, 2.7 nmol/kg, injection once every 2 days) and the long-acting glucagon derivative (2.0 nmol/kg, injection once every 2 days). The long-acting glucagon derivative used in this experiment was the long-acting derivative according to Example 5.

The experiment was terminated on the 28^{th} day, and changes in body weight of the mice in each group were measured at 2-day intervals during the progress of the experiment. Upon termination of the experiment, blood lipid levels, weights of fat, and blood glucose levels were measured.

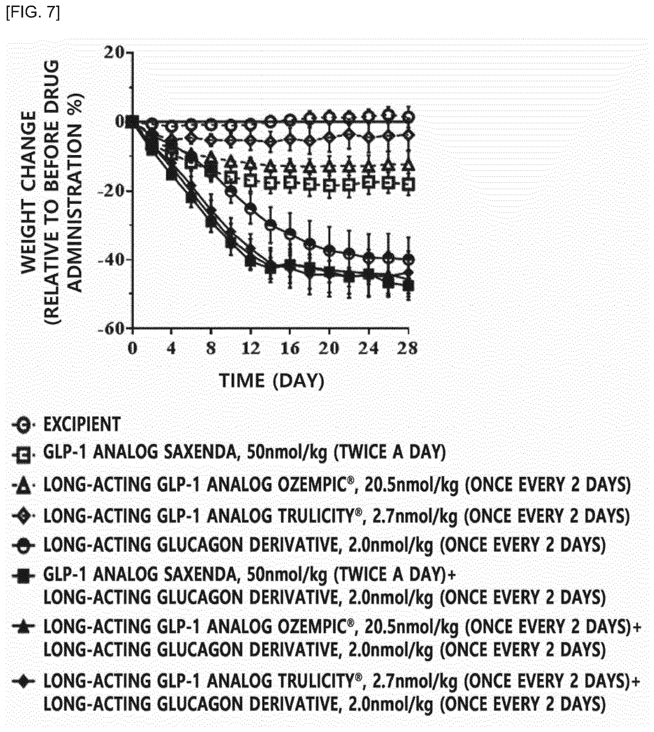

As a result of the measurement of changes in body weight, as shown in FIG. 7, it was confirmed that the groups co-administered of the glucagon long-acting derivative (2.0 nmol/kg, once every 2 days) respectively with the GLP-1 analog (Saxenda®), the long-acting GLP-1 analog (Ozempic®), and the long-acting GLP-1 analog (Trulicity®) exhibited greater effects on weight loss by -47.54%, -45.71%, and -43.68% compared to the body weight before administration, and the weight losses were greater than the weight loss by -39.88% obtained using the glucagon long-acting derivative alone, and the effects were greater than weight losses of the control (Vehicle), the GLP-1 analog obesity drug (Saxenda®), the long-acting GLP-1 analog (Ozempic®), and the long-acting GLP-1 analog (Trulicity®) by 1.34%, -18.10%, -12.38%, and -3.81% respectively.

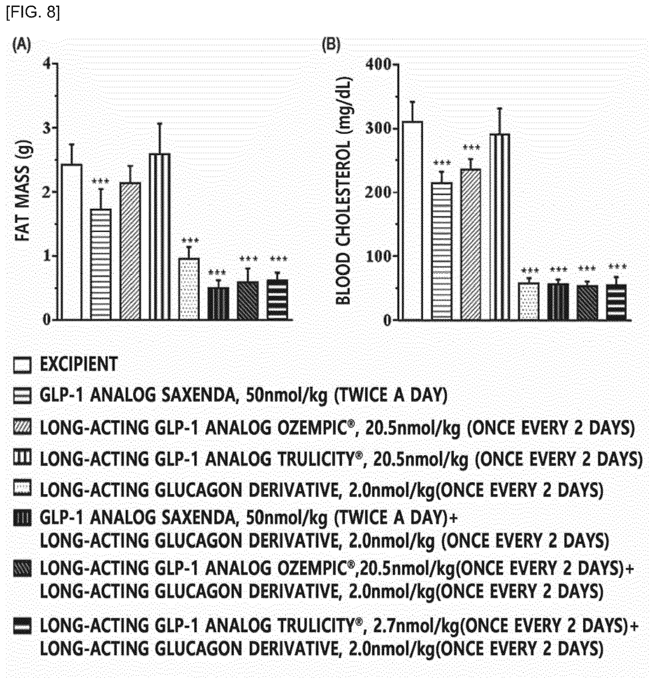

Also, as shown in FIGS. 8(A) and 8(B), decreases in fat and in blood lipid levels were additionally confirmed similarly to the weight loss effects.

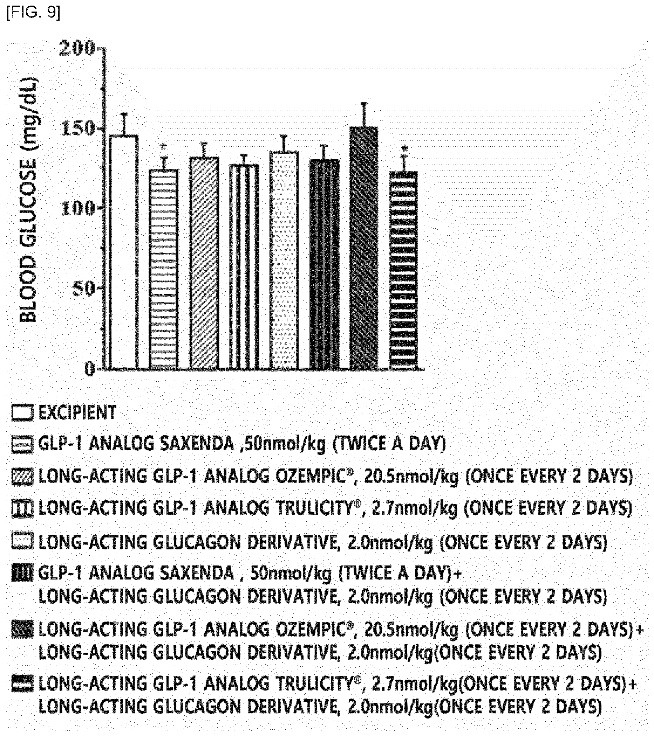

In addition, as shown in FIG. 9, it was confirmed that glucagon had greater effects on losing weight than the GLP-1 analog, as another obesity drug, and at a dose with no risk of hyperglycemia based on the fact that no increase was observed in blood glucose levels during the administration period, and furthermore, enhanced therapeutic effects of co-administration may be expected by additional weight loss via co-administration with the GLP-1 analog.

Statistical analysis was performed by comparing the excipient group (control) with the test groups by 1-way ANOVA.

The results as shown above indicate that co-administration of the substance with activity to a glucagon receptor or a conjugate thereof and the compound or substance with therapeutic activity against a metabolic syndrome may be used as a medicament for preventing or treating a metabolic syndrome.

The above description of the present invention is provided for the purpose of illustration, and it would be understood by those skilled in the art that various changes and modifications may be made without changing the technical conception and essential features of the present invention. Thus, it is clear that the above-described embodiments are illustrative in all aspects and do not limit the present invention. The various embodiments disclosed herein are not intended to be limiting, with the true scope and spirit being indicated by the following claims. The present invention is to be limited only by the terms of the appended claims, along with the full scope of equivalents to which such claims are entitled.

## Claims

1. A combination comprising (i) a substance with activity to a glucagon receptor or a conjugate thereof and (ii) a compound or substance with therapeutic activity against a metabolic syndrome.

2. The combination of claim 1, wherein the substance with activity to a glucagon receptor is native glucagon or an agonist or derivative thereof.

3. The combination of claim 2, wherein the derivative of native glucagon is that where one or more amino acids of the native glucagon are varied, and the variation is selected from the group consisting of substitution, addition, deletion, modification, and any combination thereof.

4. The combination of claim 1, wherein the substance with activity to a glucagon receptor is a peptide comprising an amino acid sequence of General Formula 1 below: 
X1-X2-QGTF-X7-SD-X10-S-X12-X13-X14-X15-X16-X17-X18-X19-X20-X21-F
-X23-X24-W-L-X27-X28-X29-X30 (General Formula 1, SEQ ID NO: 45)
wherein X1 is histidine (H), desamino-histidyl, *N*-dimethyl-histidyl, β-hydroxy imidazopropionyl, 4-imidazoacetyl, β-carboxy imidazopropionyl, tryptophan (W), or tyrosine (Y), or is absent;
X2 is α-methyl-glutamic acid, aminoisobutyric acid (Aib), D-alanine, glycine (G), *N*-methylglycine (Sar), serine (S), or D-serine;
X7 is threonine (T), valine (V), or cysteine (C);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D), glutamic acid (E), or cysteine (C);
X16 is glutamic acid (E), aspartic acid (D), serine (S), α-methyl-glutamic acid, or cysteine (C), or is absent;
X17 is aspartic acid (D), glutamine (Q), glutamic acid (E), lysine (K), arginine (R), serine (S), cysteine (C), or valine (V), or is absent;
X18 is alanine (A), aspartic acid (D), glutamic acid (E), arginine (R), valine (V), or cysteine (C), or is absent;
X19 is alanine (A), arginine (R), serine (S), valine (V), or cysteine (C), or is absent;
X20 is lysine (K), histidine (H), glutamine (Q), aspartic acid (D), arginine (R), α-methyl-glutamic acid, or cysteine (C), or is absent;
X21 is aspartic acid (D), glutamic acid (E), leucine (L), valine (V), or cysteine (C), or is absent;
X23 is isoleucine (I), valine (V), or arginine (R), or is absent;
X24 is valine (V), arginine (R), alanine (A), cysteine (C), glutamic acid (E), lysine (K), glutamine (Q), α-methyl-glutamic acid, or leucine (L), or is absent;
X27 is isoleucine (I), valine (V), alanine (A), lysine (K), methionine (M), glutamine (Q), or arginine (R), or is absent;
X28 is glutamine (Q), lysine (K), asparagine (N), or arginine (R), or is absent;
X29 is lysine (K), alanine (A), glycine (G), or threonine (T), or is absent; and X30 is cysteine (C) or is absent
(excluding a case where the amino acid sequence of General Formula 1 is identical to SEQ ID NO: 1).

5. The combination of claim 4, wherein, in General Formula 1 above,
X1 is histidine (H), tryptophan (W), or tyrosine (Y), or is absent;
X2 is serine (S) or aminoisobutyric acid (Aib);
X7 is threonine (T), valine (V), or cysteine (C);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D) or cysteine (C);
X16 is glutamic acid (E), serine (S) or cysteine (C);
X17 is aspartic acid (D), glutamic acid (E), lysine (K), arginine (R), serine (S), cysteine (C), or valine (V);
X18 is aspartic acid (D), glutamic acid (E), arginine (R), or cysteine (C); X19 is alanine (A) or cysteine (C);
X20 is glutamine (Q), aspartic acid (D), lysine (K), or cysteine (C);
X21 is aspartic acid (D), glutamic acid (E), leucine (L), valine (V), or cysteine (C);
X23 is isoleucine (I), valine (V), or arginine (R);
X24 is valine (V), arginine (R), alanine (A), glutamic acid (E), lysine (K), glutamine (Q), or leucine (L);
X27 is isoleucine (I), valine (V), alanine (A), methionine (M), glutamine (Q), or arginine (R);
X28 is glutamine (Q), lysine (K), asparagine (N), or arginine (R);
X29 is threonine (T); and
X30 is cysteine (C) or is absent.

6. The combination of claim 4, wherein, in General Formula 1 above,
X1 is histidine (H), tryptophan (W), or tyrosine (Y);
X2 is serine (S) or aminoisobutyric acid (Aib);
X7 is cysteine (C), threonine (T), or valine (V);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D) or cysteine (C);
X16 is glutamic acid (E), serine (S), or cysteine (C);
X17 is glutamic acid (E), lysine (K), arginine (R), cysteine (C), or valine (V);
X18 is arginine (R) or cysteine (C);
X19 is alanine (A) or cysteine (C);
X20 is glutamine (Q) or lysine (K);
X21 is aspartic acid (D), glutamic acid (E), valine (V), or cysteine (C);
X23 is valine (V);
X24 is valine (V) or glutamine (Q);
X27 is methionine (M);
X28 is asparagine (N) or arginine (R);
X29 is threonine (T); and
X30 is cysteine (C) or is absent.

7. The combination of claim 4, wherein, in General Formula 1 above,
X1 is tyrosine (Y);
X2 is aminoisobutyric acid (Aib);
X7 is cysteine (C), threonine (T), or valine (V);
X10 is tyrosine (Y) or cysteine (C);
X12 is lysine (K);
X13 is tyrosine (Y) or cysteine (C);
X14 is leucine (L) or cysteine (C);
X15 is aspartic acid (D) or cysteine (C);
X16 is glutamic acid (E), serine (S), or cysteine (C);
X17 is lysine (K), arginine (R), cysteine (C), or valine (V);
X18 is arginine (R) or cysteine (C);
X19 is alanine (A) or cysteine (C);
X20 is glutamine (Q) or lysine (K);
X21 is aspartic acid (D), glutamic acid (E), or cysteine (C);
X23 is valine (V);
X24 is glutamine (Q);
X27 is methionine (M);
X28 is asparagine (N) or arginine (R);
X29 is threonine (T); and
X30 is cysteine (C) or is absent.

8. The combination of claim 4, wherein, in General Formula 1 above,
X1 is histidine (H), tryptophan (W), or tyrosine (Y), or is absent;
X2 is serine (S) or aminoisobutyric acid (Aib);
X7 is threonine (T), valine (V), or cysteine (C);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D) or cysteine (C);
X16 is glutamic acid (E), serine (S), or cysteine (C);
X17 is aspartic acid (D), glutamic acid (E), lysine (K), arginine (R), serine (S), cysteine (C), or valine (V);
X18 is aspartic acid (D), glutamic acid (E), arginine (R), or cysteine (C);
X19 is alanine (A) or cysteine (C);
X20 is glutamine (Q), aspartic acid (D), or lysine (K);
X21 is aspartic acid (D) or glutamic acid (E);
X23 is valine (V);
X24 is valine (V) or glutamine (Q);
X27 is isoleucine (I) or methionine (M);
X28 is asparagine (N) or arginine (R);
X29 is threonine (T); and
X30 is cysteine (C) or is absent.

9. The combination of claim 4, wherein, in General Formula 1 above,
X1 is tyrosine (Y);
X2 is aminoisobutyric acid (Aib);
X7 is threonine (T);
X10 is tyrosine (Y);
X12 is lysine (K);
X13 is tyrosine (Y);
X14 is leucine (L);
X15 is aspartic acid (D) or cysteine (C);
X16 is glutamic acid (E), serine (S), or cysteine (C);
X17 is lysine (K) or arginine (R);
X18 is arginine (R);
X19 is alanine (A);
X20 is glutamine (Q), cysteine (C), or lysine (K);
X21 is aspartic acid (D), cysteine (C), valine (V) or glutamic acid (E);
X23 is valine (V) or arginine (R);
X24 is glutamine (Q) or leucine (L);
X27 is methionine (M);
X28 is asparagine (N) or arginine (R);
X29 is threonine (T); and
X30 is absent.

10. The combination of claim 4, wherein the peptide comprises an amino acid sequence of General Formula 2 below: 
Y-Aib-QGTF-X7-SD-X10-S-X12-Y-L-X15-X16-X17-R-A-X20-X21-F-V-X24-W-
L-M-N-T-X30 (General Formula 2, SEQ ID NO: 46)
 wherein, in General Formula 2 above,
X7 is threonine (T), valine (V), or cysteine (C);
X10 is tyrosine (Y) or cysteine (C);
X12 is lysine (K) or cysteine (C);
X15 is aspartic acid (D) or cysteine (C);
X16 is glutamic acid (E) or serine (S);
X17 is lysine (K) or arginine (R);
X20 is glutamine (Q) or lysine (K);
X21 is aspartic acid (D) or glutamic acid (E);
X24 is valine (V) or glutamine (Q); and
X30 is cysteine (C) or is absent.

11. The combination of claim 4, wherein the peptide has a pi value different from a pi value (6.8) of the native glucagon.

12. The combination of claim 4, wherein each amino acid of at least one amino acid pair among the amino acid pairs of X10 and X14, X12 and X16, X16 and X20, X17 and X21, X20 and X24, and X24 and X28 in General Formula 1 is substituted with glutamic acid or lysine capable of forming a ring.

13. The combination of claim 12, wherein each amino acid of the amino acid pair of X12 and X16, the amino acid pair of X16 and X20, or the amino acid pair of X17 and X21 is substituted with glutamic acid or lysine capable of forming a ring.

14. The combination of claim 4, wherein a ring is formed between amino acids of at least one amino acid pair among the amino acid pairs of X10 and X14, X12 and X16, X16 and X20, X17 and X21, X20 and X24, and X24 and X28 in General Formula 1 above.

15. The combination of claim 4, wherein the C-terminus of the peptide is amidated.

16. The combination of claim 4, wherein the C-terminus of the peptide is not modified.

17. The combination of claim 4, wherein the peptide is a derivative of native glucagon capable of activating the glucagon receptor.

18. The combination of claim 4, wherein the peptide comprises an amino acid sequence selected from the group consisting of SEQ ID NOS: 2 to 44.

19. The combination of claim 10, wherein the peptide comprises an amino acid sequence selected from the group consisting of SEQ ID NOS: 12, 13, 15, and 36 to 44.

20. The combination of claim 1, wherein the conjugate is in a form in which a biocompatible material is linked to the substance with activity to a glucagon receptor.

21. The combination of claim 20, wherein the substance with activity to a glucagon receptor is in the form of a peptide, wherein a biocompatible material is linked to a peptide site with activity to a glucagon receptor.

22. The combination of claim 20, wherein the biocompatible material is selected from the group consisting of a polymer, a fatty acid, cholesterol, albumin and fragments thereof, an albumin binding material, a polymer of a repeating unit with a particular amino acid sequence, an antibody, an antibody fragment, an FcRn binding material, *in vivo* connective tissue or a derivative thereof, a nucleotide, fibronectin, transferrin, a saccharide, heparin, and elastin.

23. The combination of claim 22, wherein the polymer is selected from the group consisting of polyethylene glycol, polypropylene glycol, an ethylene glycol-propylene glycol copolymer, polyoxyethylated polyol, polyvinylalcohol, a polysaccharide, dextran, polyvinylethylether, a biodegradable polymer, a lipid polymer, chitin, hyaluronic acid, an oligonucleotide, and any combination thereof.

24. The combination of claim 22, wherein the FcRn binding material comprises an immunoglobulin Fc region.

25. The combination of claim 18, wherein the substance with activity to a glucagon receptor is linked to the biocompatible material via a linker.

26. The combination of claim 25, wherein the linker is selected from the group consisting of a peptide, a fatty acid, a saccharide, a polymer, a low-molecular-weight compound, a nucleotide, and any combination thereof.

27. The combination of claim 26, wherein the polymer is selected from the group consisting of polyethylene glycol, polypropylene glycol, an ethylene glycol-propylene glycol copolymer, polyoxyethylated polyol, polyvinylalcohol, a polysaccharide, dextran, polyvinylethylether, a biodegradable polymer, a lipid polymer, chitin, hyaluronic acid, an oligonucleotide, and any combination thereof.

28. The combination of claim 25, wherein the linker is polyethylene glycol.

29. The combination of claim 24, wherein the immunoglobulin Fc region is aglycosylated.

30. The combination of claim 24, wherein the immunoglobulin Fc region comprises one selected from the group consisting of: (a) CH1 domain, CH2 domain, CH3 domain, and CH4 domain; (b) CH1 domain and CH2 domain; (c) CH1 domain and CH3 domain; (d) CH2 domain and CH3 domain; (e) a combination of one or more domains selected from the CH1 domain, CH2 domain, CH3 domain, and CH4 domain and an immunoglobulin hinge region or a part of the hinge region; and (f) a dimer of each domain of the heavy chain constant region and the light chain constant region.

31. The combination of claim 24, wherein the polypeptide comprising the immunoglobulin Fc region is in a dimeric form.

32. The combination of claim 24, wherein the immunoglobulin Fc region is a derivative of native Fc from which a region capable of forming a disulfide bond is deleted, from which some amino acids of the N-terminus are removed, to which a methionine residue of the N-terminus is added, from which a complement-binding site is removed, or from which an antibody-dependent cell-mediated cytotoxicity (ADCC) region is removed.

33. The combination of claim 24, wherein the immunoglobulin Fc region is an Fc region derived from immunoglobulin selected from the group consisting of IgG, IgA, IgD, IgE, and IgM.

34. The combination of claim 33, wherein the immunoglobulin Fc region is an IgG4 Fc region.

35. The combination of claim 24, wherein the immunoglobulin Fc region is an aglycosylated Fc region derived from human IgG4.

36. The combination of claim 26, wherein the linker is linked to a cysteine residue of a peptide with activity to the glucagon receptor.

37. The combination of claim 26, wherein the linker of the conjugate is linked to both the peptide site and the biocompatible material via covalent bonds respectively formed by reactions between one end of the linker and an amine or thiol group of the biocompatible material and between the other end of the linker and an amine or thiol group of the peptide site with activity to a glucagon receptor.

38. The combination of claim 1, wherein the compound or substance with therapeutic activity against a metabolic syndrome is selected from the group consisting of a glucagon-like peptide-1 (GLP-1) receptor agonist, an insulinotropic peptide, a Leptin receptor agonist, a dipeptidyl peptidase-IV (DPP-IV) inhibitor, a Y5 receptor antagonist, a melanin-concentrating hormone (MCH) receptor antagonist, a Y2/4 receptor agonist, a melanocortin 3/4 (MC3/4) receptor agonist, a gastric/pancreatic lipase inhibitor, a 5-hydroxytryptamine receptor 2C (5HT2c), a G protein-coupled) agonist, a β3A receptor agonist, an amylin receptor agonist, a ghrelin antagonist, a ghrelin receptor antagonist, a peroxisome proliferator-activated receptor alpha (PPARα) agonist, a peroxisome proliferator-activated receptor delta (PPARδ) agonist, a farnesoid X receptor (FXR) agonist, an acetyl-CoA carboxylase inhibitor, peptide YY, cholecystokinin (CCK), Xenin, glicentin, obestatin, secretin, nesfatin, insulin, a glucose-dependent insulinotropic peptide (GIP), biguanides, sulfonylureas, meglitinide, thiazolidinedione (TZD), a sodium-glucose cotransporter (SGLT2) inhibitor, and an α-glucosidase inhibitor.

39. The combination of claim 38, wherein the GLP-1 receptor agonist is one selected from the group consisting of exenatide, lixisenatide, dulaglutide, liraglutide, semaglutide, albiglutide, and taspoglutide.

40. The combination of claim 38, wherein the DPP-4 inhibitor is selected from the group consisting of Sitagliptin, Vildagliiptin, Saxagliptin, Alogliptin, Linagliptin, Anagliptin, Teneligliptin, Trelagliptin, migliptin, Omarigliptin, Evogliptin, and Dutogliptin; and the sodium-glucose cotransporter (SGLT2) inhibitor is selected from the group consisting of Empagliflozin, Dapagliflozin, Canagliflozin, Remogliflozin, Remogliflozin Etabonate, Sergliflozin, Ipragliflozin, Tofogliflozin, Luseogliflozin, and Ertugliflozin.

41. The combination of claim 1, wherein the metabolic syndrome is selected from the group consisting of impaired glucose tolerance, hypercholesterolemia, dyslipidemia, obesity, diabetes, hypertension, non-alcoholic steatohepatitis (NASH), arteriosclerosis caused by dyslipidemia, atherosclerosis, arteriosclerosis, coronary heart disease, and stroke.

42. A pharmaceutical composition for preventing or treating a metabolic syndrome comprising the combination of any one of claims 1 to 40.

43. A pharmaceutical composition for preventing or treating a metabolic syndrome comprising a substance with activity to a glucagon receptor or a conjugate thereof, which is likely to be co-administered with a compound or substance with therapeutic activity against the metabolic syndrome.

44. A kit for preventing or treating a metabolic syndrome comprising:
(i) a substance with activity to a glucagon receptor or a conjugate thereof; and
(ii) a compound or substance with therapeutic activity against a metabolic syndrome.

45. A pharmaceutical composition for preventing or treating a metabolic syndrome, hypoglycemia, or congenital hyperinsulinism comprising a peptide with activity to a glucagon receptor or a conjugate thereof.

46. The pharmaceutical composition of claim 45, wherein the metabolic syndrome is selected from the group consisting of impaired glucose tolerance, hypercholesterolemia, dyslipidemia, obesity, diabetes, hypertension, non-alcoholic steatohepatitis (NASH), arteriosclerosis caused by dyslipidemia, atherosclerosis, arteriosclerosis, coronary heart disease, and stroke.

47. The pharmaceutical composition of claim 45, wherein the peptide with activity to the glucagon receptor is native glucagon or an agonist or derivative thereof.

48. The pharmaceutical composition of claim 45, wherein the peptide is a peptide comprising an amino acid sequence of General Formula 1 below: 
X1-X2-QGTF-X7-SD-X10-S-X12-X13-X14-X15-X16-X17-X18-X19-X20-X21-F
-X23-X24-W-L-X27-X28-X29-X30 (General Formula 1, SEQ ID NO: 45)
 wherein, in General Formula 1 above,
X1 is histidine (H), desamino-histidyl, N-dimethyl-histidyl, β-hydroxy imidazopropionyl, 4-imidazoacetyl, β-carboxy imidazopropionyl, tryptophan (W), or tyrosine (Y), or is absent;
X2 is α-methyl-glutamic acid, aminoisobutyric acid (Aib), D-alanine, glycine (G), *N*-methylglycine (Sar), serine (S), or D-serine;
X7 is threonine (T), valine (V), or cysteine (C);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D), glutamic acid (E), or cysteine (C);
X16 is glutamic acid (E), aspartic acid (D), serine (S), α-methyl-glutamic acid, or cysteine (C), or is absent;
X17 is aspartic acid (D), glutamine (Q), glutamic acid (E), lysine (K), arginine (R), serine (S), cysteine (C), or valine (V), or is absent;
X18 is alanine (A), aspartic acid (D), glutamic acid (E), arginine (R), valine (V), or cysteine (C), or is absent;
X19 is alanine (A), arginine (R), serine (S), valine (V), or cysteine (C), or is absent;
X20 is lysine (K), histidine (H), glutamine (Q), aspartic acid (D), arginine (R), α-methyl-glutamic acid, or cysteine (C), or is absent;
X21 is aspartic acid (D), glutamic acid (E), leucine (L), valine (V), or cysteine (C), or is absent;
X23 is isoleucine (I), valine (V), or arginine (R), or is absent;
X24 is valine (V), arginine (R), alanine (A), cysteine (C), glutamic acid (E), lysine (K), glutamine (Q), α-methyl-glutamic acid, or leucine (L), or is absent;
X27 is isoleucine (I), valine (V), alanine (A), lysine (K), methionine (M), glutamine (Q), or arginine (R), or is absent;
X28 is glutamine (Q), lysine (K), asparagine (N), or arginine (R), or is absent;
X29 is lysine (K), alanine (A), glycine (G), or threonine (T), or is absent; and
X30 is cysteine (C) or is absent
(excluding a case where the amino acid sequence of General Formula 1 is identical to SEQ ID NO: 1).

49. The pharmaceutical composition of claim 48, wherein, in General Formula 1 above,
X1 is histidine (H), tryptophan (W), or tyrosine (Y), or is absent;
X2 is serine (S) or aminoisobutyric acid (Aib);
X7 is threonine (T), valine (V), or cysteine (C);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D) or cysteine (C);
X16 is glutamic acid (E), serine (S) or cysteine (C);
X17 is aspartic acid (D), glutamic acid (E), lysine (K), arginine (R), serine (S), cysteine (C), or valine (V);
X18 is aspartic acid (D), glutamic acid (E), arginine (R), or cysteine (C);
X19 is alanine (A) or cysteine (C);
X20 is glutamine (Q), aspartic acid (D), lysine (K), or cysteine (C);
X21 is aspartic acid (D), glutamic acid (E), leucine (L), valine (V), or cysteine (C);
X23 is isoleucine (I), valine (V), or arginine (R);
X24 is valine (V), arginine (R), alanine (A), glutamic acid (E), lysine (K), glutamine (Q), or leucine (L);
X27 is isoleucine (I), valine (V), alanine (A), methionine (M), glutamine (Q), or arginine (R);
X28 is glutamine (Q), lysine (K), asparagine (N), or arginine (R);
X29 is threonine (T); and
X30 is cysteine (C) or is absent.

50. The pharmaceutical composition of claim 48, wherein, in General Formula 1 above,
X1 is histidine (H), tryptophan (W), or tyrosine (Y);
X2 is serine (S) or aminoisobutyric acid (Aib);
X7 is cysteine (C), threonine (T), or valine (V);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D) or cysteine (C);
X16 is glutamic acid (E), serine (S), or cysteine (C);
X17 is glutamic acid (E), lysine (K), arginine (R), cysteine (C), or valine (V);
X18 is arginine (R) or cysteine (C);
X19 is alanine (A) or cysteine (C);
X20 is glutamine (Q) or lysine (K);
X21 is aspartic acid (D), glutamic acid (E), valine (V), or cysteine (C);
X23 is valine (V);
X24 is valine (V) or glutamine (Q);
X27 is methionine (M);
X28 is asparagine (N) or arginine (R);
X29 is threonine (T); and
X30 is cysteine (C) or is absent.

51. The pharmaceutical composition of claim 48, wherein, in General Formula 1 above,
X1 is tyrosine (Y);
X2 is aminoisobutyric acid (Aib);
X7 is cysteine (C), threonine (T), or valine (V);
X10 is tyrosine (Y) or cysteine (C);
X12 is lysine (K);
X13 is tyrosine (Y) or cysteine (C);
X14 is leucine (L) or cysteine (C);
X15 is aspartic acid (D) or cysteine (C);
X16 is glutamic acid (E), serine (S), or cysteine (C);
X17 is lysine (K), arginine (R), cysteine (C), or valine (V);
X18 is arginine (R) or cysteine (C);
X19 is alanine (A) or cysteine (C);
X20 is glutamine (Q) or lysine (K);
X21 is aspartic acid (D), glutamic acid (E), or cysteine (C);
X23 is valine (V);
X24 is glutamine (Q);
X27 is methionine (M);
X28 is asparagine (N) or arginine (R);
X29 is threonine (T); and
X30 is cysteine (C) or is absent.

52. The pharmaceutical composition of claim 48, wherein, in General Formula 1 above,
X1 is histidine (H), tryptophan (W), or tyrosine (Y), or is absent;
X2 is serine (S) or aminoisobutyric acid (Aib);
X7 is threonine (T), valine (V), or cysteine (C);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D) or cysteine (C);
X16 is glutamic acid (E), serine (S) or cysteine (C);
X17 is aspartic acid (D), glutamic acid (E), lysine (K), arginine (R), serine (S), cysteine (C), or valine (V);
X18 is aspartic acid (D), glutamic acid (E), arginine (R), or cysteine (C);
X19 is alanine (A) or cysteine (C);
X20 is glutamine (Q), aspartic acid (D), or lysine (K);
X21 is aspartic acid (D) or glutamic acid (E);
X23 is valine (V);
X24 is valine (V) or glutamine (Q);
X27 is isoleucine (I) or methionine (M);
X28 is asparagine (N) or arginine (R);
X29 is threonine (T); and
X30 is cysteine (C) or is absent.

53. The pharmaceutical composition of claim 48, wherein, in General Formula 1 above,
X1 is tyrosine (Y);
X2 is aminoisobutyric acid (Aib);
X7 is threonine (T);
X10 is tyrosine (Y);
X12 is lysine (K);
X13 is tyrosine (Y);
X14 is leucine (L);
X15 is aspartic acid (D) or cysteine (C);
X16 is glutamic acid (E), serine (S) or cysteine (C);
X17 is lysine (K) or arginine (R);
X18 is arginine (R);
X19 is alanine (A);
X20 is glutamine (Q), cysteine (C), or lysine (K);
X21 is aspartic acid (D), cysteine (C), valine (V) or glutamic acid (E);
X23 is valine (V) or arginine (R);
X24 is glutamine (Q) or leucine (L);
X27 is methionine (M);
X28 is asparagine (N) or arginine (R);
X29 is threonine (T); and
X30 is absent.

54. The pharmaceutical composition of claim 48, wherein the peptide is a peptide comprising an amino acid sequence of General Formula 2 below: 
Y-Aib-QGTF-X7-SD-X10-S-X12-Y-L-X15-X16-X17-R-A-X20-X21-F-V-X24-W-
L-M-N-T-X30 (General Formula 2, SEQ ID NO: 46)
 wherein, in General Formula 2 above,
X7 is threonine (T), valine (V), or cysteine (C);
X10 is tyrosine (Y) or cysteine (C);
X12 is lysine (K) or cysteine (C);
X15 is aspartic acid (D) or cysteine (C);
X16 is glutamic acid (E) or serine (S);
X17 is lysine (K) or arginine (R);
X20 is glutamine (Q) or lysine (K);
X21 is aspartic acid (D) or glutamic acid (E);
X24 is valine (V) or glutamine (Q); and
X30 is cysteine (C) or is absent.

55. The pharmaceutical composition of claim 45, wherein the peptide has a pi value different from a pi value (6.8) of the native glucagon.

56. The pharmaceutical composition of claim 45, wherein the C-terminus of the peptide is amidated.

57. The pharmaceutical composition of claim 45, wherein the C-terminus of the peptide is not modified.

58. The pharmaceutical composition of claim 45, wherein the peptide is native glucagon capable of activating a glucagon receptor.

59. The pharmaceutical composition of claim 45, wherein the peptide comprises an amino acid sequence selected from the group consisting of SEQ ID NOS: 2 to 44.

60. The pharmaceutical composition of any one of claims 45 to 59, wherein the conjugate is in the form of a long-acting conjugate in which a biocompatible material is linked to a peptide site with activity to a glucagon receptor.

61. The pharmaceutical composition of claim 60, wherein the biocompatible material is selected from the group consisting of a polymer, a fatty acid, cholesterol, albumin and fragments thereof, an albumin binding material, a polymer of a repeating unit with a particular amino acid sequence, an antibody, an antibody fragment, an FcRn binding material, *in vivo* connective tissue or a derivative thereof, a nucleotide, fibronectin, transferrin, a saccharide, heparin, and elastin.

62. The pharmaceutical composition of claim 61, wherein the polymer is selected from the group consisting of polyethylene glycol, polypropylene glycol, an ethylene glycol-propylene glycol copolymer, polyoxyethylated polyol, polyvinylalcohol, a polysaccharide, dextran, polyvinylethylether, a biodegradable polymer, a lipid polymer, chitin, hyaluronic acid, an oligonucleotide, and any combination thereof.

63. The pharmaceutical composition of claim 61, wherein the FcRn binding material is a polypeptide comprising an immunoglobulin Fc region.

64. The pharmaceutical composition of claim 60, wherein the peptide site is linked to the biocompatible material via a linker.

65. The pharmaceutical composition of claim 61, wherein the linker is selected from the group consisting of a peptide, a fatty acid, a saccharide, a polymer, a low-molecular-weight compound, a nucleotide, and any combination thereof.

66. The pharmaceutical composition of claim 65, wherein the polymer is selected from the group consisting of polyethylene glycol, polypropylene glycol, an ethylene glycol-propylene glycol copolymer, polyoxyethylated polyol, polyvinylalcohol, a polysaccharide, dextran, polyvinylethylether, a biodegradable polymer, a lipid polymer, chitin, hyaluronic acid, an oligonucleotide, and any combination thereof.

67. The pharmaceutical composition of claim 64, wherein the linker is polyethylene glycol.

68. The pharmaceutical composition of claim 63, wherein the immunoglobulin Fc region is an IgG4 Fc region.

69. The pharmaceutical composition of claim 63, wherein the immunoglobulin Fc region is an aglycosylated Fc region derived from human IgG4.

70. A pharmaceutical composition for preventing or treating hypoglycemia comprising: (i) the pharmaceutical composition according to any one of claims 45 to 59 comprising an isolated peptide or the pharmaceutical composition according to any one of claims 60 to 69 in the form of a long-acting conjugate; and (ii) a pharmaceutically acceptable excipient.

71. A pharmaceutical composition for preventing or treating congenital hyperinsulinism comprising: (i) the pharmaceutical composition according to any one of claims 45 to 59 comprising an isolated peptide or the pharmaceutical composition according to any one of claims 60 to 69 in the form of a long-acting conjugate; and (ii) a pharmaceutically acceptable excipient.

72. A pharmaceutical composition for preventing or treating obesity comprising: (i) the pharmaceutical composition according to any one of claims 45 to 59 comprising an isolated peptide or the pharmaceutical composition according to any one of claims 60 to 69 in the form of a long-acting conjugate; and (ii) a pharmaceutically acceptable excipient.

73. A pharmaceutical composition for preventing or treating a metabolic syndrome comprising: (i) the pharmaceutical composition according to any one of claims 45 to 59 comprising an isolated peptide or the pharmaceutical composition according to any one of claims 60 to 69 in the form of a long-acting conjugate; and (ii) a pharmaceutically acceptable excipient.

74. The pharmaceutical composition of claim 73, wherein the metabolic syndrome is selected from the group consisting of impaired glucose tolerance, hypercholesterolemia, dyslipidemia, obesity, diabetes, hypertension, non-alcoholic steatohepatitis (NASH), arteriosclerosis caused by dyslipidemia, atherosclerosis, arteriosclerosis, coronary heart disease, and stroke.
